(19) **Europäisches Patentamt**
**European Patent Office**
**Office européen des brevets**

(11) **EP 2 986 630 B1**

(12) **EUROPEAN PATENT SPECIFICATION**

(45) Date of publication and mention of the grant of the patent:
**15.08.2018 Bulletin 2018/33**

(51) Int Cl.:
**C07K 14/47** *(2006.01)* **C07K 16/28** *(2006.01)*
**C07K 16/46** *(2006.01)*

(21) Application number: **14710220.6**

(22) Date of filing: **06.03.2014**

(86) International application number:
**PCT/EP2014/054390**

(87) International publication number:
**WO 2014/170063 (23.10.2014 Gazette 2014/43)**

(54) **NOVEL BISPECIFIC BINDING MOLECULES WITH ANTITUMORAL ACTIVITY**

BISPEZIFISCHE BINDUNGSMOLEKÜLE MIT ANTITUMORAKTIVITÄT

MOLÉCULES DE LIAISON BISPÉCIFIQUE AYANT UNE ACTIVITÉ ANTITUMORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.04.2013 EP 13164555**

(43) Date of publication of application:
**24.02.2016 Bulletin 2016/08**

(73) Proprietor: **Covagen AG**
**6300 Zug (CH)**

(72) Inventors:
• **BULLER, Fabian**
 **CH-8049 Zürich (CH)**
• **WÜLLNER, Ulrich**
 **CH-8102 Oberengstringen (CH)**
• **KLUPSCH, Kristina**
 **CH-8047 Zürich (CH)**
• **ZBINDEN, Irene**
 **CH-8048 Zürich (CH)**
• **SANTIMARIA, Roger**
 **CH-8002 Zürich (CH)**
• **ATTINGER-TOLLER, Isabella**
 **CH-8051 Zürich (CH)**
• **KÖNIG-FRIEDRICH, Susann**
 **78224 Singen (DE)**
• **BERTSCHINGER, Julian**
 **CH-8913 Ottenbach (CH)**
• **GRABULOVSKI, Dragan**
 **CH-8049 Zürich (CH)**

• **BRACK, Simon**
 **CH-8400 Winterthur (CH)**
• **SILACCI, Michela**
 **CH-8037 Zürich (CH)**
• **WOODS, Richard**
 **CH-8049 Zürich (CH)**
• **HACHEMI, Helen**
 **CH-8052 Zürich (CH)**
• **HENNE, Patricia**
 **CH-8957 Spreitenbach (CH)**
• **VON DER BEY, Ulrike**
 **78359 Orsingen-Nenzingen (DE)**

(74) Representative: **Verhage, Richard Abraham et al**
**Janssen Vaccines & Prevention B.V.**
**Archimedesweg 4-6**
**2333 CN Leiden (NL)**

(56) References cited:
**WO-A1-2011/023685 WO-A1-2011/116387**

• HUI REN ET AL: "Anti-HER-2xanti-CD3 Bi-specific Antibodies Inhibit Growth of HCT-116 Colorectal Carcinoma Cells in Vitro and in Vivo", ASIAN PACIFIC JOURNAL OF CANCER PREVENTION, vol. 13, no. 6, 30 June 2012 (2012-06-30), pages 2795-2798, XP055079880, ISSN: 1513-7368, DOI: 10.7314/APJCP.2012.13.6.2795

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BAEUERLE PATRICK A ET AL: "BiTE: Teaching antibodies to engage T-cells for cancer therapy", CURRENT OPINION IN MOLECULAR THERAPEUTICS, THOMSON REUTERS (SCIETIFIC) LTD, vol. 11, no. 1, 1 February 2009 (2009-02-01), pages 22-30, XP009151509, ISSN: 2040-3445**
- **D GRABULOVSKI ET AL: "Bispecific Fynomer-antibody fusion proteins targeting two epitopes on HER2", CANCER RESEARCH, vol. 72, no. 24 -suppl. 3, 15 December 2012 (2012-12-15), XP055079857, DOI: 10.1158/0008-5472.SABCS12-P5-18-25**
- **COVAGEN ADVANCED BIOPHARMACEUTICALS: "Covagen utilizes the unique versatility of Fynomers to creat next generation biologics", INTERNET CITATION, 1 November 2011 (2011-11-01), page 1, XP002669730, Retrieved from the Internet: URL:http://www.proteins-congress.com/wordpress/wp-content/uploads/2011/11/Bispecific-Fynomers-Innovations-in-antibody-fusion-proteins.pdf**
- **MICHELA SILACCI ET AL: "COVA301: a highly potent bispecific inhibitor of IL-17A and TNF-alpha", MABS, 1 September 2011 (2011-09-01), page 16, XP055079407,**
- **GRABULOVSKI DRAGAN ET AL: "A novel, non-immunogenic Fyn SH3-derived binding protein with tumor vascular targeting properties", JOURNAL OF BIOLOGICAL CHEMISTRY, AMERICAN SOCIETY FOR BIOCHEMISTRY AND MOLECULAR BIOLOGY, vol. 282, no. 5, 2 February 2007 (2007-02-02), pages 3196-3204, XP002601897, ISSN: 0021-9258, DOI: 10.1074/JBC.M609211200**

**Description**

The present invention relates to a bispecific binding molecule that specifically binds to (i) CD3 and (ii) a surface target antigen on cancer cells, wherein the binding molecule comprises (a) an antibody specifically binding to CD3, and (b) a Fyn-SH3-derived polypeptide binding to a surface target antigen on cancer cells, wherein said Fyn-SH3-derived polypeptide consists of the amino acid sequence of SEQ ID NO: 1, with the proviso that (i) at least one amino acid within amino acids positions 10 to 19 of SEQ ID NO: 1 is substituted, deleted or added, and (ii) at least one amino acid amino acid within amino acids positions 29 to 36 of SEQ ID NO: 1 is substituted, deleted or added, provided that said polypeptide has at least 85% sequence identity to the amino acid sequence of SEQ ID NO: 1, wherein the determination of identity excludes amino acid positions 12 to 17 and 31 to 34 of SEQ ID NO: 1.

There is increasing evidence that T cells are able to control tumor growth and survival in cancer patients, both in early and late stages of the disease (Mlecnik B. et al., (2011), Cancer Metastasis Rev, 30, pp. 5-12). However, tumor-specific T-cell responses are difficult to mount and sustain in cancer patients, and are limited by numerous immune escape mechanisms of tumor cells selected during immunoediting (Bauerle PA et al. (2009) Cancer Res, 69(12), pp. 4941-4944). An alternative approach to engage T-cells for cancer therapy are antibodies, which are bispecific for a surface target antigen on cancer cells, and for CD3 on T-cells (Bargou et al. (2008) Science, 321(5891), pp. 974-977). These are typically capable of connecting any kind of cytotoxic T-cell to a cancer cell, independently of T-cell receptor specificity, costimulation, or peptide antigen presentation.

Murine and humanized anti-CD3 antibodies are well-known in the art, such as muromonab-CD3 (Norman DJ (1995) Ther Drug Monit 17(6), pp. 615-620), teplizumab and otelixizumab (Chatenoud L. (2010) 6(3), pp. 149-157), and Cris-7 (Alberola-Ila J. et al. (1991), J Immunol, 146(4), pp. 1085-1092). Conrad et al gives an overview of other anti-CD3 antibodies including SP34 (Conrad et al. (2007) Cytometry, 71(11), pp. 925-933).

As mentioned, bispecific antibodies recognizing both CD3 and a surface target antigen on cancer cells are well-known in the art (see also May C. et al. (2012), Biochemical Pharmacology, 84, pp. 1105-1112). A bispecific antibody targeting CD3 and a surface antigen on cancer cells (HER-2) is also mentioned in (Hui R, et al, 2012, Asian Pacific Journal of Cancer Prevention 13: 2795-2798). It is important to note that historically, a major obstacle in developing bispecific antibodies as therapeutic agents has been the difficulty in designing these complex molecules with favourable drug-like properties and producing them in sufficient quantity and quality to support drug development (Chan A. and Carter P. (2010), Nat Rev Immunol, 10(5), pp. 301-316). This is exemplified by the early bispecific approaches in the CD3 field that employed quadroma technology which results from the fusion of two unique murine hybridoma clones expressing monoclonal antibodies (mAbs) with selected specificities (Staerz et al. (1986) Proc Natl Acad Sci USA, 83, pp. 1453-1457). Bispecific antibodies prepared through this method rely on the random pairing of IgG heavy and light chains. Despite some biological effects observed on cells expressing the antigen, this approach did not lead to a significant impact in the clinical course of disease (Kufer P. et al. (2004) Trends Biotechnol, 22, pp. 238-244). As mentioned above, these first generation bispecific antibodies demonstrated two major limitations, which included the difficulty of producing large scale homogeneous batches, and the lack of efficacy of murine antibody fragments. In addition, human anti-mouse antibody responses were seen in most treated patients, which severely decreased the efficacy of the murine molecules and limited multiple administrations (May C. et al. (2012), Biochemical Pharmacology, 84, pp. 1105-1112).

Lindhofer et al. identified a method to decrease the level of heterogeneity through the pairing of rat IgG2a and mouse IgG2b (Lindhofer et al. (1995) J Immunol, 155, pp. 219-225). The authors found that fusion of these rat and mouse isotype specific hybridomas increased the incidence of correctly paired bispecific antibodies due to preferential species-restricted heavy/light chain pairing. Additionally, they identified sequential pH elution methods that facilitated the purification of the functional bispecific antibody from parental antibodies. The hybrid antibodies generated by this method incorporated binding sites for two different antigens, such as CD3 and a tumor antigen, and retained its intact Fc that can bind to the FcγR receptors expressed on macrophages, NK cells, and dendritic cells similar to conventional antibodies. These bispecific antibodies, termed TriomAbs®, have the combined effect of redirecting T cells, along with Fc receptor expressing cells to the tumor and have been reported to eliminate tumor cells by a combination of T-cell mediated lysis and ADCC (Linke R. et al (2010) Mabs, 2, pp. 129-136). This format led to the development of a number of bispecific antibodies which have been evaluated in clinical trials. These include catumaxomab (anti-EpCAM x anti-CD3), ertumaxomab (anti-HER2 x anti-CD3), and FBTA05 (anti-CD20 x anti-CD3) (reviewed in Chames P. et al., (2009), Mabs, 1, pp. 539-547). Catumaxomab is the first TriomAb® that was developed and phase 2/3 demonstrated significant improvement in survival which led to catumaxomab receiving EU approval in 2009 for the treatment of malignant ascites in patients with EpCAM-positive tumors, and was the first bispecific antibody approved for clinical use (Jager M. et al. (2012), Cancer Res, 69, 4941-4944). The HER2 targeting TriomAb® ertumaxomab is currently being investigated in clinical trials in patients with HER2 expressing advanced solid tumors. FBTA05, the third TriomAb® being developed to target CD20 expressed on B-cell malignancies, was shown to mediate specific lysis of B-cell lines with low level CD20 expression derived from chronic lymphocytic leukemia (CLL) patients. Moreover, FBTA05 demonstrated a significantly higher eradication rate when compared to rituximab in vitro. A Phase 1/2 study evaluating the safety and efficacy of FBTA05 in combination with donor lymphocyte infusions for treatment of relapsed or refractory disease in CD20 positive non-Hodgkin lymphoma (NHL) patients after allogeneic transplantation is ongoing (Chames P. et al., (2009), Mabs, 1, pp. 539-547). Major limitations to this TriomAb® format exist due to the development of human anti-mouse and anti-rat antibodies by patients in response to repeated administration of the non-humanized mouse/rat hybrid antibody format (Linke R. et al (2010) Mabs, 2, pp. 129-136).

Some of the limitations of TriomAb® antibodies have been addressed by antibody engineering and the generation of recombinant bispecific molecules based on novel formats (see overview in May C. et al. (2012), Biochemical Pharmacology, 84, pp. 1105-1112). Among the bispecific antibody designs that have been tested, the format termed BiTE® (for bi-specific T-cell engager), has demonstrated the greatest success in the clinic to date. The BiTE® recombinant format is based on two single-chain antibodies that are covalently linked by a peptide linker (resulting in a bispecific scFv antibody fragment format). A total of four variable domains encoded by four distinct genes are aligned on a single polypeptide chain encoded by just one gene. BiTEs® have been studied in great detail for their in vitro and in vivo selective T-cell activation at picomolar (pM) concentrations, as well as their mechanism of action (Baeuerle PA et al., (2009), Cancer Res, 69, pp. 4941-4944). BiTEs® that target CD19 (expressed by most human B cell malignancies (Dreier T. et al., (2003), J Immunol, 170, pp. 4397-4402)) and EpCAM, which is expressed on a number of solid tumors (Brischwein K. et al. (2006), Mol Immunol, 43, pp. 1129-1143) are the most advanced molecules and are currently being tested in clinical trials. Blinatumomab (also known as MT103), which is a recombinant anti-CD3 x anti-CD19 BiTE® molecule is being studied as a treatment of patients with relapsed non-hodgkin-lymphoma (NHL) and acute lymphoblastic leukemia (ALL). The first confirmed responses to blinatumomab as a single agent occurred in NHL patients (Bargou R. et al. (2008), Science, 321, pp. 974-977). More recently, a phase 2 clinical study was conducted to determine the efficacy of blinatumomab in minimal residual disease (MRD)-positive B-lineage ALL (Topp MS et al. (2011) J Clin Oncol, 29, pp. 2493-2498). Twenty-one patients were treated at a dose level of 0.015 mg/m$^2$/day, of whom sixteen patients became MRD negative. The probability for relapse-free survival was reported to be 78% at a median follow-up of 405 days. Blinatumomab was efficacious and well-tolerated in patients with MRD-positive B-lineage ALL and treatment resulted in prolonged leukemia-free survival. In addition to CD19 and EpCAM, preclinical studies have shown that BiTE® antibodies can also specifically target tumor cells expressing EGFR, CEA, EPHA2, melanoma-associated chondroitin sulfate proteoglycan, IGFR1, CD33, fibroblast-activating protein alpha, prostate stem cell antigen (PSCA), c-MET, PSMA and HER2 (Baeuerle PA et al., (2009), Cancer Res, 69, pp. 4941-4944; Friedrich M. (2012) Mol Cancer Ther, 11(12), pp. 2664-2673). BiTE® molecules targeting CD3 and a surface antigen on cancer cells and advantages of such molecules over some other bispecific antibody formats are also disclosed in (Baeuerle P, et al, 2009, Curr Opin Mol Ther 11: 22-30).

Because of the small size of BiTE® antibodies (about 55 kDa), they are characterized by a short serum half-life. For example, continuous intravenous infusion of blinatumomab by a portable pump is required for the administration of this drug. Therefore, the short in vivo half-life is one of the major limitations of the BiTE® antibodies (Kontermann R. (2005) Acta Pharmacologica Sinica, 26(1), pp. 1-9).

Other bispecific formats based on scFv antibody fragments recognizing both CD3 and a surface target antigen on cancer cells are known in the art, such as the DART™ platform, which consist of two distinct polypeptides that are coexpressed to generate a covalently linked heterodimeric complex with one binding site for each of 2 specificities (Moore et al, (2011), Blood, 117, pp. 4542-4551), and the tetravalent tandem diabody structure called tandab®, where two polypeptides with each four variable domains from two different antibodies arrange head-to-tail to a homodimeric molecule (Mølhøj M. et al, (2007), Mol Immunol, 44(8), pp. 1935-1943).

In addition to the rather short half-life of scFv-based bispecific strategies, scFv-based constructs have a tendency to form aggregates due to 'domain exchange' of the V-regions with partners from other molecules (Moore et al, (2011), Blood, 117, pp. 4542-4551), or, as shown for the DART™ platform, they depend on more complicated expression systems to form heterodimeric complexes (Moore et al, (2011), Blood, 117, pp. 4542-4551).

In recent years, a number of other bispecific formats have been generated (reviewed in Chan A. and Carter P. (2010), Nat Rev Immunol, 10(5), pp. 301-316, and Kontermann R. (2012), Mabs, 4(2), pp. 182-197) some of which have been designed to target CD3 and a tumor cell surface antigen. Human IgG-like bispecific technologies have also been evolved in this field, for example CD3 bispecifics based on the Biclonics™ ENGAGE platform that uses single light chain antibodies (www.merus.nl), "kappa-lambda-body™", that relies on single heavy chain antibodies (www.novimmune.com) and bispecific IgG1/IgG2 antibodies (Strop et al. (2012) J Mol Biol, 420, pp. 204-219). Despite these recent advances in the engineering of bispecific formats for redirected T-cell killing, there are still drawbacks for these newer technologies, such as the requirement of co-expression of either two heavy chains with a single light chain, or one heavy chain with two light chains and the subsequent more complicated downstream purification processes, or the need of suitable redox conditions for heterodimerization in the case of IgG1/IgG2 bispecifics. Therefore, there is a large medical need in the creation of bispecifics with drug-like properties, such as high-level expression, favorable physicochemical properties (such as high solubility and stability plus low propensity to aggregate) and long serum half-life. The need for improved bispecifics is also exemplified by the emergence of scientific conferences discussing bispecific compounds. For example, it has been stated at the 2nd World Bispecific Antibody Summit that the industry's focus on bispecifics is intensifying. It has been contemplated that bispecifics have been the subject of several impressive deals worth more than $6.5 billion in the past 2 years. On the one hand there are exciting opportunities to embrace and huge scope for growth, but there are still many issues that need to be addressed before this new class of drug can reach its full potential (http://www.nature.com/natureevents/science/events/14667; 2nd_World_Bispecific_Antibody_Summit).

Thus, there is a large medical need for novel bispecific formats useful in the treatment of tumors, which have favourable drug-like properties, can be produced in sufficient quantity and quality to support drug development and are suitable as therapeutics. Accordingly, the object of the present invention is the provision of such novel bispecific binding molecules which target CD3 and a tumor cell surface antigen.

The invention therefore relates in a first embodiment to a bispecific binding molecule that specifically binds to (i) CD3 and (ii) a surface target antigen on cancer cells, wherein the binding molecule comprises (a) an antibody specifically binding to CD3, and (b) a Fyn-SH3-derived polypeptide binding to a surface target antigen on cancer cells, wherein said Fyn-SH3-derived polypeptide consists of the amino acid sequence of SEQ ID NO: 1, with the proviso that (i) at least one amino acid within amino acids positions 10 to 19 of SEQ ID NO: 1 is substituted, deleted or added, and (ii) at least one amino acid amino acid within amino acids positions 29 to 36 of SEQ ID NO: 1 is substituted, deleted or added, provided that said polypeptide has at least 85% sequence identity to the amino acid sequence of SEQ ID NO: 1, wherein the determination of identity excludes amino acid positions 12 to 17 and 31 to 34 of SEQ ID NO: 1.

The term "bispecific binding molecule that specifically binds to (i) CD3 and (ii) a surface target antigen on cancer cells" means that the binding molecule of the present invention is capable of specifically binding to an antigen of CD3 and an antigen of a surface target antigen on cancer cells, said antigen being different from CD3. Moreover, the bispecific binding molecule of the present invention is capable of binding to said two targets at the same time. Hence, also the term "CD3 bispecific binding molecule" of the invention includes two binding specificities, one against CD3 ("anti-CD3") and the second against a different surface target antigen on cancer cells ("anti-surface target antigen on cancer cells" or "anti-tumor antigen").

In accordance with the present invention, a molecule is considered to bind specifically (also referred to herein as interacting specifically) when the respective molecule does not or essentially does not cross-react with an epitope of similar structure. Cross-reactivity of a panel of molecules under investigation may be tested, for example, by assessing binding of said panel of molecules under conventional conditions to the epitope of interest as well as to a number of more or less (structurally and/or functionally) closely related epitopes. Only those molecules that bind to the epitope of interest in its relevant context (e.g. a specific motif in the structure of a protein) but do not or do not essentially bind to any of the other epitope are considered specific for the epitope of interest. Corresponding methods are described e.g. in Harlow and Lane, Antibodies: A Laboratory Manual, Cold Spring Harbor Laboratory Press, 1988 or Harlow and Lane, Using Antibodies: A Laboratory Manual, Cold Spring Harbor Laboratory Press, 1999. The term "a molecule that essentially does not cross-react with an epitope of similar structure", as used herein, refers to a molecule that binds to the target antigen with at least 5-times higher affinity than to an epitope of similar structure, more preferably at least 10-times higher affinity, such as e.g. at least 50-times higher affinity, more preferably at least 100-times higher affinity, such as e.g. at least 500-times higher affinity. Even more preferably, it binds with at least 1.000-times higher affinity to the target antigen than to an epitope of similar structure, such as e.g. at least 10.000-times higher affinity and most preferably at least 100.000-times higher affinity.

Preferably, the bispecific binding molecule of the invention binds to CD3 with a $K_D$ of $5x10^{-7}$ to $10^{-12}$ M, more preferably $10^{-8}$ to $10^{-12}$ M, most preferably $10^{-9}$ to $10^{-12}$ M. Likewise, the bispecific binding molecule of the invention binds the selected surface target antigen on cancer cells with a $K_D$ of $10^{-7}$ to $10^{-12}$ M, more preferably $10^{-8}$ to $10^{-12}$ M, most preferably $10^{-9}$ to $10^{-12}$ M.

An epitope may be a conformational or a continuous epitope. In polypeptide antigens, a conformational (or discontinuous) epitope is characterized by the presence of two or more discrete amino acid residues which are separated in the primary sequence, but are located near each other on the surface of the molecule when the polypeptide folds into the native three-dimensional structure to constitute the epitope (Sela, (1969) Science 166, 1365 and Laver, (1990) Cell 61, 553-6). The two or more discrete amino acid residues contributing to the epitope are present in separate sections or even in more than one (poly)peptide chain of the antigen. In contrast, a linear or continuous epitope consist of two or more discrete amino acid residues which are located adjacent in a single linear segment of a (poly)peptide chain.

The term "surface target antigen on cancer cells" refers to an antigen that is either not expressed on non-tumor cells or is expressed on tumor cells in a higher amount than on non-tumor cells. The terms "surface target antigen on cancer cells", "tumor antigen" and "tumor associated antigen" are used interchangeably herein. The surface target antigen on cancer cells is not CD3 antigen and more preferably not an antigen within a component of the T-cell receptor. Preferably, the antigen is expressed on tumor cells in an at least two times higher amount as on non-tumor cells, more preferably an least five times higher amount, such as e.g. an at least 10-times higher amount, even more preferably an at least 100-time higher amount, such as e.g. an at least 1000-times higher amount and most preferably an at least 10.000-times higher amount. Suitable target antigens include any such antigen that is expressed more strongly on tumor cells. Preferably the antigen is one of the antigens defined herein below.

The term "peptide" refers to a linear molecular chain of up to 30 amino acids. On the other hand, the term "polypeptide" as used herein interchangeably with the term "protein" describes linear molecular chains of amino acids, including single chain proteins or their fragments, containing more than 30 amino acids. The term "(poly)peptide" as used herein refers to both peptides and polypeptides. (Poly)peptides may further form oligomers consisting of at least two identical or different molecules. The corresponding higher order structures of such multimers are, correspondingly, termed homo- or heterodimers, homo- or heterotrimers etc. Homodimers, trimers etc. of fusion proteins also fall under the definition of the term "polypeptide". Furthermore, peptidomimetics of such (poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "peptide" also refer to naturally modified (poly)peptides where the modification is effected e.g. by glycosylation, acetylation, phosphorylation and similar modifications which are well known in the art.

In accordance with the present invention, the binding molecule comprises or consists of an antibody as defined in (a) and a Fyn-SH3-derived polypeptide as defined in (b).

It will be appreciated that one or more copies of said Fyn-SH3-derived polypeptide may be present in the bispecific binding molecule of the invention, such as e.g. two, three or four copies of the Fyn-SH3-derived polypeptide. It is preferred that two copies of the Fyn-SH3-derived polypeptide are present. If two copies of the Fyn-SH3-derived polypeptide are present, the following preferred options are available: (i) The first of said two polypeptides is linked to the first N-terminus of the two heavy chains of said antibody and the second of said two polypeptides is linked to the second N-terminus of the two heavy chains of said antibody (i.e. in other terms the first of copy of said two polypeptides is linked to the N-terminus of the first heavy chain of said antibody, and the second copy of said two polypeptides is linked to the N-terminus of the second heavy chain of said antibody), (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody.

The term "Fyn SH3-derived polypeptide", used interchangeably herein with the term "Fynomer®", refers to a non-immunoglobulin-derived binding polypeptide (e.g. a so-called scaffold) derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

The SH3 domain of the Fyn kinase (Fyn SH3) comprises 63 residues, namely amino acids 83 to 145 of the sequence reported by Semba et al. (1986) (Proc. Natl. Acad. Sci. U S A 83(15): 5459-63) and Kawakami et al. (1986) (Mol Cell Biol. 6(12): 4195-201), with the sequence GVTLFVALYD<u>YEARTEDD</u>LSFHKGEKFQIL<u><u>NSSE</u></u>GDW-WEARSLTTGETGYIPSNYVAPVDSIQ, as shown in SEQ ID NO: 1. Fyn is a 59 kDa member of the Src family of tyrosine kinases. As a result of alternative splicing, the Fyn protein exists in two different isoforms differing in their kinase domains: one form is found in thymocytes, splenocytes and some hematolymphoid cell lines, while a second form accumulates principally in brain (Cooke and Perlmutter (1989),New Biol. 1(1): 66-74). The biological functions of Fyn, which is an intracellular protein, are diverse and include signalling via the T cell receptor, regulation of brain function as well as adhesion mediated signalling (Resh (1998) Int. J. Biochem. Cell Biol. 30(11): 1159-62). Just as other SH3 domains, the Fyn SH3 is composed of two antiparallel β-sheets and contains two flexible loops (the RT-Src and n-Src-loops) in order to interact with other proteins. The sequences of the two flexible loops (called RT-Src and n-Src-loops) are underlined and double-underlined in SEQ ID NO: 1, respectively. The amino acid sequence of Fyn SH3 of SEQ ID NO: 1 is fully conserved among man, mouse, rat and monkey (gibbon).

As has been shown in the art (WO 2008/022759; Grabulovski et al. (2007) JBC, 282, p. 3196-3204), the Fyn SH3 domain is a particularly attractive scaffold for the generation of binding polypeptides, i.e. Fynomers®. The reason for this is because Fynomers® (i) can be expressed in bacteria in soluble form in high amounts, (ii) do not aggregate when stored in solution, (iii) are very stable ($T_m$ 70.5 °C), (iv) lack cysteine residues, and (v) are originally derived from human featuring an amino acid sequence completely conserved from mouse to man, thereby reducing unwanted immunogenic reactions.

The Fynomer® of the invention is preferably not a natural SH3 domain containing protein existing in or isolated from nature. In other words, the scope of the invention preferably excludes wild type SH3 domain containing proteins. There are abundant SH3 domain containing proteins in nature. These natural SH3 proteins have a binding affinity to their natural ligands. Most of these natural SH3 ligands have a PxxP motif. The Fynomers® of the invention are engineered proteins designed for having affinities to non-natural targets being distinct from the targets of wild-type SH3 domains. A non-natural target of a SH3 domain may be any target provided that said target is not a natural (i.e. wild-type), intracellular ligand of a wild type SH3 domain existing in nature. For example, a non-natural target of a SH3 domain may be any target existing in nature, preferably existing in a mammalian, more preferably existing in a human, again with the proviso that said target is not a natural, intracellular ligand of a wild type SH3 domain existing in nature-. More preferably, the Fynomers® of the invention essentially have no binding affinity to any natural, intracellular SH3 binding ligands, most preferably not to any natural, intracellular SH3 binding ligand having a PxxP motif.

The derivation of a specifically binding Fyn SH3-derived polypeptide for a particular target antigen has been described in the art. For example, a library of different Fyn SH3 can be created in which the sequence as shown in SEQ ID NO: 1 above has been altered. Preferably, the alteration is carried out (i) in the sequence representing the RT- loop (as shown underlined above in SEQ ID NO: 1) or optionally in a position up to two amino acids adjacent to said sequence (i.e. the sequence DYEARTEDDL in SEQ ID NO: 1), or (ii) in the Scr loop (as shown double-underlined above in SEQ ID NO: 1) or optionally in a position up to two amino acids adjacent to said sequence (i.e. the sequence ILNSSEGD in SEQ ID NO: 1) or (iii) in both sequences simultaneously. Preferably, the alteration is a substitution, deletion or addition as described in the art (see e.g. WO 2008/022759; Grabulovski et al. (2007) JBC, 282, p. 3196-3204). Means and methods for altering an amino acid sequence are well known in the art and are described in the art, e.g. in Grabulovski et al. (2007) JBC, 282, p. 3196-3204.

Subsequently, this Fyn SH3 library can be cloned into a phagemid vector, such as e.g. pHEN1 (Hoogenboom et al. "Multi-subunit proteins on the surface of filamentous phage: methodologies for displaying antibody (Fab) heavy and light chains", Nucleic Acids Res, 19(15):4133-7, 1991) and the library may be subsequently displayed on phage and subjected to panning, preferably repeated rounds of panning such as e.g. at least two, more preferably at least three rounds of panning against the respective antigen. Then, screening for binding (poly)peptides can be performed by established techniques, such as e.g. monoclonal phage-ELISA. Sequencing of the thus identified clones may then be employed to reveal the enriched sequences. The thus identified binding (poly)peptide may further be subjected to further maturation steps, such as e.g. by generating additional libraries based on alterations of the identified sequences and repeated phage display and panning steps. Finally, cross-reactivity and immunogenicity of the resulting Fyn SH3-derived polypeptide may be analysed and a Fyn SH3-derived polypeptide specific for the target antigen can be selected.

Methods of phage display screening and optimization of binding (poly)peptides are generally known in the art.
In the context of this invention the RT loop of the Fyn kinase SH3 domain (sometimes also designated RT-Src-loop) consists of the amino acids "EARTED" that are located in positions 12 to 17 in SEQ ID NO: 1. The positions to be substituted, deleted and/or added, i.e. to be mutated, in or adjacent to the RT loop are in accordance with the present invention amino acids 10 to 19, preferably 11 to 18, and more preferably 12 to 17.

Likewise, in the context of this invention the src loop of the FYN kinase (sometimes also designated n-Src-loop) consists of the amino acids NSSE that are located in positions 31 to 34 in SEQ ID NO: 1. The positions to be substituted, deleted and/or added, i.e. to be mutated, in or adjacent to the src loop are in accordance with the present invention amino acids 29 to 36, preferably 30 to 35, more preferably 31 to 34.

The recited at least 85% sequence identity embrace any higher value of sequence identity. Envisaged values of sequence identity are at least 86%, at least 87%, at least 88%, at least 89% at least 90%, at least 91%, at least 92%, at least 93%, at least 94%, at least 95%, at least 96%, at least 97%, at least 98%, at least 99% and 100%. When determining sequence identity, the indicated amino acid positions (12 to 17 and 31 to 34), which positions are defined in relation to the sequence consisting of SEQ ID NO: 1, are to be disregarded. In other terms, while items (i) and (ii) of the main embodiment require differences with regard to the wild type SH3 domain sequence of SEQ ID NO: 1, the remainder of the Fynomer® sequence has a sequence identity to the wild-type between 85% and 100%. Determining the such defined sequence identity can be done, for example, by performing a pair wise sequence alignment, disregarding the sequence identity reported by the alignment program and re-evaluating the degree of sequence identity such that positions 12 to 17 and 31 to 34 of SEQ ID NO: 1 are not considered. Such re-evaluation of sequence identity can be done by the skilled person without further ado in view of these determination instructions. As mentioned, the ranges from 12 to 17 and 31 to 34 are underlined in the presentation of the sequence of SEQ ID NO: 1 herein above. When including two flanking amino acids at either side, the ranges from 10 to 19 and 29 to 36 (always in terms of amino acids of the sequence consisting of SEQ ID NO: 1) are obtained, which are the regions according to items (i) and (ii) of the main embodiment where, in either case, one, two, three, four, five or six amino acid substitutions, deletions or additions are to be effected.

As used herein, the term "amino acid sequence identity" between amino acid sequences is meant to relate to the common and widely used alignment and comparison techniques of the person skilled in biochemistry. The amino acid sequence identity of two amino acid sequences can be determined by common alignment methods and tools. For example, for determining the extent of an amino acid sequence identity of an arbitrary polypeptide relative to the amino acid sequence of SEQ ID NO: 1, the SIM Local similarity program is preferably employed (Xiaoquin Huang and Webb Miller (1991), Advances in Applied Mathematics, vol. 12: 337-357), that is freely available from the authors and their institute (see also the world wide web: http://www.expasy.org/tools/sim-prot.html). For multiple alignment analysis ClustalW is preferably used (Thompson et al. (1994) Nucleic Acids Res., 22(22): 4673-4680). The extent of the amino acid sequence identity of a polypeptide to the amino acid sequence of SEQ ID NO: 1 is determined relative to the complete sequence of SEQ ID NO: 1, with the exception of positions 12 to 17, and 31 to 34 of SEQ ID NO: 1.

A substitution may be a conservative or a non-conservative substitution, but preferably is a conservative substitution. In some embodiments, a substitution also includes the exchange of a naturally occurring amino acid with a non-naturally occurring amino acid. A conservative substitution comprises the substitution of an amino acid with another amino acid having a chemical property similar to the amino acid that is substituted. Preferably, the conservative substitution is a substitution selected from the group consisting of: (i) a substitution of a basic amino acid with a different basic amino acid; (ii) a substitution of an acidic amino acid with a different acidic amino acid; (iii) a substitution of an aromatic amino acid with a different aromatic amino acid; (iv) a substitution of a non-polar, aliphatic amino acid with a different non-polar, aliphatic amino acid; and (v) a substitution of a polar, uncharged amino acid with a different polar, uncharged amino acid. A basic amino acid is selected from the group consisting of arginine, histidine, and lysine. An acidic amino acid is selected from aspartate or glutamate. An aromatic amino acid is selected from the group consisting of phenylalanine, tyrosine and tryptophane. A non-polar, aliphatic amino acid is selected from the group consisting of glycine, alanine, valine, leucine, methionine and isoleucine. A polar, uncharged amino acid is selected from the group consisting of serine, threonine, cysteine, proline, asparagine and glutamine. In contrast to a conservative amino acid substitution, a non-conservative amino acid substitution is the exchange of one amino acid with any amino acid that does not fall under the above-outlined conservative substitutions (i) through (v).

The CD3 (cluster of differentiation 3) T-cell co-receptor or simply referred to herein as "CD3" is a protein complex and is composed of four distinct chains. In mammals, the complex contains a CD3$\gamma$ chain, a CD3$\delta$ chain, and two CD3$\varepsilon$ chains. These chains associate with a molecule known as the T-cell receptor (TCR) and the $\zeta$-chain to generate an activation signal in T lymphocytes. The TCR, $\zeta$-chain, and CD3 molecules together comprise the TCR complex. As discussed above, murine and humanized anti-CD3 antibodies are well-known in the art, such as muromonab-CD3 (Norman DJ (1995) Ther Drug Monit 17(6), pp. 615-620), teplizumab and otelixizumab (Chatenoud L. (2010) 6(3), pp. 149-157), Cris-7 (Alberola-Ila J. et al. (1991), J Immunol, 146(4), pp. 1085-1092) and others such as SP34 (Conrad et al. (2007) Cytometry, 71(11), pp. 925-933). In addition, several bispecific antibody formats recognizing both CD3 and a surface target antigen on cancer cells for the treatment of cancer patients have been described in the literature. However and as mentioned, many bispecific CD3 formats exhibit poor drug-like properties, such as non-satisfying ease of production, production yield, product solubility and pharmacokinetic parameters of the product.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')$_2$, Fv or scFv fragments, single domain V$_H$ or V-like domains, such as VhH or V-NAR-domains, as well as multimeric formats such as minibodies, diabodies, tribodies, tetrabodies or chemically conjugated Fab'-multimers (see, for example, Altshuler et al., 2010., Holliger and Hudson, 2005). The term "antibody" also includes embodiments such as chimeric (human constant domain, non-human variable domain), single chain and humanized (human antibody with the exception of non-human CDRs) antibodies. Humanized, full-length IgG antibodies consisting of two heavy chains and two light chains are most preferred. The term "full-length antibody" refers therefore to antibodies consisting of two heavy chains and two light chains.

An "Fc part of an antibody" is a term well-known to the skilled artisan and defined on the basis of papain cleavage of antibodies. Depending on the amino acid sequence of the constant region of their heavy chains, immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called [alpha], [delta], [epsilon], [gamma], and [mu], respectively. The Fc part of an antibody is directly involved in ADCC (antibody-dependent cell-mediated cytotoxicity) and CDC (complement-dependent cytotoxicity) based on complement activation, C1q binding and Fc receptor binding. The four human IgG isotypes bind different receptors, such as the neonatal Fc receptor, the activating Fc gamma receptors, FcgRI, FcgRIIa, and FcgRIIIa, the inhibitory receptor FcgRIIb, and C1q with different affinities, yielding very different activities. It is known that the affinities to activating and inhibiting receptors of an Fc part of a human antibody can be engineered and modified (see Strohl W. (2009) Curr Opin Biotechnol, 20, p. 685-691).

The antibody specifically binding to CD3 within the bispecific binding molecule of the invention preferably comprises an Fc part which allows for extending the *in vivo* half-life of the bispecific binding molecule of the invention. Such Fc part is preferably from human origin, more preferably a human Fc part of an IgG1 antibody, even more preferably an engineered human Fc part of an IgG1 with activating or silencing effector functions, wherein silencing effector functions are preferred over activating effector functions. Most preferably, such an Fc part is an engineered human Fc part of an IgG1 with silencing effector functions with a mutation in L234 and L235, numbering according to EU index of Kabat (see Johnson G. and Wu T.T. (2000) Nucleic Acids Res. 28 p. 214-218). The antibody having SEQ ID NOs 2 and 3 which has been used in the Examples herein below for the preparation of the constructs COVA420, COVA422 and COVA467 is an anti-CD3 antibody with silencing effector functions and mutations in positions L234 and L235. Also the antibody having SEQ ID NOs 171 and 172 which has been used in the Examples herein below for the preparation of the constructs

EP 2 986 630 B1

COVA493, COVA494, COVA497 and COVA499 is an anti-CD3 antibody with silencing effector mutations in positions D265 and P329, numbering according to EU index of Kabat (see Shields RL. et al (2001) J. Biol. Chem. 276 p. 6591 - 6604).

Various techniques for the production of antibodies and fragments thereof are well known in the art and described, e.g. in Altshuler et al., 2010. Thus, polyclonal antibodies can be obtained from the blood of an animal following immunisation with an antigen in mixture with additives and adjuvans and monoclonal antibodies can be produced by any technique which provides antibodies produced by continuous cell line cultures. Examples for such techniques are described, e.g. Harlow and Lane (1988) and (1999) and include the hybridoma technique originally described by Köhler and Milstein, 1975, the trioma technique, the human B-cell hybridoma technique (see e.g. Kozbor, 1983; Li et al., 2006) and the EBV-hybridoma technique to produce human monoclonal antibodies (Cole et al., 1985). Furthermore, recombinant antibodies may be obtained from monoclonal antibodies or can be prepared *de novo* using various display methods such as phage, ribosomal, mRNA, or cell display. A suitable system for the expression of the recombinant (humanized) antibodies or fragments thereof may be selected from, for example, bacteria, yeast, insects, mammalian cell lines or transgenic animals or plants (see, e.g., US patent 6,080,560; Holliger and Hudson, 2005). Further, techniques described for the production of single chain antibodies (see, inter alia, US Patent 4,946,778) can be adapted to produce single chain antibodies specific for the target of this invention. Surface plasmon resonance as employed in the BIAcore system can be used to increase the efficiency of phage antibodies.

Bispecific binding molecules of the invention may be prepared by any of the many conventional and well known techniques such as plain organic synthetic strategies, solid phase-assisted synthesis techniques or by commercially available automated synthesizers. On the other hand, they may also be prepared by conventional recombinant techniques alone or in combination with conventional synthetic techniques. A bispecific binding molecules according to the present invention may be obtained by combining compounds as defined in (a) and (b) herein above.

As mentioned above, some bispecific antibodies recognizing both CD3 and a surface target antigen on cancer cells exhibit very potent anti-tumoral killing activity. Thus, BiTE® antibodies exhibit a high potency of redirected lysis of cells expressing the target tumor antigen. The $EC_{50}$ values of BiTE® antibodies in general range from 0.1 to 50 pM (Bauerle PA and Reinhardt C. (2009) Cancer Res. 69(12), pp. 4941-4944). A potent cell killing of tumor cells is needed in order to exert the pharmacologically desired effect. In a review, Rader C. reports that the potent killing ability of the BiTE® format is attributed to (1) its small size that brings target and effector cells in close proximity enabling the formation of cytolytic synapses and (2) its monovalent engagement of the TCR complex preventing systemic activation of effector cells in the absence of target cells (Rader C, (2011), Blood, 117(17), pp. 4403-4404). In addition, the prior art describes that the BiTE® class of bispecific antibodies have typically 100-10,000 fold higher efficacy in tumor cell lysis relative to other CD3-bispecific formats and complete monoclonal IgG1 antibodies (Wolf et al (2005) Drug Discov Today 10(18), pp. 1237-1244). A complete antibody also forms part of the bispecific binding molecule of the invention. Mølhøj M. et al further showed that the smaller and monovalent BiTE® format (55 kDa) is much more potent than the larger, bivalent tandabs® format (around 115 kDa) (more than 2000-fold in certain assays) (Mølhøj M. et al, (2007), Mol Immunol, 44(8), pp. 1935-1943). The authors note that the monovalent binding of the BiTE® to CD3 contributes to the potent killing activity of the BiTE® molecules.

In summary, the prior art teaches that small, monovalent anti-CD3 x anti-tumor antigen bispecific antibody formats (e.g. the BiTE® format) exhibit a more potent redirected T cell killing activity than large, bivalent CD3 bispecific formats. The prior art thus discourages from using large, bivalent CD3 bispecific formats, because potent anti-tumoral killing is typically obtained with small, monovalent CD3 bispecific formats.

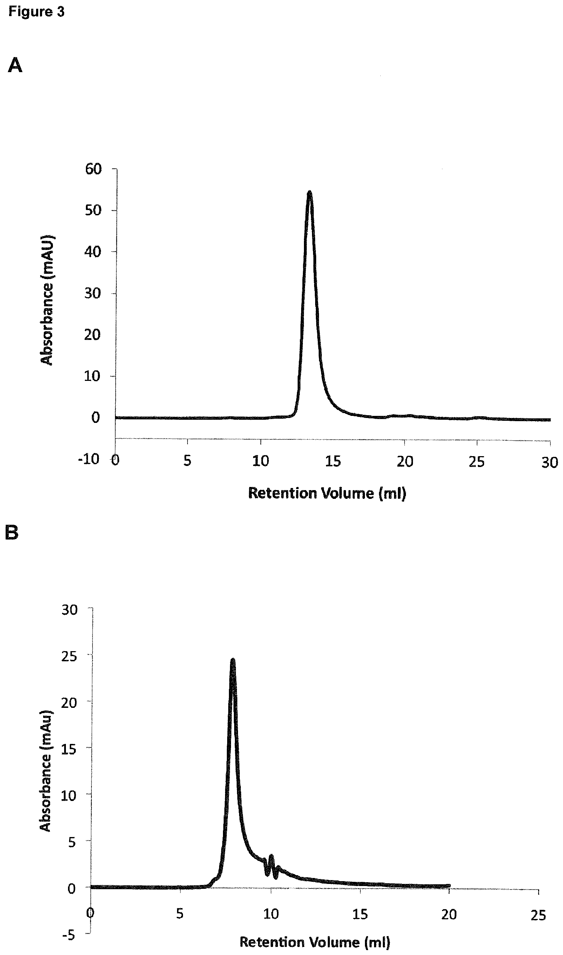

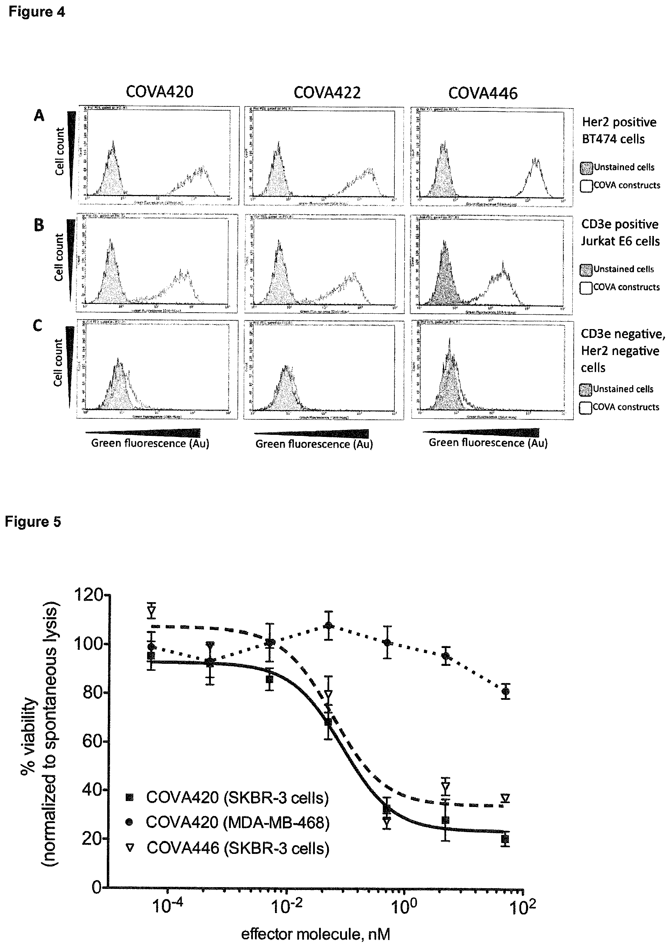

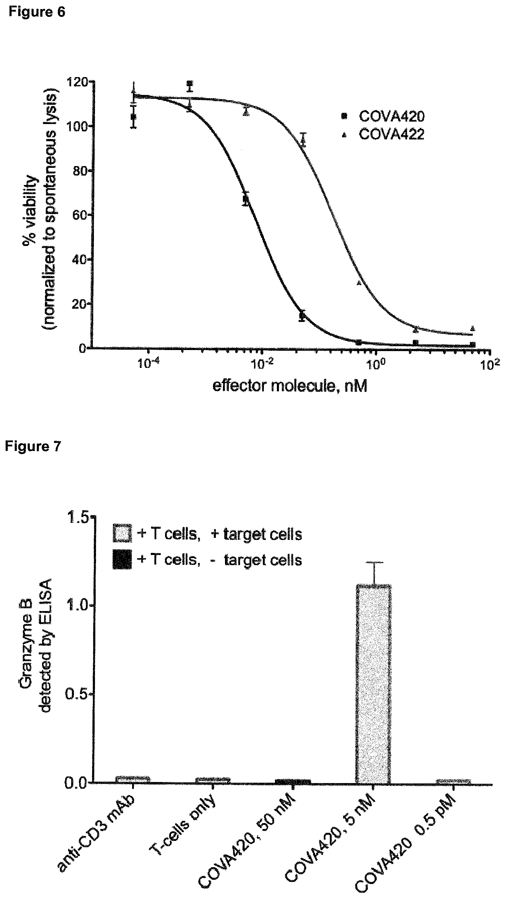

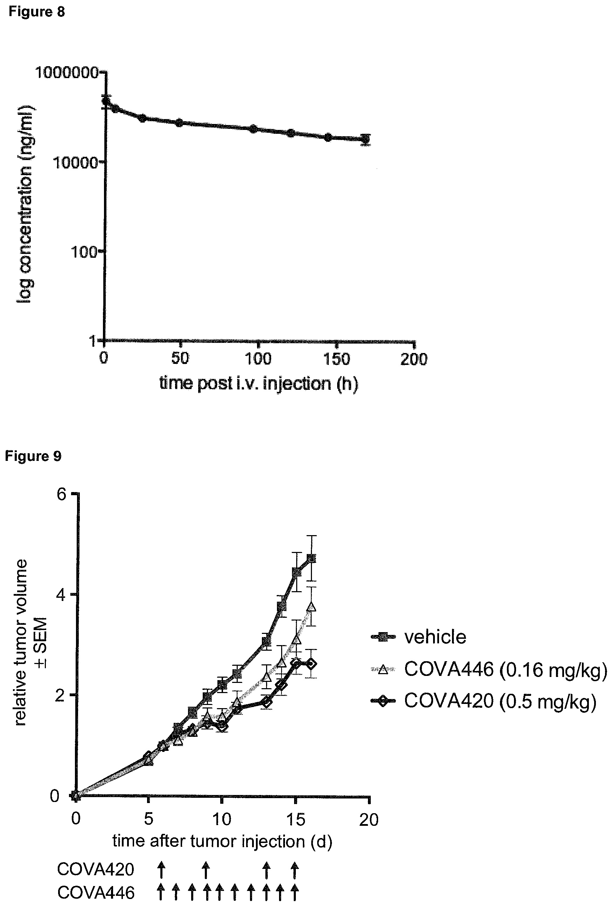

It was surprisingly found in connection with the present invention that a bispecific binding molecule consisting of a complete anti-CD3 antibody and two copies of a Fynomer binding to a tumor associated antigen is able to mediate potent redirected T-cell killing activity. Noteworthy, the bispecific binding molecule of the invention has a size of about 165kDa. The T-cell mediated cytotoxicity of the large bispecific binding molecule of the invention is in contrast to the above-discussed prior art teaching unexpectedly in a comparable range as typically seen for small, monovalent CD3 bispecific molecules; i.e. the BiTE® format. Comparative data evidencing the very potent anti-tumoral killing activity of both the CD3 bispecific binding molecule of the invention and the prior art monovalent CD3 bispecific molecules are provided in the Examples of this specification. More specifically, Examples of fusion proteins consisting of an anti-CD3 antibody and Fynomers binding to three different tumor associated antigens are shown. As shown in Example 1 and Figure 5, the CD3/HER2 bispecific molecule of the invention COVA420 (SEQ ID NOs: 163 and 164) shows a cytotoxic efficacy being as good as the cytotoxic efficacy of a small, monovalent, bispecific scFv control (SEQ ID NO: 167), both with $EC_{50}$ values in the double-digit pM range ($EC_{50}$ values of 87 pM and 60 pM, respectively). Example 2, which describes the EGFR/CD3 bispecific molecules of the invention COVA493 (SEQ ID NOs: 170 and 172), COVA494 (SEQ ID NOs: 171 and 198), COVA497 (SEQ ID NOs: 199 and 172), COVA499 (SEQ ID NOs: 171 and 200)) as well as Example 3 which describes the CD33/CD3 bispecific molecule of the invention COVA467 (SEQ ID NO: 2 and 175) additionally show the potent redirected T-cell killing activity of the bispecific molecules of the invention. In addition, Figure 6 shows that picomolar $EC_{50}$ values were obtained for both bispecific molecules of the invention, COVA420 (SEQ ID NOs: 163 and 164) and COVA422 (SEQ ID NOs: 165 and 166), in killing assay using enriched T-cells as mediators (8 pM and 175 pM, respectively). Moreover, as shown by the Examples herein, the Fynomer-antibody fusions of the invention show very favorable expression yields (in the two-digit mg/l range after transient transfection of CHO cells), very comparable to the bispecific scFv control antibody formats (SEQ ID NO: 167, SEQ ID NO: 173 and SEQ ID NO: 176). In addition, the Fynomer-antibody fusions of the invention have favorable biophysical properties and do not aggregate (Figure 3; Figure 11 and Figure 15.A), and exhibit a long, IgG-like half-life in vivo (Figure 8). The increased half-life directly translates its significant benefit for the patient since no continuous infusion will be necessary and/or longer intervals between successive treatments become possible, which also helps to reduce the cost burden for healthcare systems. After injections twice a week, COVA420 (SEQ ID NOs: 163 and 164) exhibited a significant tumor growth retardation in a tumor model in vivo, which was superior as compared to the scFv control molecule (COVA446 (SEQ ID NO: 167); daily injections), further demonstrating the potent anti-tumoral efficacy of COVA420 (Figure 9).

Moreover and as mentioned, because of their small size monovalent CD3 bispecific molecules are characterized by a short serum half life of only a few hours. It is thus often necessary to administer small size monovalent CD3 bispecific molecules by continuous intravenous infusion with portable mini-pumps which is not convenient for the patient to be treated. Advantageously, the bispecific molecules of the invention have about the same serum half-life as typically obtained with full-length antibodies (see Examples), which serum half-life is superior to the serum half life of monovalent CD3 bispecific molecules. Another major limitation of monovalent CD3 bispecific molecules is that they lack the constant region of an antibody which is associated with functional properties such as binding to $F_c$-receptors. The bispecific molecule of the invention comprises the constant region of an anti-CD3 antibody.

Hence, the CD3 bispecific binding molecule of the invention combines advantages of monovalent CD3 bispecific molecules and complete anti-CD3 antibodies.

Another advantage of the CD3 bispecific binding molecule of the invention over monovalent CD3 bispecific molecules, in particular over BiTE® antibodies is due to the well-known and above-discussed fact that many surface target antigens being overexpressed in cancer cells are also expressed - although at significantly lower level - on non-tumor cells. A bispecific molecule, which mediates potent redirected T-cell killing activity, ideally exerts its activity selectively on the cancer cells having high levels of the surface antigen and not on normal cells having low levels of the surface antigen.

Therefore, although BiTE® antibodies exhibit a high potency of redirected lysis of cells expressing the tumor antigen, one major drawback of BiTE® antibodies is that they are also to a considerable extent kill non-non-tumor cells expressing the tumor antigen at a lower level. For instance, Lutterbuese et al. reported that an anti-CD3/EGFR BiTE® showed severe signs of toxicity in monkeys at doses of 31 and 154 $\mu$g/kg/d within 56 hours after the start of infusion (Lutterbuese R. et al. (2010) PNAS, vol 107, no.28, pp. 12605-12610). Histopathological analysis of the animals receiving C-BiTE at 31 or 154 $\mu$g/kg/d showed signs of liver and kidney toxicity, which may be a result of redirected lysis of cells expressing low levels of EGFR in these organs. Furthermore, the authors note cell death in all tissues known to express EGFR, i.e., salivary glands, liver, stomach, small intestine, colon, rectum, kidneys, adrenal glands, ureter, urinary bladder, prostate and epididymides.

The non-selective killing of EGFR expressing cells by bispecific scFv antibody fragments is also confirmed in the Examples herein below. Breast cancer MDA-MB-468 cells considerably overexpress EGFR while colon cancer HT-29 cells have an expression level of EGFR which is almost equal to non-tumor cells (Yingjie et al (2005); Mol Cancer Ther, 4(3):435-442). As shown in Example 2.3, a bispecific anti-CD3/EGFR scFv antibody fragment (SEQ ID NO: 173) mediated a potent redirected lysis of cells with high EGFR expression levels (MDA-MB-468 cells) as well as low EGFR expression levels (HT-29 cells). The $EC_{50}$ values were 0.2 pM and 2.5 pM for the killing of MDA-MB-468 and HT-29 cells, respectively (i.e. only a factor 12.5 difference). As exemplified for EGFR, bispecific scFv antibody fragments are thus not able to mediate potent redirected T-cell killing activity selectively on the cells overexpressing the surface tumor antigen. A considerable undesired cell-killing side effect of non-tumor cells was observed by the inventors as well as in the above-discussed document Lutterbuese R. et al.

In contrast, it was surprisingly found that bispecific binding molecules consisting of an anti-CD3 antibody and a Fynomer binding to a tumor associated antigen are able to mediate potent redirected T-cell killing activity selectively to cells overexpressing the antigen, while cells with lower levels of the antigen were not killed even at high concentrations. As shown in Example 2.3 for four different bispecific binding molecules each consisting of an anti-CD3 antibody and two copies of a Fynomer binding to EGFR, the difference in killing activity between high and low EGFR-expressing cells ranged between a factor of 580 and > 3'500.

In addition, it is evident from Example 1.7 of the present invention that also bispecific scFv antibody fragments targeting CD3 and HER2-expressing cells non-selectively kill both cells expression HER2 at high levels as well as at low levels. Ovarian cancer SKOV-3 cells considerably overexpress HER2 while breast cancer MCF-7 cells have a much lower expression level of HER2 (Lattrich et al. (2008), Oncology Reports, 19:811-817). As shown in Example 1.7, a bispecific anti-CD3/HER2 scFv antibody fragment (SEQ ID NO: 167) mediated a potent redirected lysis of cells with high HER2 expression levels (SKOV-3 cells) as well as low HER2 expression levels (MCF-7 cells). The $EC_{50}$ values were 2.3 pM and 53 pM for the killing of SKOV-3 and MCF-7 cells, respectively (i.e. only a factor 23 difference). Hence, also bispecific scFv antibody fragments against CD3 and HER2 are not able to mediate potent redirected T-cell killing activity selectively on the cells overexpressing the surface tumor antigen. Again a considerable undesired cell-killing side effect of non-tumor cells was observed by the inventors thereby confirming the results obtained in connection with bispecific scFv antibody fragments against CD3 and EGFR.

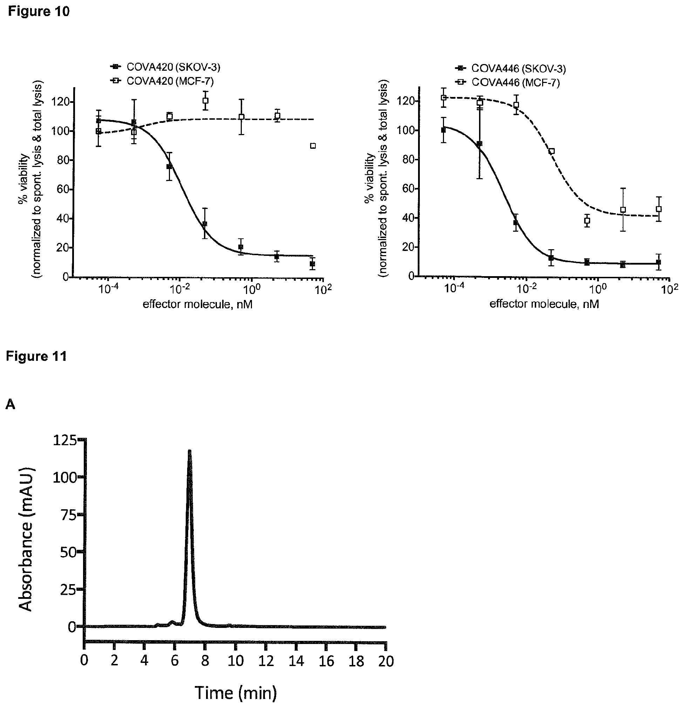

It was also again surprisingly found that bispecific binding molecules consisting of an anti-CD3 antibody and a Fynomer binding to a tumor associated antigen, namely HER2, are able to mediate potent redirected T-cell killing activity selectively to cells overexpressing the antigen, while cells with lower levels of the antigen were not killed even at high concentrations. As shown in Example 1.7 and Figure 10 for a bispecific binding molecules consisting of an anti-CD3 antibody and two copies of a Fynomer binding to HER2 (SEQ ID NOs 163 and 164), the difference in killing activity between high and low HER2-expressing cells was a factor of >4310.

The selective killing of tumor cells overexpressing the antigen while sparing cells with normal antigen levels directly translates into a significant benefit for the patient since the therapeutic window for CD3-based approaches is improved. Hence, the unwanted cell-killing side effect of non-tumor cells can be minimized or even avoided by using the bispecific constructs of the invention.

In accordance with a preferred embodiment of the invention, the antibody specifically binding to CD3 is teplizumab, otelixizumab, Cris-7 or SP34.

The antibodies teplizumab, otelixizumab (Chatenoud L. (2010) 6(3), pp. 149-157), Cris-7 (Alberola-IIa J. et al. (1991), J Immunol, 146(4), pp. 1085-1092) and SP34 are known from the prior art. Teplizumab (CAS-Nr: 876387-05-2), also called MGA031 and hOKT3-gamma1 (Ala-Ala), is a humanized, anti-CD3 monoclonal antibody. Teplizumab binds to an epitope of the CD3-epsilon chain expressed on mature T lymphocytes and, by doing so, may modulate the pathological immunologic responses underlying multiple autoimmune diseases. Specifically, teplizumab may inhibit unwanted effector T cells and enhance beneficial regulatory T cell functions, thus promoting immune tolerance. Otelixizumab (CAS Nr 881191-44-2), also known as TRX4, likewise targets the epsilon chain of CD3. Monoclonal antibody Cris-7 recognizes the CD3 antigen on mature human T-cells. The CD3 antigen is a protein complex composed of four distinct chains (CD3$\gamma$, CD3$\delta$ and two CD3$\epsilon$) that associate with the T cell receptor (TCR) and the $\zeta$-chain to generate an activation signal in T lymphocytes. The antibody can be obtained from BMA BIOMEDICALS. SP34 can be obtained from BD Biosciences.

In accordance with the present invention it is preferred that humanized versions of Cris-7 or SP34 are used as the antibody specifically binding to CD3.

Means and methods for humanizing antibodies are well-established in the art; see, for example Riechmann et al. (1988), Nature 332 (6162): 332-323 or Kashmiri et al., Methods 36 (1): 25-34.

The antibodies teplizumab and otelixizumab are antibodies with silenced effector functions. It is also preferred that a modified version of Cris-7 or SP34 having silenced effector functions is used as the antibody specifically binding to CD3. As further detailed herein above, it is most preferred that an antibody having silenced effector functions has mutations in L234 and L235, or, in positions D265 and P329, numbering according to EU index of Kabat. It has to be understood that is also preferred that a humanized version of Cris-7 or SP34 having silenced effector functions is employed as the antibody specifically binding to CD3.

In accordance with a preferred embodiment of the invention, the anti-CD3 antibody comprises SEQ ID NO: 2 and/or SEQ ID NO: 3, or comprises or consists of SEQ ID NO: 2 and SEQ ID NO: 3.

It has to be understood throughout this specification that SEQ ID NO: 2 and SEQ ID NO: 3 are the V$_H$ and V$_L$ region of an anti-CD3 antibody, respectively. In case the anti-CD3 antibody of the invention comprises the V$_H$ region of SEQ ID NO: 2 and not simultaneously the V$_L$ region of SEQ ID NO: 3 it is to be understood that the anti-CD3 antibody comprises the V$_L$ region of an anti-CD3 antibody other than SEQ ID NO: 3. Likewise, in case the anti-CD3 antibody of the invention comprises the V$_L$ region of SEQ ID NO: 3 and not simultaneously the V$_H$ region of SEQ ID NO: 2 it is to be understood that the anti-CD3 antibody comprises the V$_H$ region of an anti-CD3 antibody other than SEQ ID NO: 2.

In another preferred embodiment of the invention, the anti-CD3 antibody comprises SEQ ID NO: 171 and/or SEQ ID NO: 172, or comprises or consists of SEQ ID NO: 171 and SEQ ID NO: 172.

It has to be understood throughout this specification that SEQ ID NO: 171 and SEQ ID NO: 172 are the V$_H$ and V$_L$ region of an anti-CD3 antibody, respectively. In case the anti-CD3 antibody of the invention comprises the V$_H$ region of SEQ ID NO: 171 and not simultaneously the V$_L$ region of SEQ ID NO: 172 it is to be understood that the anti-CD3 antibody comprises the V$_L$ region of an anti-CD3 antibody other than SEQ ID NO: 172. Likewise, in case the anti-CD3 antibody of the invention comprises the V$_L$ region of SEQ ID NO: 172 and not simultaneously the V$_H$ region of SEQ ID NO: 171 it is to be understood that the anti-CD3 antibody comprises the V$_H$ region of an anti-CD3 antibody other than SEQ ID NO: 171.

As is evident from the Examples herein below an anti-CD3 antibody comprising SEQ ID NO: 2 and SEQ ID NO: 3 and an anti-CD3 antibody comprising SEQ ID NO: 171 and SEQ ID NO: 172 have been used to illustrate the invention.

In accordance with a further preferred embodiment of the invention, the binding molecule comprises two Fyn-SH3-derived polypeptides specifically binding to HER2, CD19, EGFR, CEA, EPHA2, melanoma-associated chondroitin sulfate proteoglycan, IGFR1, CD33, fibroblast-activating protein alpha, prostate stem cell antigen (PSCA), c-MET, Ep-CAM, or PSMA. Within this list EGFR, HER2 and CD33 are most preferred. In this list of three targets EGFR and HER2 are preferred, EGFR being most preferred.

Hence, preferred binding molecules of the invention comprise two Fyn-SH3-derived polypeptides specifically binding to HER2 and comprise SEQ ID NO: 2 and/or SEQ ID NO: 3; comprise two Fyn-SH3-derived polypeptides specifically binding to CD19 and comprise SEQ ID NO: 2 and/or SEQ ID NO: 3; comprise two Fyn-SH3-derived polypeptides specifically binding to EGFR and comprise SEQ ID NO: 2 and/or SEQ ID NO: 3; comprise two Fyn-SH3-derived polypeptides specifically binding to CEA and comprise SEQ ID NO: 2 and/or SEQ ID NO: 3; comprise two Fyn-SH3-derived polypeptides specifically binding to EPHA2 and comprise SEQ ID NO: 2 and/or SEQ ID NO: 3; comprise two Fyn-SH3-derived polypeptides specifically binding to melanoma-associated chondroitin sulfate proteoglycan and comprise SEQ ID NO: 2 and/or SEQ ID NO: 3; comprise two Fyn-SH3-derived polypeptides specifically binding to IGFR1 and comprise SEQ ID NO: 2 and/or SEQ ID NO: 3; comprise two Fyn-SH3-derived polypeptides specifically binding to CD33 and comprise SEQ ID NO: 2 and/or SEQ ID NO: 3; comprise two Fyn-SH3-derived polypeptides specifically binding to fibroblast-activating protein alpha and comprise SEQ ID NO: 2 and/or SEQ ID NO: 3; comprise two Fyn-SH3-derived polypeptides specifically binding to prostate stem cell antigen (PSCA) and comprise SEQ ID NO: 2 and/or SEQ ID NO: 3; comprise two Fyn-SH3-derived polypeptides specifically binding to c-MET and comprise SEQ ID NO: 2 and/or SEQ ID NO: 3; comprise two Fyn-SH3-derived polypeptides specifically binding to EpCAM and comprise SEQ ID NO: 2 and/or SEQ ID NO: 3; or comprise two Fyn-SH3-derived polypeptides specifically binding to PSMA and comprise SEQ ID NO: 2 and/or SEQ ID NO: 3.

Likewise, preferred binding molecules of the invention comprise two Fyn-SH3-derived polypeptides specifically binding to HER2 and comprise SEQ ID NO: 171 and/or SEQ ID NO: 172; comprise two Fyn-SH3-derived polypeptides specifically binding to CD19 and comprise SEQ ID NO: 171 and/or SEQ ID NO: 172; comprise two Fyn-SH3-derived polypeptides specifically binding to EGFR and comprise SEQ ID NO: 171 and/or SEQ ID NO: 172; comprise two Fyn-SH3-derived polypeptides specifically binding to CEA and comprise SEQ ID NO: 171 and/or SEQ ID NO: 172; comprise two Fyn-SH3-derived polypeptides specifically binding to EPHA2 and comprise SEQ ID NO: 171 and/or SEQ ID NO: 172; comprise two Fyn-SH3-derived polypeptides specifically binding to melanoma-associated chondroitin sulfate proteoglycan and comprise SEQ ID NO: 171 and/or SEQ ID NO: 172; comprise two Fyn-SH3-derived polypeptides specifically binding to IGFR1 and comprise SEQ ID NO: 171 and/or SEQ ID NO: 172; comprise two Fyn-SH3-derived polypeptides specifically binding to CD33 and comprise SEQ ID NO: 171 and/or SEQ ID NO: 172; comprise two Fyn-SH3-derived polypeptides specifically binding to fibroblast-activating protein alpha and comprise SEQ ID NO: 171 and/or SEQ ID NO: 172; comprise two Fyn-SH3-derived polypeptides specifically binding to prostate stem cell antigen (PSCA) and comprise SEQ ID NO: 171 and/or SEQ ID NO: 172; comprise two Fyn-SH3-derived polypeptides specifically binding to c-MET and comprise SEQ ID NO: 171 and/or SEQ ID NO: 172; comprise two Fyn-SH3-derived polypeptides specifically binding to EpCAM and comprise SEQ ID NO: 171 and/or SEQ ID NO: 172; or comprise two Fyn-SH3-derived polypeptides specifically binding to PSMA and comprise SEQ ID NO: 171 and/or SEQ ID NO: 172.

The above-discussed proteins and antigens are well characterized in the art for being or comprising surface target antigens expressed on cancer cells.

HER2 (Human Epidermal Growth Factor Receptor 2) also known as Neu, ErbB-2, CD340 (cluster of differentiation 340) or p185 is a protein that in humans is encoded by the *ERBB2* gene. HER2 is a 185-kDa receptor first described in 1984 (Schlechter et al (1984) Nature 312:513-516). HER2 is a member of the epidermal growth factor receptor (EGFR/ErbB) family. Human HER2 is represented by the NCBI reference: NP_004439. Amplification or over-expression of this gene has been shown to play an important role in the pathogenesis and progression of certain aggressive types of breast cancer and in recent years it has evolved to become an important biomarker and target of therapy for the disease. Other HER2 positive tumors include ovarian cancer and gastric cancer.

B-lymphocyte antigen CD19 or CD19 (Cluster of Differentiation 19), is a protein that in humans is encoded by the CD19 gene (NCBI reference: NP_001171569). Since CD19 is a hallmark of B-cells, the protein has been used to diagnose and as a target to treat cancers that arise from this type of cell - notably B-cell lymphoma, including B-ALL and non-hodgkin lymphoma (NHL).

The epidermal growth factor receptor (EGFR; ErbB-1; HER1 in humans) is the cell-surface receptor for members of the epidermal growth factor family (EGF-family) of extracellular protein ligands (NCBI reference: NP_005219). EGFR is known for its role in lung cancer, head and neck cancer and colorectal cancer.

Carcinoembryonic antigen (CEA) is a glycoprotein involved in cell adhesion (NCBI reference: AAA66186). It was found that serum from individuals with colorectal carcinoma, gastric carcinoma, pancreatic carcinoma, lung carcinoma and breast carcinoma, as well as individuals with medullary thyroid carcinoma, have higher levels of CEA than healthy individuals (above 2.5 ng/ml).

EPH receptor A2 (ephrin type-A receptor 2) is a protein (NCBI reference: AAH37166) that in humans is encoded by the EPHA2 gene. The role of EPHA2 in cancer is known, for example from Kinch MS, Carles-Kinch K (2003). Clin.

Exp. Metastasis 20 (1): 59-68. Studies have reported that EphA2 is overexpressed in many cancer types including cutaneous melanoma. EphA2 was reported to promote migration of glioma and prostate cancer cells in a ligand-independent manner (Udayakumar et al (2011), Oncogene 30: 4921-4929)

Chondroitin sulfate proteoglycan 4 (NCBI reference: NP_001888), also known as melanoma-associated chondroitin sulfate proteoglycan (MCSP) or neuron-glial antigen 2 (NG2), is a chondroitin sulfate proteoglycan that in humans is encoded by the *CSPG4* gene. CSPG4 plays a role in stabilizing cell-substratum interactions during early events of melanoma cell spreading on endothelial basement membranes. It represents an integral membrane chondroitin sulfate proteoglycan expressed by human malignant melanoma cells.

The Insulin-like Growth Factor 1 (IGF-1) Receptor (NCBI reference: P08069) is a protein found on the surface of human cells. It is a transmembrane receptor that is activated by a hormone called Insulin-like growth factor 1 (IGF-1) and by a related hormone called IGF-2. The IGF-1R is implicated in several cancers, including breast, prostate, and lung cancers.

CD33 or Siglec-3 is a transmembrane receptor expressed on cells of myeloid lineage. CD33 can be used as a target for the treatment for acute myeloid leukemia. Human CD33 is represented by the NCBI Reference Sequence: NP_001763.3) and has been described in the art, for example in Raponi et al. (2011) Leuk. Lymphoma 52(6):1098-1107.

Fibroblast activation protein (NCBI reference: AAB49652), alpha (FAP) also known as seprase or 170 kDa melanoma membrane-bound gelatinase is a protein that in humans is encoded by the FAP gene. It is selectively expressed in reactive stromal fibroblasts of epithelial cancers, granulation tissue of healing wounds, and malignant cells of bone and soft tissue sarcomas.

Prostate stem cell antigen (NCBI reference number: AAC39607) is a protein that in humans is encoded by the *PSCA* gene. This gene is up-regulated in a large proportion of prostate cancers and is also detected in cancers of the bladder and pancreas.

c-Met (MET or MNNG HOS Transforming gene) is a proto-oncogene that encodes a protein known as hepatocyte growth factor receptor (HGFR) (NCBI reference: NP_000236). Abnormal MET activation in cancer correlates with poor prognosis, where aberrantly active MET triggers tumor growth, formation of new blood vessels that supply the tumor with nutrients, and cancer spread to other organs. MET is deregulated in many types of human malignancies, including cancers of lung, kidney, liver, stomach, breast, and brain.

Epithelial cell adhesion molecule (EpCAM) is a protein that in humans is encoded by the *EPCAM* gene. EpCAM is a pan-epithelial differentiation antigen that is expressed on almost all carcinomas. Human EpCAM is represented by the UniProtKB/Swiss-Prot accession number: P16422.2 (publication date 22 Feb 2012) and has been described in the art, for example in Strnad et al. (1989) Cancer Res. 49(2):314-317.

Prostate-specific membrane antigen (PSMA) (also known as Glutamate carboxypeptidase II) (NCBI refernce: AAA60209) is a type 2 integral membrane glycoprotein found in prostate tissues and a few other tissues. It is a therapeutic target for prostate cancer.

As evident from the above, each of HER2, CD19, EGFR, CEA, EPHA2, melanoma-associated chondroitin sulfate proteoglycan, IGFR1, CD33, fibroblast-activating protein alpha, prostate stem cell antigen (PSCA), c-MET, Ep-CAM, or PSMA is involved in the pathogenesis and the treatment of particular cancer types. In case the binding molecule of the invention comprises two Fyn-SH3-derived polypeptides specifically binding to HER2, CD19, EGFR, CEA, EPHA2, melanoma-associated chondroitin sulfate proteoglycan, IGFR1, CD33, fibroblast-activating protein alpha, prostate stem cell antigen (PSCA), c-MET, EpCAM, or PSMA it preferred that the respective binding molecule is used for treatment of a cancer associated with the overexpression of HER2, CD19, EGFR, CEA, EPHA2, melanoma-associated chondroitin sulfate proteoglycan, IGFR1, CD33, fibroblast-activating protein alpha, prostate stem cell antigen (PSCA), c-MET, Ep-CAM, or PSMA, respectively.

The binding molecule of the invention may comprise two Fyn-SH3-derived polypeptides specifically binding to HER2, wherein (i) the first of said two polypeptides is linked to the first N-terminus of the two heavy chains of said antibody and the second of said two polypeptides is linked to the second N-terminus of the two heavy chains of said antibody (i.e. in other terms the first of copy of said two polypeptides is linked to the N-terminus of the first heavy chain of said antibody, and the second copy of said two polypeptides is linked to the N-terminus of the second heavy chain of said antibody), (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody.

In the context of the above options it is preferred that the binding molecule comprises two Fyn-SH3-derived polypeptides specifically binding to HER2, wherein the first of said two polypeptides is linked to the first N-terminus of the two heavy chains of said antibody and the second of said two polypeptides is linked to the second N-terminus of the two heavy chains of said antibody.

Specific binding to HER2 means that the polypeptides of the invention specifically bind to HER2 but not other related proteins such as EGFR. The "polypeptide specifically binding to HER2" is preferably a "polypeptide inhibiting the activity of HER2". Such a polypeptide has the capability to reduce or completely abolish the activity of HER2, which activity is described herein above in detail. In this regard it is preferred that inhibiting HER2 activity means inhibiting the binding of the receptor to its ligands. It is furthermore preferred with increasing preference that the polypeptide inhibits the activity of HER2 by at least 10%, at least 20%, at least 30%, at least 40%, at least 50%, at least 60%, at least 70%, at least 80%, at least 90%, at least 95%, or 100%. It is also preferred with increasing preference that the $IC_{50}$ value for the inhibition of HER2 by the polypeptides of the invention is 1000nM or less, 500 nM or less, 400nM or less, 300nM or less, 200nM or less, 100nM or less, or 75nM or less. Preferably, the polypeptides of the invention bind to HER2 with a $EC_{50}$ values as determined by FACS of $10^{-7}$ to $10^{-12}$ M, more preferably $10^{-8}$ to $10^{-12}$ M, most preferably $10^{-9}$ to $10^{-12}$ M.

The two Fyn-SH3-derived polypeptides specifically binding to HER2 preferably bind to the same epitopes of HER2. As is evident from the Examples herein below, the anti-HER2 binding Fynomer® C12 (SEQ ID NO: 4) has been used to illustrate the invention. It is therefore preferred that the binding molecule comprises two copies of SEQ ID NO: 4.

It is thus preferred that the Fyn-SH3-derived polypeptide specifically binding to HER2 has with increasing preference at least 65%, at least 70%, at least 75%, at least 80%, at least 85%, at least 90%, and at least 95% sequence identity to the amino acid sequence of SEQ ID NO: 4 (Fynomer® C12).

More preferably the Fyn-SH3-derived polypeptide specifically binding to HER2 has the following formula I, provided that said polypeptide has at least 85% sequence identity to the amino acid sequence of SEQ ID NO: 1, wherein the determination of identity excludes amino acid positions corresponding to amino acid positions 12 to 17 and 31 to 34 of SEQ ID NO: 1.

$GVTLFVALYDYX_1X_2X_3X_4X_5X_6X_7X_8X_9X_{10}LSFHKGEKFQILX_{11}X_{12}X_{13}X_{14}X_{15}X_{16}GX_{17}WWX_{18}ARSLTTGEX_{19}$

$GX_{20}IPS\ X_{21}YVAPVDSIQ$ \qquad (Formula I),

wherein $X_1$ to $X_7$ and $X_{11}$ to $X_{13}$ and $X_{17}$ to $X_{21}$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; and wherein $X_8$ to $X_{10}$ and $X_{14}$ to $X_{16}$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; or wherein one or more of $X_8$ to $X_{10}$ and $X_{14}$ to $X_{16}$ is absent (SEQ ID NO: 5 or preferably SEQ ID NO: 168, respectively). It is also preferred that the binding molecule of the invention comprises or consists of two Fyn-SH3-derived polypeptides of SEQ ID NO: 5, wherein the first of said two polypeptides is linked to the first N-terminus of the two heavy chains of said antibody and the second of said two polypeptides is linked to the second N-terminus of the two heavy chains of said antibody, and an anti-CD3 antibody of SEQ ID NO: 2 and SEQ ID NO: 3. It is likewise preferred that the binding molecule of the invention comprises or consists of two Fyn-SH3-derived polypeptides of SEQ ID NO: 168, wherein the first of said two polypeptides is linked to the first N-terminus of the two heavy chains of said antibody and the second of said two polypeptides is linked to the second N-terminus of the two heavy chains of said antibody, and an anti-CD3 antibody of SEQ ID NO: 2 and SEQ ID NO: 3.

Even more preferably,

$X_1$ is selected from: T, E, D, Q, Y, V, W, N, S, F or K

$X_2$ is selected from: S, A, R or T

$X_3$ is selected from: Y, R, H, T, N, V, W or S

$X_4$ is selected from: N, D, M, Y, R, P, E, L, H, T, G or F

$X_5$ is selected from: T, S, P, Q, R, K, G, A, D, M, N, L, F, Y or E

$X_6$ is selected from: R, M, K, D, F, T, G, H, S, P, N, Q, Y, L, A or P

$X_7$ is selected from: D, G, V, L, H, N, R, F, S or A

$X_8$ is selected from: G, S, E, D, P, Y or is absent

$X_9$ is selected from: Q, D, S, H or is absent

$X_{10}$ is selected from: D, V or is absent

$X_{11}$ is selected from: R, K, Q, N, S, G, W, M, H, L, F, E, T, P, A, D or V

$X_{12}$ is selected from: M, R, E, G, N, D, S, A, Q, F, P, K, Y, T, H, V, L or W

$X_{13}$ is selected from: E, W, P, R, K, S, V, N, D, H, G, T, Q, A, Y, L or M

$X_{14}$ is selected from: D, R, Q, S, A, N, P, I, H, T, Y, E, L, K, M, V, I, W or is absent

$X_{15}$ is selected from: G, S, I, L, A, V, T, E, D, Q, R, P, K, M, H, Y or is absent

$X_{16}$ is selected from: K, G, R, A, T, V, S, I, E, Q, P, D, N, H or is absent

$X_{17}$ is selected from: V, D, T, I or Y

$X_{18}$ is selected from: E, A, R, T or Q

$X_{19}$ is selected from: T, I or V

$X_{20}$ is selected from: Y, L or F and

$X_{21}$ is selected from: N or S (SEQ ID NO: 6).

It is furthermore preferred that the binding molecule of the invention comprises or consists of two Fyn-SH3-derived polypeptides of SEQ ID NO: 6, wherein the first of said two polypeptides is linked to the first N-terminus of the two heavy chains of said antibody and the second of said two polypeptides is linked to the second N-terminus of the two heavy chains of said antibody, and an anti-CD3 antibody of SEQ ID NO: 2 and SEQ ID NO: 3.

In another more preferred embodiment, the residues X in formula I are independently selected from: $X_1$ to $X_{10}$ is TSYNTRD (i.e. wherein $X_8$ to $X_{10}$ are absent; SEQ ID NO: 7); $X_{11}$ to $X_{16}$ is RMED (i.e. wherein $X_{14}$ to $X_{16}$ are absent; SEQ ID NO: 8); $X_{17}$ is V; $X_{18}$ is E; $X_{19}$ is T; $X_{20}$ is Y and/or $X_{21}$ is N. It is thus furthermore preferred that the binding molecule of the invention comprises or consists of two Fyn-SH3-derived polypeptides of formula I, wherein the residues X in formula I are independently selected from: $X_1$ to $X_{10}$ is TSYNTRD (i.e. wherein $X_8$ to $X_{10}$ are absent; SEQ ID NO: 7); $X_{11}$ to $X_{16}$ is RMED (i.e. wherein $X_{14}$ to $X_{16}$ are absent; SEQ ID NO: 8); $X_{17}$ is V; $X_{18}$ is E; $X_{19}$ is T; $X_{20}$ is Y and/or $X_{21}$ is N, wherein the first of said two polypeptides is linked to the first N-terminus of the two heavy chains of said antibody and the second of said two polypeptides is linked to the second N-terminus of the two heavy chains of said antibody, and an anti-CD3 antibody of SEQ ID NO: 2 and SEQ ID NO: 3. It is also preferred that the above-recited specific residues $X_{11}$ to $X_{21}$ are used in combination.

Preferably, the above-defined Fyn-SH3-derived polypeptide specifically binding to HER2 comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 4 retains or essentially retains the binding capacity of the binding (poly)peptide consisting of SEQ ID NO: 4, i.e. the strength (e.g. as determined via the dissociation constant) of binding to the respective target epitope is retained or essentially retained. The binding capacity of the Fyn-SH3-derived polypeptide specifically binding to HER2 is essentially retained if at least 60% of its binding capacity is retained. Preferably, at least 75% or more preferably at least 80% of its binding capacity is retained. More preferred is that at least 90% such as at least 95%, even more preferred at least 98% such as at least 99% of the binding capacity is retained. Most preferred is that the binding capacity is fully, i.e. to 100%, retained.

The Fyn SH3-derived polypeptide C12 (SEQ ID NO: 4) has a dissociation constant for its specific epitope on HER2 of $7 \times 10^{-8}$ M when determined by surface plasmon resonance (SPR). For this, the Fyn SH3-derived polypeptide is captured, for example by a His-tag specific antibody, which has been immobilized on a BIAcore sensor chip. Upon injection of the antigen containing the specific epitope, formation of the complex is monitored and kinetic association ($k_{on}$) and kinetic dissociation constants ($k_{off}$), or dissocation constants ($K_D$), are obtained by curve fitting using the BIAcore evaluation software. Accordingly, the binding capacity of the binding (poly)peptide comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 4 is essentially retained if a dissociation constant, preferably under the same conditions, for HER2-binding of at least $1 \times 10^{-6}$ M is retained, preferably at least $1 \times 10^{-7}$ M. Also in accordance with the invention are binding (poly)peptides having an increased binding capacity compared to the binding (poly)peptide consisting of SEQ ID NO:4, i.e. more than 100% activity. For example, envisaged herein are binding (poly)peptides having a dissociation constant of at least $1 \times 10^{-8}$ M, such as e.g. at least $1 \times 10^{-9}$ M, more preferably at least $1 \times 10^{-10}$ M, such as e.g. at least $1 \times 10^{-11}$ M, even more preferably at least $1 \times 10^{-12}$ M and most preferably at least $1 \times 10^{-13}$ M. Methods of assessing the binding capacity are well known in the art and include, without being limiting, surface Plasmon resonance (SPR) techniques or ELISA.

In another preferred embodiment of the present invention the Fyn SH3-derived polypeptide (i.e. the polypeptide without the anti-CD3 antibody) has sequence selected from the group consisting of SEQ ID NOs 4 and 9 to 159. The Fyn SH3-derived polypeptide is most preferably C12 (SEQ ID NO: 4). It is accordingly also preferred that the binding molecule of the invention comprises of two Fyn-SH3-derived polypeptides selected from the group consisting of SEQ ID NOs 4 and 9 to 159 (wherein SEQ ID NOs 4 is most preferred), wherein the first of said two polypeptides is linked to the first N-terminus of the two heavy chains of said antibody and the second of said two polypeptides is linked to the second N-terminus of the two heavy chains of said antibody, and an anti-CD3 antibody of SEQ ID NO: 2 and SEQ ID NO: 3.

In the above embodiments, wherein the Fyn-SH3-derived polypeptides are Fyn-SH3-derived polypeptides specifically binding to HER2 it is hence particularly preferred that the antibody specifically binding to CD3 comprises SEQ ID NO: 2 and/or 3.

According to another preferred embodiment of the invention, the antibody specifically binding to CD3 and the Fyn-SH3-derived polypeptide are linked by a linker. In case the bispecific binding molecule of the invention comprises or consists of two Fyn-SH3-derived polypeptides and an antibody specifically binding to CD3, the first Fyn-SH3-derived polypeptide is linked to the antibody specifically binding to CD3 by a linker and the second Fyn-SH3-derived polypeptide is linked to the antibody specifically binding to CD3 by a linker.

The term "linker" as used in accordance with the present invention relates to a sequel of amino acids (i.e. peptide linkers) as well as to non-peptide linkers, which separate the antibody specifically binding to CD3 and the Fyn-SH3-derived polypeptide of the invention. It will be appreciated that where the binding molecule of the present comprises a single polypeptide chain, the linker is a peptide linker.

The nature, i.e. the length and/or composition (such as e.g. amino acid sequence) of the linker may modify or enhance the stability and/or solubility of the bispecific molecule, it may enhance the flexibility of the resulting binding molecule and/or may improve the binding to the target antigen by reducing sterical hindrance. The length and composition of a linker depends on the composition of the respective binding (poly)peptides of the binding molecule of the invention. The skilled person is well aware of methods to test the suitability of different linkers. For example, the properties of the binding molecule can easily be tested by analysing its binding affinity when using different types of linkers. In addition, the respective measurements for each binding (poly)peptide alone may be carried out and compared to the binding affinity of the binding molecule. The stability of the resulting molecule can be measured by methods known in the art, such as e.g. using an ELISA method to determine the residual binding capacity of the molecule after incubation in human serum at 37°C for several time periods.

The term "non-peptide linker", as used in accordance with the present invention, refers to linkage groups having two or more reactive groups but excluding peptide linkers. For example, the non-peptide linker may be a polymer, such as e.g. polyethylene glycol, having reactive groups at both ends, which individually bind to reactive groups of the binding portions of the molecule of the invention, for example, an amino terminus, a lysine residue, a histidine residue or a cysteine residue. The reactive groups of the non-peptide linker include a hydroxyl group, an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide polymer may be the same or different. For example, the non-peptide polymer may have a maleimide group at one end and an aldehyde group at another end. Preferably, the polymer is polyethylene glycol.

Preference is given to peptidic (or peptide) linkers, more specifically to oligopeptides having a length from 2 to 30 amino acids. Use of a single amino acid is also deliberately envisaged. Preferred length ranges of peptide linkers are from 5 to 15 amino acids. Other preferred lengths are 3, 4, 6, 7, 8, 9, 10, 11, 12, 13, 14, 16, 17, 18, 19 or 20 amino acids.

Particularly preferred are linkers which are peptides which consist to at least 50%, at least 60%, at least 70%, at least 80%, at least 90% or 100% of small amino acids such as glycine, serine and alanine. Particularly preferred are linkers consisting of glycines and serines only. Most preferred are the linkers of SEQ ID NOs: 160 to 162 with special preference given to a linker being a peptide consisting of the sequence of SEQ ID NO: 162.

According to a more preferred embodiment of the invention, the linker is a peptide-linker. In accordance with even more preferred embodiment of the invention, the linker has a sequence selected from the group consisting of SEQ ID NOs: 162, 160 and 161. The linker of SEQ ID NO: 162 is most preferred.

In the above embodiments, where the Fyn-SH3-derived polypeptides are Fyn-SH3-derived polypeptides specifically binding to HER2 it is in addition particularly preferred that the antibody specifically binding to CD3 and the two Fyn-SH3-derived polypeptides specifically binding to HER2 are linked via a linker comprising SEQ ID NO: 162.

As is evident from the Examples herein below, an antibody comprising SEQ ID NO: 2 and SEQ ID NO: 3 has been fused to the Fynomer® C12 (SEQ ID NO: 4) via a linker having SEQ ID NO: 162. Fusions to the N-terminus (COVA420) as well as the C-terminus (COVA422) of the heavy chain of said antibody were generated.

According to a further preferred embodiment of the invention, the binding molecule comprises two Fyn-SH3-derived polypeptides specifically binding to EGFR, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody.

Specific binding to EGFR means that the polypeptides of the invention specifically bind to EGFR but not other related proteins such as HER2. The "polypeptide specifically binding to EGFR" is preferably a "polypeptide inhibiting the activity of EGFR". Such a polypeptide has the capability to reduce or completely abolish the activity of EGFR, which activity is described herein above in detail. In this regard it is preferred that inhibiting EGFR activity means inhibiting the binding of the receptor to its ligands. It is furthermore preferred with increasing preference that the polypeptide inhibits the activity of EGFR by at least 10%, at least 20%, at least 30%, at least 40%, at least 50%, at least 60%, at least 70%, at least 80%, at least 90%, at least 95%, or 100%. It is also preferred with increasing preference that the $IC_{50}$ value for the inhibition of EGFR by the polypeptides of the invention is 1000nM or less, 500 nM or less, 400nM or less, 300nM or less, 200nM or less, 100nM or less, or 75nM or less. Preferably, the polypeptides of the invention bind to EGFR with a $EC_{50}$ values as determined by FACS of $10^{-7}$ to $10^{-12}$ M, more preferably $10^{-8}$ to $10^{-12}$ M, most preferably $10^{-9}$ to $10^{-12}$ M.

The two Fyn-SH3-derived polypeptides specifically binding to EGFR bind preferably to the same epitopes of EGFR. As is evident from the Examples herein below, the anti-EGFR binding Fynomers® ER7L2D6 (SEQ ID NO: 169) and ER9L3D7 (SEQ ID NO: 187) have been used to illustrate the invention. It is therefore preferred that the binding molecule comprises two copies of SEQ ID NO: 169 or SEQ ID NO: 187.

It is preferred that the Fyn-SH3-derived polypeptide specifically binding to EGFR has with increasing preference at least 65%, at least 70%, at least 75%, at least 80%, at least 85%, at least 90%, and at least 95% sequence identity to the amino acid sequence of SEQ ID NO: 169 (Fynomer® ER7L2D6) or ER9L3D7 (SEQ ID NO: 187). It is also preferred that Fyn-SH3-derived polypeptide specifically binding to EGFR comprises or consists of an amino acid sequence selected from the group consisting of: (a)

GVTLFVAX$_1$YDYEARGLX$_2$RX$_3$FDLSFHKGEKFQILX$_4$X$_5$X$_6$X$_7$X$_8$GDWWEARSLTTGETGYIPSNYVAPVD SIQ   (Formula II),

wherein amino acid positions X$_1$ to X$_8$ may be any amino acid sequence, and one of amino positions X$_4$ to X$_8$ is optionally absent, and (b) an amino acid sequence which is at least 85% identical, preferably at least 90% and more preferably at least 95% to the amino acid sequence of (a), wherein the identity determination excludes amino acid positions X$_3$ to X$_7$ and provided that the amino acid sequence EARGLX$_2$RX$_3$F (SEQ ID NO: 209), X$_2$ being preferably N or H and X$_3$ being preferably M or L (SEQ ID NO: 204) in amino acid positions 12 to 20 of formula (II) is conserved.

More preferably the Fyn-SH3-derived polypeptide specifically binding to EGFR has the following formula II, provided that said polypeptide has at least 85% sequence identity to the amino acid sequence of SEQ ID NO: 1, wherein the determination of identity excludes amino acid positions corresponding to amino acid positions 12 to 17 and 31 to 34 of SEQ ID NO: 1. In this regard it is noted that in the following formula II amino acid positions 12 to 17 of SEQ ID NO: 1 correspond to amino acid positions 12 to 20 due to the addition of three amino acids into the RT loop of SEQ ID NO: 1.

GVTLFVAX$_1$YDYEARGLX$_2$RX$_3$FDLSFHKGEKFQILX$_4$X$_5$X$_6$X$_7$X$_8$GDWWEARSLTTGETGYIPSNYVAPVD SIQ   (Formula II),

wherein X$_1$ to X$_7$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; and wherein X$_8$ is each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; or wherein X$_8$ is absent (SEQ ID NO: 201 or preferably SEQ ID NO: 202, respectively). It is also preferred that the binding molecule of the invention comprises or consists of two Fyn-SH3-derived polypeptides of SEQ ID NO: 201, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 171 and SEQ ID NO: 172. It is likewise preferred that the binding molecule of the invention comprises or consists of two Fyn-SH3-derived polypeptides of SEQ ID NO: 202, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 171 and SEQ ID NO: 172.

Even more preferably,

X$_1$ is selected from: L or V

X$_2$ is selected from: N or H

X$_3$ is selected from: M or L

X$_4$ is selected from: S, N, Q, A, D, T or L

X$_5$ is selected from: F, T, R or P

$X_6$ is selected from: S, E, T or Q

$X_7$ is selected from: E, T, S or N and

$X_8$ is selected from: S or is absent (SEQ ID NO: 203).

It is thus furthermore preferred that the binding molecule of the invention comprises or consists of two Fyn-SH3-derived polypeptides of SEQ ID NO: 203, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 171 and SEQ ID NO: 172.

Preferably, the above-defined Fyn-SH3-derived polypeptide specifically binding to EGFR comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 169 or SEQ ID NO: 187 retains or essentially retains the binding capacity of the binding (poly)peptide consisting of SEQ ID NO: 169 or SEQ ID NO: 187, i.e. the strength (e.g. as determined via the EC50 value) of binding to the respective target epitope is retained or essentially retained. The binding capacity of the Fyn-SH3-derived polypeptide specifically binding to EGFR is essentially retained if at least 60% of its binding capacity is retained. Preferably, at least 75% or more preferably at least 80% of its binding capacity is retained. More preferred is that at least 90% such as at least 95%, even more preferred at least 98% such as at least 99% of the binding capacity is retained. Most preferred is that the binding capacity is fully, i.e. to 100%, retained.

The Fyn SH3-derived polypeptide ER7L2D6 (SEQ ID NO: 169) has a $EC_{50}$ value for binding in FACS experiments for its specific epitope on EGFR of 3.3 nM and ER9L3D7 (SEQ ID NO: 187) an $EC_{50}$ value of 15.0 nM. For the determination of these $EC_{50}$ values, purified Fynomers at concentrations between 0.46 nM and 333 nM were incubated with EGFR positive MDA-MB-468 cells. Bound Fynomers were detected by means of the mouse anti-myc tag antibody 9E10 (co-incubated with the Fynomer at a molar ratio of Fynomer: 9E10 of 3:2), then by an anti-mouse IgG Alex488 conjugate, followed by analysis on a cytometer. The mean fluorescence intensity values were determined and plotted versus the log10 values of the Fynomer concentration to determine the $EC_{50}$ values with the curve fitting software GraphPad prism. Accordingly, the binding capacity of the binding (poly)peptide comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 169 or 187 is essentially retained if a dissociation constant, preferably under the same conditions, for EGFR-binding of at least $1 \times 10^{-6}$ M is retained, preferably at least $1 \times 10^{-7}$ M. Also in accordance with the invention are binding (poly)peptides having an increased binding capacity compared to the binding (poly)peptide consisting of SEQ ID NO: 169 or SEQ ID NO: 187, i.e. more than 100% activity. For example, envisaged herein are binding (poly)peptides having a dissociation constant of at least $1 \times 10^{-8}$ M, such as e.g. at least $1 \times 10^{-9}$ M, more preferably at least $1 \times 10^{-10}$ M, such as e.g. at least $1 \times 10^{-11}$ M, even more preferably at least $1 \times 10^{-12}$ M and most preferably at least $1 \times 10^{-13}$ M. Methods of assessing the binding capacity are well known in the art and include, without being limiting, surface Plasmon resonance (SPR) techniques or ELISA.

It is also preferred that the binding molecule of the invention comprises or consists of two of the above-defined Fyn-SH3-derived polypeptides specifically binding to EGFR comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 169 or SEQ ID NO: 187, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 171 and SEQ ID NO: 172.

In another preferred embodiment of the present invention the Fyn SH3-derived polypeptide has a sequence selected from the group consisting of SEQ ID NOs 169 and 187 to 197. The Fyn SH3-derived polypeptide is most preferably ER7L2D6 (SEQ ID NO: 169). It is accordingly also preferred that the binding molecule of the invention comprises two Fyn-SH3-derived polypeptides selected from the group consisting of SEQ ID NOs 169 and 187 to 197 (wherein SEQ ID NO: 169 is most preferred), wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 171 and SEQ ID NO: 172.

In the above embodiments, wherein the Fyn-SH3-derived polypeptides are Fyn-SH3-derived polypeptides specifically binding to EGFR it is hence particularly preferred that the antibody specifically binding to CD3 comprises SEQ ID NO: 171 and/or 172.

As mentioned, according to another preferred embodiment of the invention, the antibody specifically binding to CD3 and the Fyn-SH3-derived polypeptide are linked by a linker. According to a more preferred embodiment of the invention, the linker is a peptide-linker. In accordance with even more preferred embodiment of the invention, the linker has a sequence selected from the group consisting of SEQ ID NOs: 162, 160 and 161.

In the above embodiments, where the Fyn-SH3-derived polypeptides are Fyn-SH3-derived polypeptides specifically binding to EGFR it is in addition particularly preferred that the antibody specifically binding to CD3 and the two Fyn-SH3-derived polypeptides specifically binding to EGFR are linked via a linker comprising SEQ ID NO: 162.

As is evident from the Examples herein below, an anti-CD3 antibody comprising SEQ ID NO: 171 and SEQ ID NO: 172 has been fused to the Fynomer® ER7L2D6 (SEQ ID NO: 169) via a linker having SEQ ID NO: 162. A fusion to the N-terminus of the light chain of said antibody was generated (i.e. COVA494 consisting of SEQ ID NO: 171 and 198).

Since the present disclosure provides for the first time Fyn-SH3-derived polypeptides specifically binding to EGFR, the present disclosure also relates in an embodiment to a polypeptide specifically binding to EGFR, wherein the polypeptide comprises or consists of an amino acid sequence selected from the group consisting of: (a)

$$\text{GVTLFVAX}_1\text{YDYEARGLX}_2\text{RX}_3\text{FDLSFHKGEKFQILX}_4\text{X}_5\text{X}_6\text{X}_7\text{X}_8\text{GDWWEARSLTTGETGYIPSNYVAPVD}$$
$$\text{SIQ} \quad \text{(Formula II)},$$

wherein amino acid positions $X_1$ to $X_8$ may be any amino acid sequence, and one of amino positions $X_4$ to $X_8$ is optionally absent, and (b) an amino acid sequence which is at least 85% identical, preferably at least 90% and more preferably at least 95% to the amino acid sequence of (a), wherein the identity determination excludes amino acid positions $X_3$ to $X_7$ and provided that the amino acid sequence EARGLX$_2$RX$_3$F (SEQ ID NO: 209), $X_2$ being preferably N or H and $X_3$ being preferably M or L (SEQ ID NO: 204) in amino acid positions 12 to 20 of formula (II) is conserved.

The phrase "the identity determination excludes amino acid positions $X_4$ to $X_7$ as used herein specifies that the calculation of the sequence identity with regard to formula (II) does not take into account amino acid positions $X_4$ to $X_7$ but is confined to the remainder of the amino acids positions of SEQ ID NO: 1. The condition "provided that the amino acid sequence EARGLX$_2$RX$_3$F (SEQ ID NO: 209), $X_2$ being preferably N or H and $X_3$ being preferably M or L (SEQ ID NO: 204) in amino acid positions 12 to 20 of formula (II) is conserved" as used herein specifies that no amino acids changes may be introduced into amino acid positions 12 to 20. In other terms, the amino acid positions corresponding to amino acids position 12 to 20 of formula (II) have the sequence EARGLX$_2$RX$_3$F (SEQ ID NO: 209), $X_2$ being preferably N or H and $X_3$ being preferably M or L (SEQ ID NO: 204) in all polypeptides falling under the ambit of the above embodiment of the disclosure and the following preferred examples thereof.

In a preferred embodiment of the Fyn-SH3-derived polypeptides specifically binding to EGFR

$X_1$ is selected from: L or V

$X_2$ is selected from: N or H

$X_3$ is selected from: M or L

$X_4$ is selected from: S, N, Q, A, D, T or L

$X_5$ is selected from: F, T, R or P

$X_6$ is selected from: S, E, T or Q

$X_7$ is selected from: E, T, S or N, and

$X_8$ is selected from: S or is absent (SEQ ID NO: 203)

In a more preferred embodiment of the Fyn-SH3-derived polypeptides specifically binding to EGFR, the polypeptide comprises or consists of an amino acid sequence selected from the group consisting of any one of SEQ ID NOs 169 and 187 to 197.

As it is shown in the Examples herein below, SEQ ID NOs 169 and 187 to 197 were found to specifically bind to EGFR.

The present disclosure furthermore relates in an embodiment to a fusion construct comprising the Fyn-SH3-derived polypeptides specifically binding to EGFR of the invention fused to a further compound, said further compound preferably not being an anti-CD3 antibody. The further compound is preferably a pharmaceutically active compound, a prodrug, a pharmaceutically acceptable carrier, a diagnostically active compound, a cell penetrating enhancer, and/or a compound modulating serum half-life. In more detail, the further compound is preferably selected from the group consisting of (a) a fluorescent dye, (b) a photosensitizer, (c) a radionuclide, (d) a contrast agent for medical imaging, (e) a cytokine, (f) a toxic compound, (g) a chemokine, (h) a pro-coagulant factor, (i) an enzyme for pro-drug activation, (k) an albumin binder, (I) an albumin, (m) an IgG binder, or (n) polyethylene glycol. Most preferably the further compound consists of or comprises an antibody light chain, an antibody heavy chain, an $F_c$ domain of an antibody, an antibody, or a combination thereof. Within this list an antibody is most preferred. The antibody is preferably fused to two copies the Fyn-SH3-derived polypeptides specifically binding to EGFR.

The present disclosure furthermore relates in an embodiment to nucleic acid molecule encoding the Fyn-SH3-derived polypeptides specifically binding to EGFR and, where applicable, the fusion constructs.

The present disclosure also relates in an embodiment to a pharmaceutical or diagnostic composition comprising the Fyn-SH3-derived polypeptides specifically binding to EGFR and/or the nucleic acid molecule encoding the Fyn-SH3-derived polypeptides specifically binding to EGFR and/or the fusion construct comprising the Fyn-SH3-derived polypeptides specifically binding to EGFR fused to a further compound. The pharmaceutical composition is preferably used in a method of treating or preventing cancer.

The present invention relates in a further preferred embodiment of the binding molecule that specifically binds to (i) CD3 and (ii) a surface target antigen on cancer cells to a binding molecule which comprises two Fyn-SH3-derived polypeptides specifically binding to CD33, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody.

In the context of the above options it is preferred that the binding molecule comprises two Fyn-SH3-derived polypeptides specifically binding to CD33, wherein the first of said two polypeptides is linked to the first C-terminus of the two light chains of said antibody and the second of said two polypeptides is linked to the second C-terminus of the two light chains of said antibody.

Specific binding to CD33 means that the polypeptides of the invention specifically bind to CD33 but not other related proteins such as other members of the Siglec family. The "polypeptide specifically binding to CD33" is preferably a "polypeptide inhibiting the activity of CD33". Such a polypeptide has the capability to reduce or completely abolish the activity of CD33, which activity is described herein above in detail. In this regard it is preferred that inhibiting CD33 activity means inhibiting the binding of the receptor to its ligands. It is furthermore preferred with increasing preference that the polypeptide inhibits the activity of CD33 by at least 10%, at least 20%, at least 30%, at least 40%, at least 50%, at least 60%, at least 70%, at least 80%, at least 90%, at least 95%, or 100%. It is also preferred with increasing preference that the $IC_{50}$ value for the inhibition of CD33 by the polypeptides of the invention is 1000nM or less, 500 nM or less, 400nM or less, 300nM or less, 200nM or less, 100nM or less, or 75nM or less. Preferably, the polypeptides of the invention bind to CD33 with a $EC_{50}$ value as determined by FACS of $10^{-7}$ to $10^{-12}$ M, more preferably $10^{-8}$ to $10^{-12}$ M, most preferably $10^{-9}$ to $10^{-12}$ M.

The two Fyn-SH3-derived polypeptides specifically binding to CD33 bind preferably to the same epitopes of CD33. As is evident from the Examples herein below, the anti-CD33 binding Fynomer® EE1L1B3 (SEQ ID NO: 174) has been used to illustrate the invention. It is therefore preferred that the binding molecule comprises two copies of SEQ ID NO: 174.

It is preferred that the Fyn-SH3-derived polypeptide specifically binding to CD33 has with increasing preference at least 65%, at least 70%, at least 75%, at least 80%, at least 85%, at least 90%, and at least 95% sequence identity to the amino acid sequence of SEQ ID NO: 174 (Fynomer® EE1L1B3). It is also preferred that the Fyn-SH3-derived polypeptides specifically binding to CD33 comprises or consists of an amino acid sequence selected from the group consisting of: (a) GVTLFVALYDYEALGAHELSFHKGEKFQIL$X_1X_2X_3X_4X_5X_6$GPFWEAHSLTTGETGWIPSNYVAPVDSIQ (Formula III), wherein amino acid amino acid positions $X_1$ to $X_6$ may be any amino acid sequence, and one or two of amino positions $X_1$ to $X_6$ is/are optionally absent, and (b) an amino acid sequence which is at least 85% identical, preferably at least 90% and more preferably at least 95% to the amino acid sequence of (a), wherein the identity determination excludes amino acid positions $X_1$ to $X_4$ and provided that the amino acid sequence EALGAHE (SEQ ID NO: 208) in amino acid positions 12 to 18 of formula (III) is conserved.

More preferably the Fyn-SH3-derived polypeptide specifically binding to CD33 has the following formula III, provided that said polypeptide has at least 85% sequence identity to the amino acid sequence of SEQ ID NO: 1, wherein the determination of identity excludes amino acid positions corresponding to amino acid positions 12 to 17 and 31 to 34 of SEQ ID NO: 1.

GVTLFVALYDYEALGAHELSFHKGEKFQILX$_1$X$_2$X$_3$X$_4$X$_5$X$_6$GPFWEAHSLTTGETGWIPSNYVAPVDSIQ (Formula III), wherein X$_1$ to X$_4$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; and wherein X$_5$ and X$_6$ are each independently selected from G, V, T, L, F, A, Y, D, S, H, K, E, Q, I, W, R, M, P, N and C; more preferably from G, V, T, L, F, A, Y, D, S, H, K, E, Q, I, W, R, M, P and N; or wherein X$_5$ and X$_6$ are absent (SEQ ID NO: 205 or preferably SEQ ID NO: 206, respectively). It is also preferred that the binding molecule of the invention comprises or consists of two Fyn-SH3-derived polypeptides of SEQ ID NO: 205, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 2 and SEQ ID NO: 3. It is likewise preferred that the binding molecule of the invention comprises or consists of two Fyn-SH3-derived polypeptides of SEQ ID NO: 206, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 2 and SEQ ID NO: 3.

Even more preferably,

X$_1$ is selected from: N, R, S, L, M, D or Y

X$_2$ is selected from: S, G, P, R, M or V

X$_3$ is selected from: L, T, G, S, V, R or A

X$_4$ is selected from: S, A, L, Q, D, V, K or E

X$_5$ is selected from: E, V, Q, L, A, R or is absent, and

X$_6$ is selected from: L, G or is absent (SEQ ID NO: 207).

It is furthermore preferred that the binding molecule of the invention comprises or consists of two Fyn-SH3-derived polypeptides of SEQ ID NO: 207, wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 2 and SEQ ID NO: 3.

Preferably, the above-defined Fyn-SH3-derived polypeptide specifically binding to CD33 comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 174 retains or essentially retains the binding capacity of the binding (poly)peptide consisting of SEQ ID NO: 174, i.e. the strength (e.g. as determined via the EC$_{50}$ value) of binding to the respective target epitope is retained or essentially retained. The binding capacity of the Fyn-SH3-derived polypeptide specifically binding to CD33 is essentially retained if at least 60% of its binding capacity is retained. Preferably, at least 75% or more preferably at least 80% of its binding capacity is retained. More preferred is that at least 90% such as at least 95%, even more preferred at least 98% such as at least 99% of the binding capacity is retained. Most preferred is that the binding capacity is fully, i.e. to 100%, retained.

The Fyn SH3-derived polypeptide EE1L1B3 (SEQ ID NO: 174) has an EC$_{50}$ value for binding in FACS experiments for its specific epitope on CD33 of 5.7 nM. For the determination of the EC$_{50}$ value, purified Fynomer at concentrations between 300 nM and 0.41 nM were incubated with CD33 positive U937 cells pretreated with Fc receptor blocking agents. Bound Fynomers were detected by means of the mouse anti-myc tag antibody 9E10 (co-incubated with the Fynomer at a molar ratio of Fynomer : 9E10 of 4:1), followed by an anti-mouse IgG Alex488 conjugate, followed by analysis on a cytometer. The mean fluorescence intensity values were determined and plotted versus the log10 values of the Fynomer concentration to determine the $EC_{50}$ values with the curve fitting software GraphPad prism. Accordingly, the binding capacity of the binding (poly)peptide comprising or consisting of an amino acid sequence having with increasing preference at least 65%, at least 70%, at least 75%, at least 80%, at least 85%, at least 90%, and at least 95% sequence identity to the amino acid sequence of SEQ ID NO: 174 is essentially retained if a dissociation constant, preferably under the same conditions, for CD33-binding of at least $1\times10^{-6}$ M is retained, preferably at least $1\times10^{-7}$ M. Also in accordance with the invention are binding (poly)peptides having an increased binding capacity compared to the binding (poly)peptide consisting of SEQ ID NO: 174, i.e. more than 100% activity. For example, envisaged herein are binding (poly)peptides having a dissociation constant of at least $1\times10^{-8}$ M, such as e.g. at least $1\times10^{-9}$ M, more preferably at least $1\times10^{-10}$ M, such as e.g. at least $1\times10^{-11}$ M, even more preferably at least $1\times10^{-12}$ M and most preferably at least $1\times10^{-13}$ M. Methods of assessing the binding capacity are well known in the art and include, without being limiting, surface Plasmon resonance (SPR) techniques or ELISA.

In another preferred embodiment of the present invention the Fyn SH3-derived polypeptide has a sequence selected from the group consisting of SEQ ID NOs 174 and 177 to 186. The Fyn SH3-derived polypeptide is most preferably EE1L1B3 (SEQ ID NO: 174). It is accordingly also preferred that the binding molecule of the invention comprises two Fyn-SH3-derived polypeptides selected from the group consisting of SEQ ID NOs 174 and 177 to 186 (wherein SEQ ID NOs 174 is most preferred), wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody, and an anti-CD3 antibody of SEQ ID NO: 2 and SEQ ID NO: 3.

In the above embodiments, wherein the Fyn-SH3-derived polypeptides are Fyn-SH3-derived polypeptides specifically binding to CD33 it is hence particularly preferred that the antibody specifically binding to CD3 comprises SEQ ID NO: 2 and/or 3.

According to another preferred embodiment of the invention, the antibody specifically binding to CD3 and the Fyn-SH3-derived polypeptide are linked by a linker. As mentioned, according to a more preferred embodiment of the invention, the linker is a peptide-linker. In accordance with even more preferred embodiment of the invention, the linker has a sequence selected from the group consisting of SEQ ID NOs: 162, 160 and 161.

In the above embodiments, where the Fyn-SH3-derived polypeptides are Fyn-SH3-derived polypeptides specifically binding to CD33 it is in addition particularly preferred that the antibody specifically binding to CD3 and the two Fyn-SH3-derived polypeptides specifically binding to CD33 are linked via a linker comprising SEQ ID NO: 162.

As is evident from the Examples herein below, an antibody comprising SEQ ID NO: 2 and SEQ ID NO: 3 has been fused to the EE1L1B3 (SEQ ID NO: 174) via a linker having SEQ ID NO: 162. A fusion to the C-terminus of the light chain of said antibody was generated (i.e. COVA467 consisting of SEQ ID NOs: 2 and 175).

Since the present disclosure provides for the first time Fyn-SH3-derived polypeptides specifically binding to CD33, the present disclosure further relates in an embodiment to a polypeptide specifically binding to CD33, wherein the polypeptide comprises or consists of an amino acid sequence selected from the group consisting of: (a) GVTLFVALYDYEALGAHELSFHKGEKFQILX$_1$X$_2$X$_3$X$_4$X$_5$X$_6$GPFWEAHSLTTGETGWIPSNYVAPVDSIQ (Formula III), wherein amino acid amino acid positions $X_1$ to $X_6$ may be any amino acid sequence, and one or two of amino positions $X_1$ to $X_6$ is/are optionally absent, and (b) an amino acid sequence which is at least 85% identical, preferably at least 90% and more preferably at least 95% to the amino acid sequence of (a), wherein the identity determination excludes amino acid positions $X_1$ to $X_4$ and provided that the amino acid sequence EALGAHE (SEQ ID NO: 208) in amino acid positions 12 to 18 of formula (III) is conserved.

The phrase "the identity determination excludes amino acid positions $X_1$ to $X_4$ as used herein specifies that the calculation of the sequence identity with regard to formula (III) does not take into account amino acid positions $X_1$ to $X_4$ but is confined to the remainder of the amino acids positions of SEQ ID NO: 1. The condition "provided that the amino acid sequence EALGAHE (SEQ ID NO: 208) in amino acid positions 12 to 18 of formula (III) is conserved" as used herein specifies that no amino acids changes may be introduced into amino acid positions 12 to 18. In other terms, the amino acid positions corresponding to amino acids position 12 to 18 of formula (III) have the sequence EALGAHE (SEQ ID NO: 208) in all polypeptides falling under the ambit of the above embodiment of the disclosure and the following preferred examples thereof.

In a preferred embodiment of the Fyn-SH3-derived polypeptides specifically binding to CD33 $X_1$ is selected from: N, R, S, L, M, D or Y

$X_2$ is selected from: S, G, P, R, M or V

$X_3$ is selected from: L, T, G, S, V, R or A

$X_4$ is selected from: S, A, L, Q, D, V, K or E

$X_5$ is selected from: E, V, Q, L, A, R or is absent, and

$X_6$ is selected from: L, G or is absent (SEQ ID NO: 207).

In a more preferred embodiment of the Fyn-SH3-derived polypeptides specifically binding to CD33, the polypeptide comprises or consists of an amino acid sequence selected from the group consisting of any one of SEQ ID NOs 174 and 177 to 186.

As it is shown in the Examples herein below, SEQ ID NOs 174 and 177 to 186 were found to specifically bind to CD33.

The present disclosure furthermore relates in an embodiment to a fusion construct comprising the Fyn-SH3-derived polypeptides specifically binding to CD33 fused to a further compound, said further compound preferably not being an anti-CD3 antibody. The further compound is preferably a pharmaceutically active compound, a prodrug, a pharmaceutically acceptable carrier, a diagnostically active compound, a cell penetrating enhancer, and/or a compound modulating serum half-life. In more detail, the further compound is preferably selected from the group consisting of (a) a fluorescent dye, (b) a photosentisizer, (c) a radionuclide, (d) a contrast agent for medical imaging, (e) a cytokine, (f) a toxic compound, (g) a chemokine, (h) a pro-coagulant factor, (i) an enzyme for pro-drug activation, (k) an albumin binder, (l) an albumin, (m) an IgG binder, or (n) polyethylene glycol. Most preferably the further compound consists of or comprises an antibody light chain, an antibody heavy chain, an $F_c$ domain of an antibody, an antibody, or a combination thereof. Within this list an antibody is most preferred. The antibody is preferably fused to two copies the Fyn-SH3-derived polypeptides specifically binding to CD33.

The present disclosure furthermore relates in an embodiment to nucleic acid molecule encoding the Fyn-SH3-derived polypeptides specifically binding to CD33, and, where applicable, the fusion constructs.

The present disclosure furthermore relates in an embodiment to a pharmaceutical or diagnostic composition comprising the Fyn-SH3-derived polypeptides specifically binding to CD33 and/or the nucleic acid molecule encoding the Fyn-SH3-derived polypeptides specifically binding to CD33/or the fusion construct comprising the Fyn-SH3-derived polypeptides specifically binding to CD33 fused to a further compound. The pharmaceutical composition is preferably used in a method of treating or preventing cancer.

According to another preferred embodiment of the invention the bispecific binding molecule of the invention comprises SEQ ID NOs 163 and/or 164, or comprises or consists of SEQ ID NOs 163 and 164.

As is evident from the Examples herein below, the bispecific binding molecule consisting of SEQ ID NOs 163 and 164 (COVA420) has been generated to illustrate the invention. The compound COVA420 described in the Examples herein below consists of an antibody of SEQ ID NO: 2 and 3 wherein a copy of the Fyn-SH3-derived polypeptides specifically binding to HER2 of SEQ ID NO: 4 is linked to the first and second N-terminus of the two heavy chains of said antibody via the linker having SEQ ID NO: 162. Among the compounds targeting HER2 provided in the Examples, COVA420 showed the highest expression yields, the lowest $EC_{50}$ values and optimal tumor cell selectivity, hence the best tumor cell killing properties.

According to a preferred embodiment of the invention the bispecific binding molecule comprises SEQ ID NOs 171 and/or 198.

As is evident from the Examples herein below, an anti CD-3 antibody comprising SEQ ID NO: 171 and SEQ ID NO: 172 has been fused to the Fynomer® ER7L2D6 (SEQ ID NO: 169) via a linker having SEQ ID NO: 162. In more detail, the fusion is to the N-terminus of the light chain of said antibody thereby generating the compound COVA494 (SEQ ID NO: 171 and 198). Among the compounds targeting EGFR provided in the Examples, COVA494 had favorable biophysical properties and showed the best tumor selectivity, and hence the best tumor cell killing properties.

According to a further preferred embodiment of the invention the bispecific binding molecule comprises SEQ ID NOs 2 and/or 175.

As is evident from the Examples herein below, an antibody comprising SEQ ID NO: 2 and SEQ ID NO: 3 has been fused to the EE1L1B3 (SEQ ID NO: 174) via a linker having SEQ ID NO: 162. In more detail, the fusion is to the C-terminus of the light chain of said antibody thereby generating the compound COVA467 (SEQ ID NOs: 2 and 175). Said compound showed favorable biophysical properties and tumor cell killing properties.

In another embodiment the invention relates to a nucleic acid molecule encoding the binding molecule of the invention.

Since the binding molecule of the invention may be a complex of one (or more) copies of two distinct molecules, the constituents of the binding molecule of the invention may be encoded by either one or two (or more than two) nucleic acid molecules. In a preferred embodiment, one nucleic acid molecule encodes the complete single chain molecule.

In a further embodiment the invention relates to a vector comprising the nucleic acid molecule of the invention.

Nucleic acids of the invention can be DNA, RNA, PNA and any other analogues thereof. The vectors and isolated cells, in particular host cells, may be any conventional type that suits the purpose, e.g. production of polypeptides and/or fusion proteins of the invention, therapeutically useful vectors and isolated cells, e.g. for gene therapy. The skilled person will be able to select those nucleic acids, vectors and isolated cells from an abundant prior art and confirm their particular suitability for the desired purpose by routine methods and without undue burden.

Typically, the nucleic acid is operably linked to a promoter, preferably linked to a promoter selected from the group of prokaryotic promoters comprising T5 promoter/lac operator element, T7 promotor/lac operator element, or from the group of eukaryotic promoters comprising hEF1-HTLV, CMV enh/hFerL promoter.

It is also understood that a vector of the invention is one comprising a nucleic acid of the invention and preferably being capable of producing a polypeptide or fusion protein of the invention. Preferably such a vector is selected from the group consisting of pQE vectors, pET vectors , pFUSE vectors, pUC vectors, YAC vectors, phagemid vectors, phage vectors, vectors used for gene therapy such as retroviruses, adenoviruses, adeno-associated viruses.

The invention also relates in an embodiment to a host cell or a non-human host carrying the vector of the invention.

Said host or host cell may be produced by introducing the vector of the invention into a host or host cell, which upon its presence mediates the expression of the binding molecule encoded by the vector.

In accordance with the present invention, the host may be a transgenic non-human animal transfected with and/or expressing the vector of the present invention. In a preferred embodiment, the transgenic animal is a non-human mammal, e.g. a hamster, cow, cat, pig, dog or horse.

Preferred is a host cell, such as an isolated cell which may be part of a cell culture.

Suitable prokaryotic host cells comprise e.g. bacteria of the genera *Escherichia, Bacillus, Streptomyces* and *Salmonella typhimurium.* Suitable eukaryotic host cells are e.g. fungal cells, inter alia, yeasts such as *Saccharomyces cerevisiae* or *Pichia pastoris* or insect cells such as *Drosophila* S2 and *Spodoptera* Sf9 cells and plant cells as well as mammalian cells. Appropriate culture mediums and conditions for the above-described host cells are known in the art.

Mammalian host cells include without being limiting human Hela, HEK293, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. The binding molecule of the invention can be expressed in stable cell lines that contain the gene construct encompassing the nucleic acid molecule or the vector of the invention integrated into a chromosome.

In another preferred embodiment, said cell is a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

A further embodiment of the invention relates to a pharmaceutical composition comprising the binding molecule of the invention, the nucleic acid molecule of the invention, the vector of the invention, and/or the host cell or non-human host of the invention.

Pharmaceutical compositions of the invention may be manufactured in any conventional manner. In effecting treatment of a subject suffering from the diseases indicated below, at least one compound of the present invention can be administered in any form or mode which makes the active agent bioavailable in an effective amount, including oral or parenteral routes. For example, compositions of the present invention can be administered subcutaneously, intramuscularly, intravenously, intraperitoneally, by inhalation and the like. Preference is given to subcutaneous injection, preferably weekly or biweekly or once per month.

One skilled in the art in the field of preparing formulations can readily select the proper form and mode of administration depending upon the particular characteristics of the product selected, the disease or condition to be treated, the stage of the disease or condition and other relevant circumstances (see, e.g. Remington: The Science and Practice of Pharmacy, 22nd ed., Pharmaceutical Press, (2012) (1990)).

A suitable carrier or excipient may be a liquid material which can serve as a vehicle or medium for the active ingredient. Suitable carriers or excipients are well known in the art and include, for example, stabilizers, antioxidants, pH-regulating substances, controlled-release excipients, etc. A composition according to the invention may be provided in lyophilized form. For immediate administration it is dissolved in a suitable aqueous carrier, for example sterile water for injection or sterile buffered physiological saline. If it is desirable to produce larger volumes for administration by infusion rather than as a bolus injection, it is advantageous to incorporate human serum albumin or the patient's own heparinised blood into the solvent at the time of final formulation. Alternatively, the formulation can be administered subcutaneously. The presence of an excess of a physiologically inert protein such as human serum albumin prevents loss of the pharmaceutically effective polypeptide by adsorption onto the walls of the container and tubing used for the infusion solution. If albumin is used, a suitable concentration is from 0.5 to 4.5% by weight of the saline solution.

The amount and mode of administration of the inventive compounds, i.e., fusion proteins, bispecific constructs, nucleic acids, vectors, and isolated cells for treating and/or preventing a disease or medical condition, will, of course, vary depending upon the particular compound of the invention, the individual patient group or patient, the presence of further medically active compounds and the nature and severity of the condition being treated.

However, it is presently preferred that for prophylactic and/or therapeutic use dosages of about 0.0001 mg to about 20 mg per kilogram body weight, preferably about 0.001 mg to about 5 mg per kilogram body weight should be administered.

Preferably, the frequency of administration for prophylactic and/or therapeutic uses lies in the range of about twice per week up to about once every 3 months, preferably about weekly up to about once every 10 weeks, more preferably once a week up to once per month.

In a preferred embodiment the pharmaceutical composition of the invention is for use in a method of treating or preventing cancer.

As explained in greater detail herein above, CD3 is bona fide target for the redirected T-cell killing for the treatment of various cancer types. Furthermore, specificity and effectivity of the claimed bispecific binding molecules is achieved by targeting a surface target antigen, in particular EGFR, HER2 or CD33, on cancer cells.

In a more preferred embodiment of the pharmaceutical composition of the invention said cancer is selected from the group consisting of breast cancer, hematological malignancies such as NHL, B-ALL, CML, other cancers as prostate cancer, melanoma, lung cancer, gastric cancer, ovarian cancer, NHL, B-ALL, AML, head-and-neck cancer, colorectal cancer, pancreatic cancer, thyroid cancer, glioma, bladder cancer, kidney cancer, liver cancer, or malignant ascites. Within this list breast cancer is most preferred.

A number of non-limiting examples of surface target antigens on cancer cells have been exemplified herein above. As is evident from the specification herein above these surface target antigens on cancer cells are involved in the cancer-types listed in the above embodiment of the invention.

In a more preferred embodiment of the pharmaceutical composition of the invention said cancer expresses the surface target antigen being specifically bound by the binding molecule of the invention, being specifically bound by the binding molecule encoded the nucleic acid molecule of the invention, being specifically bound by the binding molecule expressed by the vector of the invention, and/or being specifically bound by the binding molecule expressed by the host cell or non-human host of the invention in an amount being at least two times higher as the amount expressed on non-tumor cells.

Preferably, the antigen is expressed on cancer cells in an at least five times higher amount, such as e.g. an at least 10-times higher amount, even more preferably an at least 100-time higher amount, such as e.g. an at least 1000-times higher amount and most preferably an at least 10.000-times higher amount as compared to non-tumor cells.

The present disclosure also provides a method of producing the bispecific binding molecule, which method comprises (a) culturing the above defined host cells and (b) isolating the produced bispecific binding molecule.

Preferred isolated cells are host cells including CHO cells. Conditions for culturing may be chosen depending on the particular host cells used which can be done by the skilled person without further ado.

Isolation of the desired product, i.e. the bispecific binding molecule of the invention, can be effected by purification methods, which may be preceded by breaking up said isolated cells. Suitable methods are well-known in the art and at the skilled person's disposal.

As regards the embodiments and preferred features characterized in this specification, in particular in the claims, it is intended that each embodiment mentioned in a dependent claim or preferred feature is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise. Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed. Also in those cases where a dependent claim does not directly refer back to a higher-ranking claim (being an independent and/or dependent claims), it is understood that any possible combination of subject-matter covered by the claims is considered to be explicitly disclosed by the present application. For example, in case of an independent claim 1, and a dependent claim 3 referring back to dependent claim 2 which in turn refers back to independent claim 1, the combination of the subject-matter of claims 3 and 1 is clearly and unambiguously disclosed. The above considerations apply *mutatis mutandis* to all attached claims, embodiments and preferred features characterized in this specification.

Finally the disclosure relates to treating a patient suffering from any of the diseases referred to herein above by administering one or more of the compounds or pharmaceutical compositions referred to herein above.

The figures show:

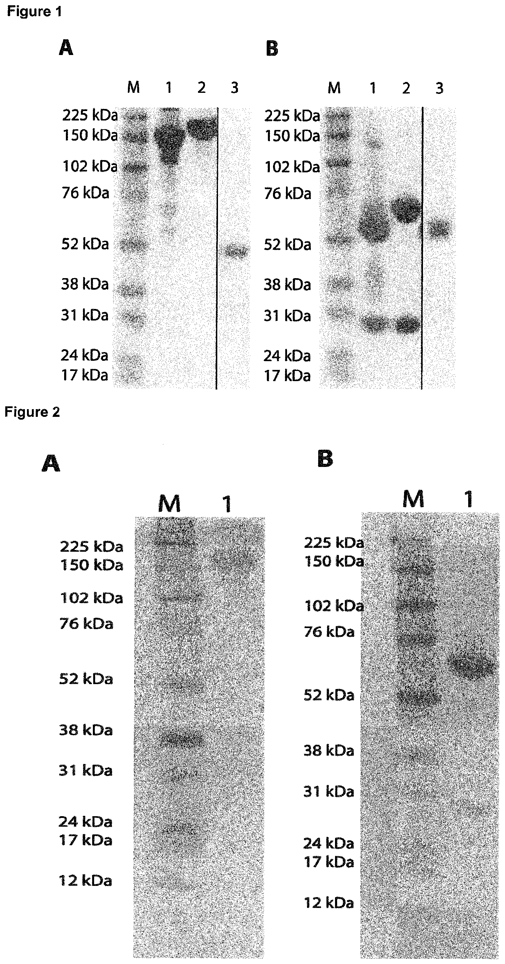

Fig. 1    shows the SDS-PAGE characterization of Fynomer®-antibody fusion proteins of the invention and control proteins. A: gel run under non-reducing conditions; B: gel run under reducing conditions. Lane M: molecular weight standard; Lane 1: anti-CD3 antibody (SEQ ID NOs 2 and 3) ; Lane 2: COVA420 (SEQ ID NOs 163 and 164) ; Lane 3: COVA446 (SEQ ID NO: 167).

Fig. 2    shows the SDS-PAGE characterization of an Fynomer®- antibody fusion proteins of the invention. A: gel run under non-reducing conditions; B: gel run under reducing conditions. Lane M: molecular weight standard; Lane 1: COVA422 (SEQ ID NOs: 165 and 166)

Fig. 3    shows size exclusion (SEC) profiles of Fynomer®-antibody fusion proteins. A: SEC profile of COVA420 (SEQ ID NOs 163 and 164) on FPLC system; B: SEC profile of COVA422 (SEQ ID NOs: 165 and 166) on HPLC system

Fig. 4    shows the flow cytometric binding analysis of Fynomer®-antibody fusion proteins COVA420 (SEQ ID NOs: 163 and 164); COVA422 (SEQ ID NOs 165 and 166), and the bispecific scFv-control COVA446 (SEQ ID NO: 167) on HER2 positive cells (Panel A) CD3 positive cells (Panel B) and cell that do not express HER2 nor CD3 (Panel C). Signals are compared to the background signal obtained with the secondary detection antibody only (grey shaded histograms).

Fig. 5    shows the redirected cell kill activity of COVA420 (SEQ ID NOs: 163 and 164) and the bispecific anti-CD3 x anti-HER2 control in single chain Fv format (COVA446, SEQ ID NO: 167) on HER2 positive SKBR-3 tumor cells. In addition, the absence of any kill activity of COVA420 on HER2 negative MDA-MB-468 cells is shown, demonstrating the specific kill activity towards HER2 positive cells. PBMCs were used as effector cells.

Fig. 6    shows the redirected cell kill activity of COVA420 (SEQ ID NOs: 163 and 164) and COVA422 (SEQ ID NOs 165 and 166) on HER2 positive SKOV-3 tumor cells using CD8+ enriched T-cells as effector cells.

Fig. 7    depicts the release of Granzyme B into the cell culture supernatant upon incubation of indicated antibody Fynomer® fusion proteins in the presence and absence of CD8+ enriched T-cells.

Fig. 8    shows the serum concentrations of COVA420 (SEQ ID NOs: 163 and 164), after intravenous injection in mice.

Fig. 9    shows the anti-tumor activity of COVA420 (SEQ ID NOs: 163 and 164) in an in vivo SKOV-3 xenograft model reconstituted with activated and expanded human T-cells. Tumor volumes are presented as RTV (relative tumor volume to day of therapy start). 0.5 mg/kg COVA420 and vehicle treatments were administered twice weekly (day 6, 9, 13, 15), and equimolar doses of COVA446 (SEQ ID NO: 167) (0.16 mg/kg) by daily intravenous (i.v.) bolus injections. Black arrows visualize dose intervals.

Fig. 10    shows the redirected cell kill activity of COVA420 (SEQ ID NOs 163 and 164) and the bispecific anti-CD3 x anti-HER2 control in single chain Fv format (COVA446, SEQ ID NO: 167) towards SKOV-3 tumor cells expressing high level of HER2 and MCF-7 tumor cells expressing low level of HER2. CD8+ enriched T-cells were used as effector cells. The percent of remaining target cell viability is shown.

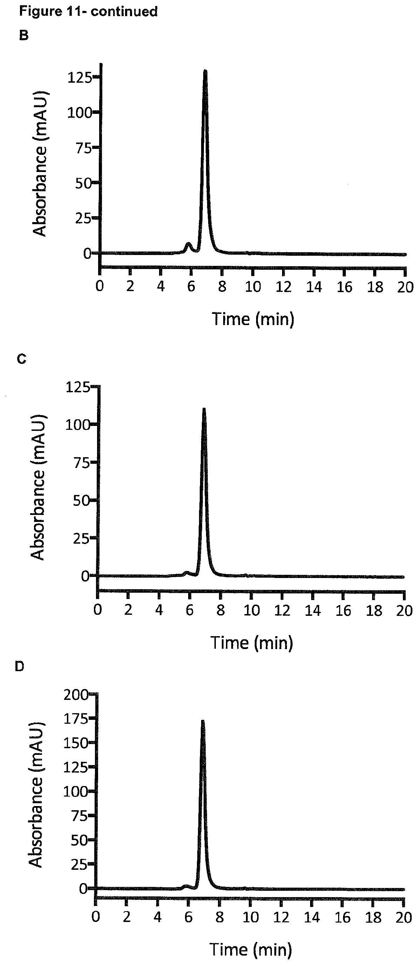

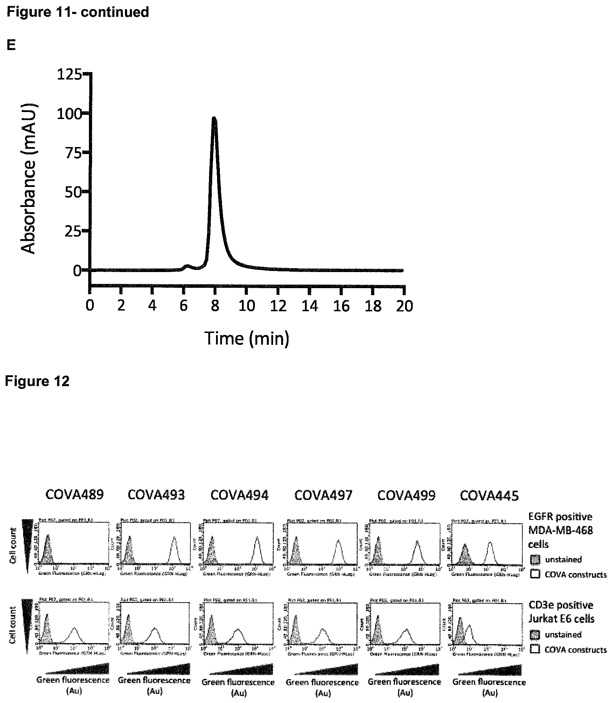

Fig. 11    shows size exclusion (SEC) profiles of Fynomer-antibody fusion proteins. A: SEC profile of COVA493 (SEQ ID NOs 170 and 172); B: SEC profile of COVA494 (SEQ ID NOs 171 and 198); C: SEC profile of COVA497 (SEQ ID NOs: 199 and 172); D: SEC profile of COVA499 (SEQ ID NOs: 171 and 200); E: SEC profile of COVA489 (SEQ ID NOs: 171 and 172).

Fig. 12    shows the flow cytometric binding analysis of the anti-CD3 antibody (COVA489, SEQ ID NOs: 171 and 172), the Fynomer®-antibody fusion proteins COVA493 (SEQ ID NOs 170 and 172), COVA494 (SEQ ID NOs: 171 and 198), COVA497 (SEQ ID NOs: 199 and 172), COVA499 (SEQ ID NOs: 171 and 200) and the bispecific anti-CD3 x anti-EGFR control in single chain Fv format (COVA445, SEQ ID NO: 173) on EGFR positive cells (MDA-MB-468, upper panel) and on CD3 positive cells (Jurkat E6-1. lower panel). Signals are compared to the background signal obtained with the secondary detection antibody only (grey shaded histograms).

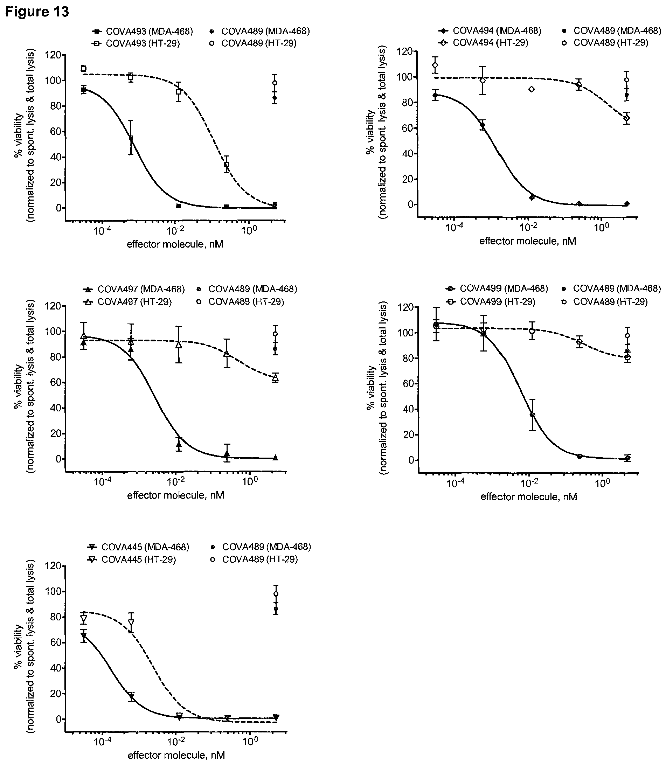

Fig. 13    shows the redirected cell kill activity of COVA493 (SEQ ID NOs 170 and 172), COVA494 (SEQ ID NOs: 171 and 198), COVA497 (SEQ ID NOs: 199 and 172), COVA499 (SEQ ID NOs: 171 and 200) and the bispecific anti-CD3 x anti-EGFR control in single chain Fv format (COVA445, SEQ ID NO: 173) towards MDA-MB-468 tumor cells expressing high level of EGFR and HT-29 tumor cells expressing low level of EGFR. In addition, the absence of any kill activity of the anti-CD3 antibody (COVA489, SEQ ID NOs: 171 and 172) at the highest concentration of 5 nM is shown. CD8+ enriched T-cells were used as effector cells. The percent of remaining target cell viability is shown.

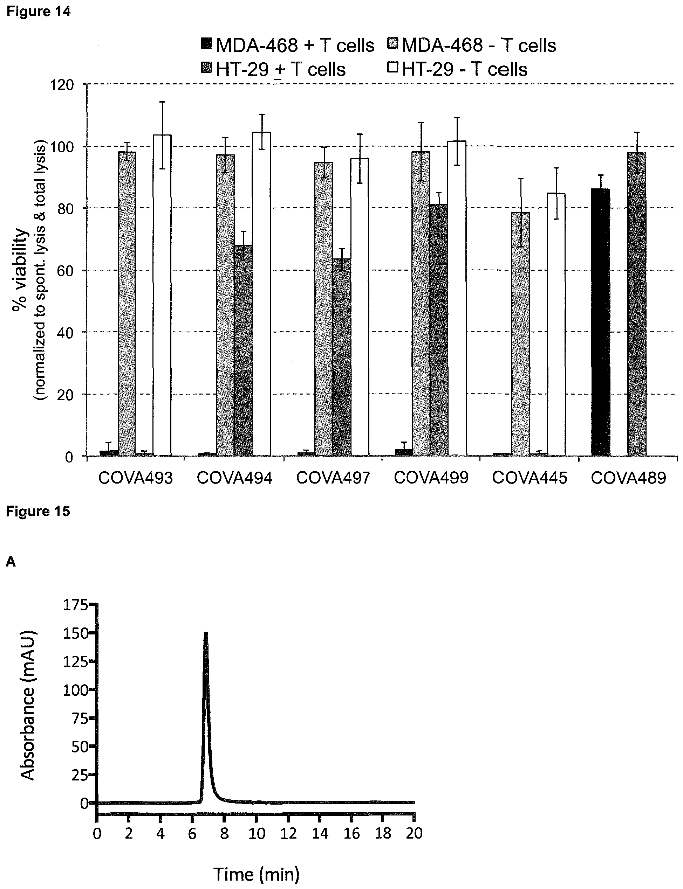

**Fig. 14** shows the redirected cell kill activity of COVA493 (SEQ ID NOs 170 and 172), COVA494 (SEQ ID NOs: 171 and 198), COVA497 (SEQ ID NOs: 199 and 172), COVA499 (SEQ ID NOs: 171 and 200) and the bispecific anti-CD3 x anti-EGFR control in single chain Fv format (COVA445, SEQ ID NO: 173) towards MDA-MB-468 and HT-29 tumor cells, expressing high and low level of EGFR respectively, in the presence or absence of effector T-cells. (n.d.): not determined.

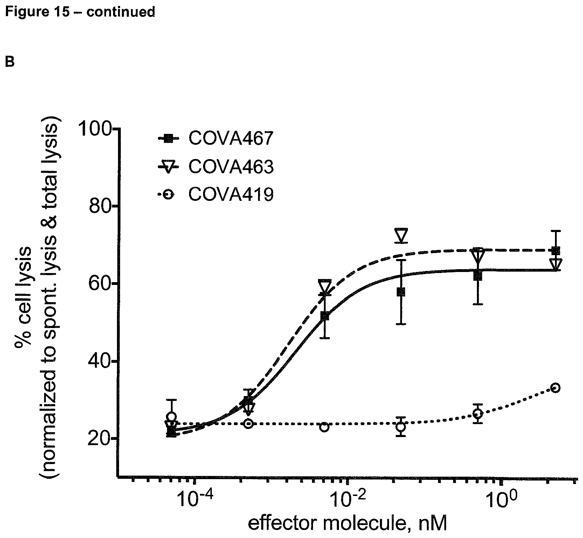

**Fig. 15** (A) shows size exclusion (SEC) profiles of the Fynomer-antibody fusion protein COVA467 (SEQ ID NOs: 2 and 175) (B) shows the redirected cell kill activity of COVA467 (SEQ ID NOs: 2 and 175) and the bispecific anti-CD3 x anti-CD33 control in single chain Fv format (COVA463, SEQ ID NO: 176) on CD33 positive U937 tumor cells. In addition, the absence of any kill activity of the unmodified anti-CD3 antibody (COVA419, SEQ ID NOs: 2 and 3) is shown. CD8+ enriched T-cells were used as effector cells. The percent of target cell lysis is shown.

The Examples illustrate the invention.

## Examples

## Example 1 - Redirected T-cell mediated cell cytotoxicity analysis towards HER2 positive tumor cells

### Example 1.1. - Expression and purification of anti-CD3 antibody anti-HER2 Fynomer® fusion proteins

The HER2 binding Fynomer® C12 (Seq ID NO: 4) has been genetically fused to the anti CD3 binding antibody (SEQ ID NOs: 2 and 3) via a 15 amino acid linker (SEQ ID NO: 162) yielding the bispecific antibody Fynomer® fusion proteins of the present invention. In COVA420 the Fynomer® C12 was fused to the N-terminus of the heavy chain of the anti CD3 antibody (SEQ ID NOs: 2 and 3). In COVA422 the Fynomer® C12 was fused to the C-terminus of the heavy chain of the anti CD3 antibody (SEQ ID NO: 2 and 3).

The anti-CD3 antibody (SEQ ID NO: 2 and 3), COVA420 (SEQ ID NO: 163 and 164), COVA422 (SEQ ID NO: 165 and 166) and the anti-CD3 x anti-HER2 scFv control (SEQ ID NO: 167) (carrying a hexa-his tag), were transiently transfected into FreeStyle CHO-S cells and expressed in serum-free/animal component-free media for 6 days. The anti-CD3 antibody and the bispecific proteins of the invention were purified from the supernatants by Protein A affinity chromatography (GE-Healthcare cat no 89928) with an ÄKTA Purifier instrument (GE Healthcare). Purification of the anti-CD3 x anti-HER2 scFv control (COVA446, SEQ ID NO: 167) was achieved by immobilized metal ion affinity chromatography via a HIStrap Excel column (GE Healthcare). Concentrations were determined by absorbance measurement at 280 nm. Yields are listed in Table 1.

### Results

Antibody Fynomer® fusion proteins of the invention and the control antibodies could be expressed and purified in a single step. A purity of >95% could be demonstrated by SDS-PAGE analysis as shown in Figures 1 and 2. Table 1 summarizes the expression yield after transient transfection into CHO cells and protein expression for 6 days at 37°C.

Table 1.

| Protein | SEQ ID NO: | Yield (mg/l) |
|---|---|---|
| Anti-CD3 antibody (COVA419) | 2, 3 | 46 |
| COVA420 | 163, 164 | 59 |
| COVA422 | 165, 166 | 21 |
| Anti-CD3 x HER2 scFv control (COVA446) | 167 | 17 |

After purification size exclusion chromatography has been performed using an ÄKTA FPLC system and a Superdex G200, 30/100 GL column (GE Healthcare) or an Agilent HPLC 12/60 system and a Bio SEC5 , 5 $\mu$m; 300 Å; 4.6x300 mm column. The bispecific proteins of the invention eluted as a single monomeric peak demonstrating that the antibody Fynomer® fusion proteins of the present invention have very favourable biophysical properties (Figure 3).

## Example 1.2 - FACS experiment with anti-HER2 Fynomer® anti-CD3 antibody fusion proteins of the current invention

The proteins COVA420 (SEQ ID NO: 163 and 164), COVA422 (SEQ ID NO: 165 and 166) and COVA446 (SEQ ID NO: 167) were incubated at a concentration of 100 nM in a total volume of 100 $\mu$l containing either (i) 1 x $10^5$ Jurkat E6-1 cells (CD3 positive cells), (ii) 1 x $10^5$ BT474 HER2 positive breast cancer cells, (iii) 1 x $10^5$ HER2 or CD3 negative MDA MB 468 cells in PBS/1% BSA/0.2 % sodium azide. As a negative control, the same cells were incubated with PBS/1% BSA/0.2 % sodium azide only instead of proteins (PBS control).

After 60 min incubation on ice, cells were washed twice with 150 uL PBS-1% BSA buffer and bound antibodies were detected by adding 5 ug/mL goat anti human-Alexa 488 conjugate (Invitrogen) for 40 min in the dark. For the bispecific scFv control COVA446 (SEQ ID NO: 167), carrying a hexa his tag, binding was detected by the addition of 5 ug/ml mouse anti HIS tag antibody (Fisher Scientific) and incubation at 4°C for 40 min followed by an additional wash step followed by an incubation with 5 ug/mL goat anti mouse Alexa 488 conjugate (Invitrogen) again for 40 min at 4°C. Finally cells were washed three times, resuspended in 100$\mu$l PBS-1%BSA, and stained cells were then analyzed on a Guava easyCyte™ flow cytometer (Millipore).

Results

Binding properties of Fynomer®-antibody fusion proteins were evaluated via flow cytometry. (COVA420 (SEQ ID NO: 163 and 164), COVA422 (SEQ ID NO: 165 and 166) and the bispecific scFv control (COVA446, SEQ ID NO: 167) specifically bound to HER2 expressing BT474 cells (panel A) and CD3 expressing Jurkat E6 cells (panel B) as shown in Figure 4. No non-specific binding was observed on cell lines that did not express either CD3 or HER2 (panel C).

## Example 1.3 - Redirected T-cell mediated cell cytotoxicity analysis towards cells expressing HER2

Polypeptides COVA420 (SEQ ID NO: 163 and 164), and the bispecific scFv control (SEQ ID NO: 167) were tested in a redirected T-cell mediated cell cytotoxicity assay using a protocol adapted from Jager et al (2009) Cancer Research, 69 (10):4270-6.
In brief, human PBMCs were used as effector cells. On the day before the experiment PBMCs were isolated from fresh buffy coat preparations obtained from Blutspende Zürich by Ficoll Plaque plus (GE Healthcare) and density gradient centrifugation using standard procedures. Isolated PBMCs were then incubated over night at a cell concentration of 4x$10^6$ cells/ml in 10% FCS, RPMI and 37°C, 5% $CO_2$. The isolated PBMCs could then serve as effector cells in redirected cell cytotoxicity assays.

Also on the night before the experiment an appropriate amount of target cells were detached by Accutase treatment and target cells were seeded in 96 well plates at cell densities ranging from 3000 - 5000 cells per well in 100 uL complete cell culture medium supplemented with 10% FCS. Assay plates were incubated over night at 37°C and 5% CO2. Target tumor cells used were SKBR-3 (ATCC cat no
HTB-30™) and MDA-MB-468 (ATCC cat no: HTB-132™). Prior to the cell kill experiment an appropriate amount of PBMCs was centrifuged and resuspended in 10% FCS, RPMI. Depending on the number of target cells used the cell concentration was adjusted ranging from 1.5 to 2.5 x$10^6$ cells/mL and cells stored at room temperature until further use.

Redirected T-cell mediated cell cytotoxicity was additionally monitored using enriched CD8+ T-cells as effector cells and SKOV-3 (ATCC cat no HTB-77™) as target tumor cells. For these assays, isolated PBMCs were further purified to obtain enriched CD8+ T-cells. CD8+ T cells were isolated using the Dynbead® Untouched™ Human CD8 T-cell kit (Life Technologies cat no: 11348D) according to manufacturers recommendation. After purification cells were then resuspended in 10% FCS, RPMI at a concentration ranging from 6x$10^5$ to 1x$10^7$ per ml.
On the day of the experiment effector molecules were diluted in 10% FCS, RPMI to a maximum concentration of 150 nM and a dilution series of 1/10 dilutions was prepared.
Finally, consumed medium was removed from the assay plates and substituted with 50 ul of fresh medium per well. Then appropriate amounts of effector molecules and effector cells were added. The final effector cell to target cell ratio was 25/1 for PBMC assays and 10/1 for assays in which CD8+ T-cells served as effector cells. The final maximum concentration of effector molecule was 50 nM. The final assay volume was 150 uL per well.
The assay plates were incubated between 24h and 72h at 37°C, 5% CO2. Then, cell culture supernatants were removed and stored at -80°C for subsequent assays (granzyme release assay, see Example 1.4 below). Prior to the addition of developing solution each well was washed with PBS twice. Cell viability was evaluated using XTT-reagent (Sigma cat no: X4626) according to manufactures recommendation. A 100% lysis control was included by treating the target cells with 1% Triton-X100 (Sigma), and the value for spontaneous lysis was obtained by treating the target cells with effector cells only ("spont lysis"). Absorbance at 450nm was measured between 2.5 and 4 h after addition of XTT substrate. All measurements were done in triplicates.

Percent cell viability was calculated using the following formula:

$$\% \text{ viability} = (\text{value} - 100\% \text{ lysis})/(\text{spont lysis} - 100\% \text{ lysis}) \times 100$$

% cell viability was then plotted against the effector molecule concentration and data were evaluated using Prism 5 (GraphPad Software) by fitting a sigmoidal dose-response.

Results

It could be demonstrated that a dose dependent cytotoxicity of COVA420 (SEQ ID NO: 163 and 164) on HER2 expressing SKBR-3 cells could be observed (Figure 5). In this representative assay, COVA420 (SEQ ID NO: 163 and 164) had an $EC_{50}$ value of 87 pM. Importantly, the cytotoxic effects were antigen dependent since no cytotoxicity was observed on HER2 negative MDA-MB-468 as shown in Figure 5.

Under the same assay conditions an $EC_{50}$ value of 60 pM was obtained for the bispecific anti-HER2 x anti-CD3 scFv-scFv control molecule (SEQ ID NO: 167). It was surprisingly found that bivalent and full length IgG based Fynomer-antibody fusion proteins show potencies in a redirected kill assay that are in the same range as currently used scFv-scFv proteins, but which do not suffer from the drawbacks of suboptimal biophysical properties and short in vivo half-life.

Table 2 summarizes the $EC_{50}$ values obtained for the proteins tested:

Table 2:

| Protein | SEQ ID NO. | $EC_{50}$ (pM) PBMCs |
|---|---|---|
| COVA420 | 163, 164 | 87 |
| scFv-scFv control (COVA446) | 167 | 60 |

In order to demonstrate that the Fynomer-antibody fusion proteins of the invention exert the killing activity through the engagement of T-cells (and not through ADCC mediated activity), redirected cell kill experiments using CD8+ enriched T-cells that cannot mediate cell killing through ADCC were performed.

Figure 6 shows that cell killing could be confirmed using CD8+ enriched T-cells as effector cells. Here, COVA420 (SEQ ID NO: 163 and 164) showed an $EC_{50}$ value of 8 pM and the $EC_{50}$ value of COVA422 (SEQ ID NO: 165 and 166) was 175 pM These results confirm the potent (sub-nM) killing activity of COVA420 (SEQ ID NO: 163 and 164) and COVA22 (SEQ ID NO: 165 and 166).

**Example 1.4** - **Analysis of Granzyme B release in the presence and absence of target cells**

The release of Granzyme B into the cell culture medium upon incubation of COVA420 (SEQ ID NO: 163 and 164) and the anti-CD3 antibody (SEQ ID NO: 2 and 3) as a control in the presence and absence of antigen positive target cells and CD8+ enriched T-cells was evaluated. Release of Granzyme B is a main indicator of T-cell mediated cytotoxic activity induced by BiTE® like agents as described by Haas A. et.al. Immunobiology, 2009, 214 (6): 441-53. Samples were incubated as described above for evaluating the cytotoxic activity of Fynomer®- antibody fusion proteins. At the end of the incubation period the cell culture supernatants were collected and the concentration of Granzyme B was evaluated by using a Granzyme B ELISA kit (R&D Systems) according to manufacturers recommendation.

Results:

Figure 7 depicts the expression level of Granzyme B after 3 days of incubation of COVA420 (SEQ ID NO: 163 and 164) in the presence of CD8+ T-Cells and in the presence and absence of antigen positive target cells at the indicated concentrations. No unspecific release of Granzyme B could be detected if T-cells were only incubated with 50 nM COVA420 (SEQ ID NOs: 163 and 164) in the absence of target cells. A pronounced increase in Granzyme B expression was observed when target cells COVA420 (SEQ ID No: 163 and 164) and T-cells were present. No substantial Granzyme B expression could be detected when COVA420 (SEQ ID No: 163 and 164) was used at concentrations in which no cytotoxic effect was detectable which indicates a correlation between cytotoxic activity and Granzyme B release (0.5 pM). The control anti-CD3 antibody (SEQ ID NO: 2 and 3) (50 nM) did not trigger Granzyme B release in the presence of tumor target and effector cells, as expected.

**Example 1.5** - **Pharmacokinetic analysis**

The pharmacokinetic profile of COVA420 (SEQ ID NO: 163 and 164) in C57BL/6 mice (Charles River) was investigated. Five C57BL/6 mice were injected i.v. with 200 $\mu$g COVA420 (SEQ ID NO: 163 and 164). After 10 min, 6, 24, 48, 96, 120, 144 and 168 hours, blood was collected into EDTA coated microvettes (Sarstedt), centrifuged for 10 min at 9300 g and the serum levels of COVA420 (SEQ ID NO: 163 and 164) was determined by ELISA. Black maxisorp microtiter plates (Nunc) were coated with 50 nM HER2 ECD (Bender MedSystems). After blocking with 2 % BSA (Sigma) in PBS, 40 $\mu$l of PBS and 10 $\mu$l of serum at appropriate dilution were applied. After incubation for 1 hr, wells were washed with PBS, and bound COVA420 (SEQ ID NO: 163 and 164) was detected with anti-hlgG-HRP (Jackson ImmunoResearch). The assay was developed with QuantaRed fluorogenic substrate (Pierce) and the fluorescence intensity was measured after 5 min at 544 nm (excitation) and 590 nm (emission). The serum levels of COVA420 (SEQ ID NO: 163 and 164) were determined using a standard curve of COVA420 (SEQ ID NO: 163 and 164) (diluted to 333 - 0.5 ng/ml). From the concentrations determined in serum at different time points and the resulting slope k of the elimination phase (plotted in a semi-logarithmic scale), the half-life was calculated using to the formula $t_{1/2}$ = ln2/-k.

Results:

Figure 8 shows the serum concentrations of COVA420 (SEQ ID NO: 163 and 164) after an iv bolus injection in mice. The half-life value for COVA420 (SEQ ID NO: 163 and 164) as determined from the elimination phase (beta phase, time-points 24h - 168h) was 140 hours. This finding demonstrates that COVA420 (SEQ ID NO: 163 and 164) has IgG-like *in vivo* PK properties , as the half-life was comparable to the half-lives obtained for other human antibodies in mice (eg. adalimumab: 102-193 hours, Humira® Drug Approval Package (Drug Approval Package, Humira®, FDA Application No.: 125057s0110, Pharmacology Review, 01/18/2008).

**Example 1.6** - **In vivo efficacy of COVA420 in HER2-overexpressing SKOV-3 tumor bearing mice reconstituted with human T-cells.**

The anti-tumor activity of COVA420 was investigated in irradiated NOD.CB17 Prkdc mice bearing a HER2-expressing human SKOV-3 tumor xenograft.

Methods:

3 x $10^6$ SKOV-3 (ATCC cat no HTB-77™) cells were injected subcutanously (s.c.) into 2 Gy-irradiated NOD.CB17 Prkdc mice (Charles River). When tumors reached an average size of ca. 50 mm$^3$, animals were treated with a single intravenous (i.v.) bolus injection of anti-asialo GM1 rabbit antibody (WAKO, Germany) one day before human T-cell injection. In vitro activated and expanded (22 days) human T-cells (Miltenyi Biotech, Germany) isolated from a buffy coat of a single healthy donor, were injected (1.6 x $10^7$ per mouse) into the peritoneal cavity. Three days after T-cell injection, mice were randomized and received 0.5 mg/kg COVA420 (SEQ ID NO: 163 and 164; n=8), vehicle (PBS; n=7) treatments twice per week (days 6, 9, 13, 15) or daily equimolar doses (= 0.16 mg/kg) of COVA446 (SEQ ID NO: 167; n=8) by i.v. bolus injection into the lateral tail vein for a total of 15 days. Treatment efficacy was assessed by tumor growth inhibition. Tumor size was measured by external caliper measurements and volume calculated using the standard hemi-ellipsoid formula: volume = (width)$^2$ x length x 0.5. Relative tumor volumes (RTV) to day 6 (initiation of therapy) are presented as mean $\pm$ SEM. Statistical analysis was performed using GraphPad Prism 6 software, version 6a. Statistical significance of anti-tumor efficacy was calculated by using an unpaired, nonparametric t-test (Mann-Whitney). Anti-tumor efficacy of COVA420 vs vehicle treatment on day 16 was further evaluated as tumor volume inhibition relative to the vehicle control, expressed as treatment-to-control ratio (T/C): T/C (%) = RTV (day 16) / RTV (day 6) (Wu (2010), Journal of Biopharmaceutical Statistics, 20:5, 954-964).

Results:

COVA420 significantly reduces SKOV-3 tumor growth by actively recruiting T-cells to the tumor (Figure 9). COVA420 treatment resulted in a significant growth inhibition as compared to the vehicle control on day 16, after 4 doses of 0.5 mg/kg COVA420 (P=0.0059). COVA420 treatment was also significantly more efficacious as compared to the daily injected 0.16 mg/kg COVA446 control (P=0.04). T/C ratio on the same day equals to 55% growth inhibition of COVA420 and 79% of COVA446 as compared to the vehicle treatment. The results demonstrate that COVA420 is pharmacologically active and exerts its anti-tumor activity by efficiently recruiting human T-cells to the tumor resulting in inhibited tumor growth as compared to the vehicle-treated control.

**Example 1.7** - **Analysis of redirected T-cell mediated cell cytotoxicity selectively towards tumor cells expressing high levels of HER2**

Polypeptides COVA420 (SEQ ID NOs: 163 and 164) and the bispecific scFv control COVA446 (SEQ ID NO: 167) were tested in a redirected T-cell mediated cell cytotoxicity assay using a protocol adapted from Jager et al (2009) Cancer Research, 69 (10):4270-6.

In brief, human PBMCs were isolated from fresh buffy coat the day before and CD8+ T-cells were isolated on the day of the experiment as described in example 1.3.

On the night before the experiment an appropriate number of target cells were detached by Accutase treatment and target cells were seeded in 96 well plates at a cell density of 5000 cells per well in 150 $\mu$l appropriate growth medium supplemented with 10% FCS. Assay plates were incubated over night at 37°C and 5% $CO_2$. Target tumor cells used were SKOV-3 (ATCC® HTB-77™) expressing high level of HER2 (approx. 1.7 x $10^6$ HER2 molecules/cell) and MCF-7 (ATCC® HTB-22™) expressing low level of HER2 (approx. 1 x $10^4$ HER2 molecules/cell). HER2 surface expression was quantified using Qifikit (Dako K0078) according to the manufacturers recommendation. Briefly, cells were stained with a saturating concentration of anti-HER2 antibody (R&D MAB1129) or isotype matched control IgG, followed by anti-mouse-FITC secondary antibody, and flow cytometric analysis. At the same time, beads coated with different well-defined quantities of mouse monoclonal antibody molecules were stained with the secondary antibody, analysed and a standard curve was plotted as a reference for molecules/cell.

On the day of the experiment, effector molecules were diluted in 10% FCS, RPMI to a maximum concentration of 150 nM and a dilution series of 1/10 dilutions was prepared.

An appropriate amount of T-cells was centrifuged and resuspended in 10% FCS, RPMI. The cell concentration was adjusted to, 1 x$10^6$ cells/mL.

Finally, consumed medium was removed from the assay plates and substituted with 50 $\mu$l of fresh 10% FCS, RPMI per well. Then 50 $\mu$l of effector molecules and 50 $\mu$l of effector T-cells were added. The final effector cell to target cell ratio was 10:1. The final maximum concentration of effector molecule was 50 nM. The final assay volume was 150 $\mu$l per well.

The assay plates were incubated for 60h at 37°C, 5% $CO_2$. Then, cell culture supernatants were removed and each well was washed once with PBS. Cell viability was evaluated using XTT-reagent (Sigma X4626) according to the manufacturer's recommendation. A 100% lysis control was included by treating the target cells with 1% Triton-X100 (Sigma), and the value for spontaneous lysis was obtained by incubating the target cells with effector cells only ("spont lysis"). Absorbance at 450nm was measured 2 h after addition of XTT substrate. All measurements were done in triplicates. Percent cell viability was calculated using the following formula:

$$\% \text{ viability} = (\text{value-100\% lysis})/(\text{spont lysis-100\% lysis})*100$$

% cell viability was then plotted against the effector molecule concentration and data were evaluated using Prism 5 (GraphPad Software) by fitting a sigmoidal dose-response.

Results

Dose dependent redirected T-cell cytotoxicity of COVA420 towards SKOV-3 target cells expressing high level of HER2 could be observed (Figure 10). Table 3 summarizes the $EC_{50}$ values obtained for the proteins tested.

Furthermore, dose dependent redirected T-cell cytotoxicity of the bispecific anti-CD3 x anti-HER2 scFv control molecule COVA446 towards MCF-7 target cells expressing low level of HER2 could be observed. In this representative assay, COVA446 had an $EC_{50}$ value of 53 pM. Surprisingly, COVA420 did not exhibit significant redirected T-cell cytotoxicity towards MCF-7 target cells expressing low level of HER2 (Table 3).

Table 3:

| Protein | SEQ ID NO. | SKOV-3 $EC_{50}$ (pM) | MCF-7 $EC_{50}$ (pM) | Factor difference |
|---|---|---|---|---|
| COVA420 | 163, 164 | 11.6 | n.d. | > 4310 |

(continued)

| Protein | SEQ ID NO. | SKOV-3 EC$_{50}$ (pM) | MCF-7 EC$_{50}$ (pM) | Factor difference |
|---|---|---|---|---|
| anti- CD3 x EGFR scFv control (COVA446) | 167 | 2.3 | 53 | 23 |

**Example 2 - Redirected T-cell mediated cell cytotoxicity analysis towards EGFR positive tumor cells**

**Example 2.1. - Expression and purification of anti-CD3 antibody anti-EGFR Fynomer fusion proteins**

DNA encoding the polypeptides shown in SEQ ID NOs: 169 and 187 to 197 were cloned into the bacterial expression vector pQE12 so that the resulting constructs carried a C-terminal myc-hexahistidine tag as described in Grabulovski et al. (Grabulovski et al. (2007) JBC, 282, p. 3196-3204). The polypeptides were expressed in the cytosol of *E. coli* bacteria in 200 μl scale cultures. Cleared lysate containing the polypeptides was diluted 5:1 in PBS/1% FCS/0.2 % sodium azide buffer containing 10 μg/ml anti-myc mouse antibody 9E10 (Roche) and added to 1x 10$^5$ MDA-MB-468 cells (ATCC® HTB-132™). After 60 min incubation on ice, cells were washed, and bound sequences were detected by anti-mouse IgG - Alexa488 conjugate (Invitrogen). The stained cells were then analyzed in a FACS analyzer. The DNA sequence of the specific binders was verified by DNA sequencing. The amino acid sequences of Fyn SH3 derived EGFR binders is presented in SEQ ID NOs: 169 and 187 to 197 as appended in the sequence listing.

The EGFR binding Fynomer ER7L2D6 (SEQ ID NO: 169) has been genetically fused to the N-terminus of the heavy chain (SEQ ID NO: 171) of a CD3 binding antibody (COVA489, SEQ ID NOs: 171 and 172) via a 15 amino acid linker (SEQ ID NO: 162) yielding the bispecific antibody Fynomer fusion protein COVA493 (SEQ ID NOs: 170 and 172). In addition, ER7L2D6 (SEQ ID NO: 1) was fused to the N-terminus of the antibody light chain resulting in the bispecific antibody Fynomer fusion protein COVA494 (SEQ OD NOs: 171 and 198).

The EGFR binding Fynomer ER9L3D7 (SEQ ID NO: 187) has been genetically fused to the N-terminus of the antibody heavy chain (SEQ ID NO: 171) of an anti-CD3 binding antibody (COVA489, SEQ ID NOs: 171 and 172) via a 15 amino acid linker (SEQ ID NO: 162) yielding the bispecific antibody Fynomer fusion protein COVA497 (SEQ ID NOs: 199 and 172). In addition, ER9L3D7 (SEQ ID NO: 2) was fused to the C-terminus of the antibody light chain (SEQ ID NOs: 172) resulting in the bispecific antibody Fynomer fusion protein COVA499 (SEQ OD NOs: 171 and 200).

The anti-CD3 antibody COVA489, the bispecific proteins COVA493, COVA494, COVA497, COVA499 and the anti-CD3 x anti-EGFR scFv control COVA445 (SEQ ID NO: 173) (carrying a hexa-his tag), were transiently transfected into FreeStyle CHO-S cells and expressed in serum-free/animal component-free media for 6 days. The anti-CD3 antibody and the bispecific proteins of the invention were purified from the supernatants by Protein A affinity chromatography (GE-Healthcare cat no 89928) with an ÄKTA Purifier instrument (GE Healthcare). Purification of the anti-CD3 x anti-EGFR scFv control (COVA445, SEQ ID NO: 173) was achieved by immobilized metal ion affinity chromatography via a HIStrap Excel column (GE Healthcare). Concentrations were determined by absorbance measurement at 280 nm. Yields are listed in Table 4.

After purification, analytical size exclusion chromatography was performed using a silica-based SEC-5 column (Agilent; 5 μm; 300 Å) on an Agilent HPLC 12/60 system.

Results

Antibody Fynomer fusion proteins of the invention and the control antibodies could be expressed and purified in a single step. Table 1 summarizes the purification yield after transient transfection into CHO cells and protein expression for 6 days at 37°C.

Table 4. Expression yields.

| Protein | SEQ ID NO: | Yield (mg/l) |
|---|---|---|
| Anti-CD3 antibody (COVA489) | 171, 172 | 66 |
| COVA493 | 170, 172 | 40 |
| COVA494 | 171, 198 | 39 |
| COVA497 | 199, 172 | 35 |
| COVA499 | 171, 200 | 74 |
| Anti-CD3 x EGFR scFv control (COVA445) | 173 | 34 |

The bispecific proteins of the invention eluted as a single monomeric peak from the size exclusion column, demonstrating that the antibody Fynomer fusion proteins of the present invention have very favorable biophysical properties (Figure 11). The bispecific constructs of the invention expressed at least as good as the unmodified anti-CD3 antibody and did not aggregate after purification as shown by SEC analysis.

**Example 2.2 - FACS assay experiment with anti-EGFR Fynomer® anti-CD3 antibody fusion proteins of the current invention**

COVA489, COVA493, COVA494, COVA497, COVA499 and COVA445 were incubated either (i) at 30 nM concentration in a total volume of 100 $\mu$l with 1 x 10$^5$ Jurkat E6-1 cells (CD3 positive cells; ATCC® TIB-152™), or (ii) at 100 nM concentration in a total volume of 100 $\mu$l with 1 x 10$^5$ EGFR-overexpressing MDA-MB-468 breast cancer cells (ATCC® HTB-132™) in PBS/1% BSA/0.2 % sodium azide. As a negative control, the same cells were incubated with PBS/1% BSA/0.2% sodium azide only instead of proteins (PBS control).

After 45 min incubation on ice, cells were washed twice with 150 uL PBS-1% BSA buffer and bound antibodies were detected by adding 4 ug/mL goat anti-human-Alexa 488 conjugate (Invitrogen) for 40 min at 4 °C. For the bispecific scFv control COVA445 carrying a hexa his tag, binding was detected by concomitant incubation of COVA445 with a mouse anti HIS tag antibody (Fisher Scientific) at a molar ratio of 1:4 (anti-HIS tag antibody : COVA445), followed by additional wash steps, followed by incubation with 4 ug/mL goat anti-mouse-Alexa 488 conjugate (Invitrogen) for 40 min at 4°C. Finally cells were washed three times, resuspended in 100$\mu$l PBS-1%BSA, and stained cells were then analyzed on a Guava easyCyte™ flow cytometer (Millipore).

Results

All constructs bound to Jurkat E6-1 cells which express human CD3, and all Fynomer-antibody fusions as well as the anti-CD3 x anti-EGFR scFv control COVA445 bound to MDA-MB-468 (Figure 12). No specific binding was observed for the anti-CD3 antibody COVA489 on MDA-MB-468, as expected.

**Example 2.3 - Analysis of redirected T-cell mediated cell cytotoxicity selectively towards tumor cells expressing high levels of EGFR**

Polypeptides COVA493, COVA494, COVA497, COVA499, the bispecific scFv control COVA445 and the anti-CD3 antibody (COVA489, SEQ ID Nos: 171 and 172) were tested in a redirected T-cell mediated cell cytotoxicity assay using a protocol adapted from Jager et al (2009) Cancer Research, 69 (10):4270-6.

In brief, on the night before the experiment an appropriate number of target cells were detached by Accutase treatment and target cells were seeded in 96 well plates at a cell density of 3000 - 5000 cells per well in 150 $\mu$l appropriate growth medium supplemented with 10% FCS. Assay plates were incubated over night at 37°C and 5% CO$_2$. Target tumor cells used were MDA-MB-468 (ATCC® HTB-132™) expressing high level of EGFR (approx. 1.5 x 10$^6$ EGFR molecules/cell) and HT-29 (ATCC® HTB-38™) expressing low level of EGFR (approx. 5 x 10$^4$ EGFR molecules/cell). EGFR surface expression was quantified as described in Example 1.7 but using a saturating concentration of anti-EGFR antibody (Millipore MABF120).

On the day of the experiment, effector molecules were diluted in 10% FCS, RPMI to a maximum concentration of 15 nM and a dilution series of 1/20 dilutions was prepared.

Human CD8+ enriched T-cells were used as effector cells. CD8+ enriched T-cells were isolated from fresh buffy coat preparations obtained from Blutspende Bern using the MACSxpress human CD8+ isolation kit (Miltenyi 130-098-194) together with the MACSxpress Separator (Milteny 130-098-309) and Red blood cell lysis solution (Milteny 130-094-183) as recommended by the manufacturer.

An appropriate amount of T-cells was centrifuged and resuspended in 10% FCS, RPMI. The cell concentration was adjusted to, 1 x10$^6$ cells/mL.

Finally, consumed medium was removed from the assay plates and substituted with 50 $\mu$l of fresh 10% FCS, RPMI per well. Then 50 $\mu$l of effector molecules and 50 $\mu$l of effector T-cells were added. The final effector cell to target cell ratio was 10:1. The final maximum concentration of effector molecule was 5 nM. The final assay volume was 150 $\mu$l per well. In order to demonstrate that the Fynomer-antibody fusion proteins of the invention exert the killing activity through the engagement of T-cells, target cells were incubated with COVA493, COVA494, COVA497, COVA499 and COVA445 at a concentration of 5 nM also in the absence of T-cells.

The assay plates were incubated for 64h at 37°C, 5% CO$_2$. Then, cell culture supernatants were removed and each well was washed once with PBS. Cell viability was evaluated using XTT-reagent (Sigma X4626) according to the manufacturer's recommendation. A 100% lysis control was included by treating the target cells with 1% Triton-X100 (Sigma), and the value for spontaneous lysis was obtained by incubating the target cells with effector cells only ("spont lysis"). Absorbance at 450nm was measured 5 h after addition of XTT substrate. All measurements were done in triplicates. Percent cell viability was calculated using the following formula:

$$\% \text{ viability} = (\text{value-100\% lysis})/(\text{spont lysis-100\% lysis})*100$$

% cell viability was then plotted against the effector molecule concentration and data were evaluated using Prism 5 (GraphPad Software) by fitting a sigmoidal dose-response.

Results

Dose dependent redirected T-cell cytotoxicity of COVA493, COVA494, COVA497 and COVA499 towards MDA-MB-468 target cells expressing high level of EGFR could be observed (Figure 13. Table 5 summarizes the $EC_{50}$ values obtained for the proteins tested.

Furthermore, dose dependent redirected T-cell cytotoxicity of COVA493 and the bispecific anti-CD3 x anti-EGFR scFv control molecule towards HT-29 target cells expressing low level of EGFR could be observed. In this representative assay, COVA493 had an $EC_{50}$ value of 117.3 pM, and COVA445 had an $EC_{50}$ value of 2.5 pM. Surprisingly, COVA494, COVA497 and COVA499 did not exhibit significant redirected T-cell cytotoxicity towards HT-29 target cells expressing low level of EGFR even at the highest concentration of 5 nM (Table 5).

The cytotoxic effects were dependent on the presence of the anti-EGFR Fynomer since no cytotoxicity was observed with the anti-CD3 antibody (COVA489, SEQ ID NOs: 13 and 14, measured at the highest concentration of 5 nM).

Table 5. EC50 values for T-cell mediated cytotoxicity. (n.a. not applicable)

| Protein | SEQ ID NO. | MDA-MB-468 $EC_{50}$ (pM) | HT-29 $EC_{50}$ (pM) | Factor difference |
|---|---|---|---|---|
| COVA493 | 170, 172 | 0.2 | 117.3 | 580 |
| COVA494 | 171,198 | 1.4 | n.a. | >3500 |
| COVA497 | 199, 172 | 2.8 | n.a. | >1750 |
| COVA499 | 171, 200 | 6 | n.a. | >830 |
| anti- CD3 x EGFR scFv control (COVA445) | 173 | 0.2 | 2.5 | 12.5 |

The cytotoxic effects were dependent on the presence of effector T-cells (Figure 14), since no cytotoxicity was observed when target cells were incubated with COVA493, COVA494, COVA497, COVA499 and the bispecific anti-CD3 x anti-EGFR scFv control molecule (COVA445).

**Example 3** - **Redirected T-cell mediated cell cytotoxicity analysis towards CD33 positive tumor cells**

**Example 3.1.** - **Expression and purification of anti-CD3 antibody anti-CD33 Fynomer fusion proteins**

DNA encoding the amino acids shown in SEQ ID NOs: 174 and 177 to 186 were cloned into the bacterial expression vector pQE12 so that the resulting constructs carried a C-terminal myc-hexahistidine tag as described in Grabulovski et al. (Grabulovski et al. (2007) JBC, 282, p. 3196-3204). The polypeptides were expressed in the cytosol of *E.coli* bacteria in 200 μl scale cultures. Monoclonal cleared lysates were used for ELISA: human recombinant CD33 (purchased from R&D as fusion to human Fcγ1) was immobilized on MaxiSorp wells (Nunc), and after blocking with 4% milk (Rapilait, Migros, Switzerland) in PBS, 12.5 μl of 10% milk in PBS containing 50 μg/ml mouse anti-myc mouse antibody 9E10 (Roche) and 50 μl of cleared lysate were applied. After incubation for 1 hr, unbound Fynomers were washed off, and bound Fynomers were detected with anti-mouse IgG - HRP antibody conjugate (Sigma). The detection of peroxidase activity was done by adding BM blue POD substrate (Roche) and the reaction was stopped by adding 1 M $H_2SO_4$. The ELISA positive clones were tested by an identical ELISA for the absence of cross reactivity to human IgG (Sigma) and to uncoated MaxiSorp wells. Furthermore, DNA encoding the polypeptides shown in SEQ ID NOs: 174 and 177 to 186 were purified from the bacterial lysates using immobilized metal affinity chromatography columns as described in Grabulovski et al. (Grabulovski et al. (2007) JBC, 282, p. 3196-3204). 93 nM purified Fynomer were incubated with 1x $10^5$ U937 cells (ATCC CRL-1593.2™) in in 100 μl PBS/1% FCS/0.2 % sodium azide containing 23 nM mouse anti-myc antibody 9E10. After 60 min incubation on ice, cells were washed, and bound sequences were detected by anti-mouse IgG - Alexa488 conjugate (Invitrogen). The stained cells were then analyzed in a FACS analyzer. The DNA sequence of the specific binders was verified by DNA sequencing. The amino acid sequences of Fyn SH3 derived CD33 binders are presented in SEQ ID NOs: 174 and 177 to 186 as appended in the sequence listing.

The CD33 binding Fynomer EE1L1B3 (Seq ID NO: 174) has been genetically fused to the C-terminus of the light chain (SEQ ID NO: 3) of an anti-CD3 binding antibody (SEQ ID NOs: 2 and 3) via a 15 amino acid linker (SEQ ID NO: 162) yielding the bispecific antibody Fynomer fusion protein COVA467 (SEQ ID NOs: 2 and 175) of the present invention. COVA467 and the anti-CD3 x anti-CD33 scFv control COVA463 (SEQ ID NO: 176) described in patent application WO 2010/037835 were expressed and purified as described in Example 1. COVA467 expressed as good as the unmodified anti-CD3 antibody (yields: 56 mg/l for COVA467 vs. 46 mg/l for the parental anti-CD3 antibody COVA419) and did not aggregate after purification as shown by SEC analysis Figure 15A.

**Example 3.2** - **Analysis of redirected T-cell mediated cell cytotoxicity towards tumor cells expressing CD33**

Polypeptides COVA467 (SEQ ID NOs: 2 and 175), and the bispecific scFv control COVA463 (SEQ ID NO: 176) were tested in a redirected T-cell mediated cell cytotoxicity assay using a protocol adapted from Jager et al (2009) Cancer Research, 69 (10):4270-6.

In brief, U937 (ATCC cat no: CRL-1593.2™) target cells were seeded in round bottom 96 well plates at a cell density of 10000 cells per well in 50 $\mu$L RPMI medium supplemented with 10% FCS. Effector molecules were diluted in 10% FCS, RPMI to a maximum concentration of 15 nM and a dilution series of 1/10 dilutions was prepared. Human CD8+ enriched T-cells were used as effector cells. CD8+ enriched T-cells were isolated as described in Example 3 and adjusted to a concentration of to 2 x10$^6$ cells/mL. Then 50 $\mu$l of effector molecules at the indicated concentrations and 50 $\mu$l of effector T-cells were added. The final effector cell to target cell ratio was 10:1. The final assay volume was 150 $\mu$l per well.

The assay plates were incubated for 24h at 37°C, 5% $CO_2$. Cell lysis was evaluated using CytoTox-Fluor™ cytotoxicity assay (Promega G9260) according to manufactures recommendation. A 100% lysis control was included by treating the target cells with 2% Saponin (Sigma) at 0h incubation. Spontaneous lysis was measured by incubating the target cells with effector T-cells only ("spont lysis"). After 24h incubation, assay plates were spun down at 400 x g for 10min and 100 $\mu$l of supernatant was transferred into black 96 well plates. CytoTox-Fluor™ reagent and assay buffer was thawed at room temperature and 60 $\mu$l of reagent were diluted in 30 ml assay buffer. Subsequently 50 $\mu$l of this mixture was added to each well of assay plate supernatant. Plates were incubated for 2h at 37°C and fluorescence was recorded at 485 nm excitation / 520 nm emission. All measurements were done in triplicates.
Percent cell viability was calculated using the following formula:

$$\% \text{ cell lysis} = (\text{value-spont lysis})/(100\% \text{ lysis-spont lysis})*100$$

% cell lysis was then plotted against the effector molecule concentration and data were evaluated using Prism 5 (GraphPad Software) by fitting a sigmoidal dose-response.

Results

It could be demonstrated that a dose dependent cytotoxicity of COVA467 (SEQ ID NOs: 2 and 175) on CD33 expressing U937 cells could be observed (Figure 15 B). In this representative assay, COVA467 (SEQ ID NOs: 2 and 175) had an $EC_{50}$ value of 2.1 pM. Importantly, the cytotoxic effects were dependent on the presence of the anti-CD33 Fynomer since no cytotoxicity was observed with the anti-CD3 antibody (COVA419, SEQ ID NOs: 2 and 3).
Under the same assay conditions an $EC_{50}$ value of 1.6 pM was obtained for the bispecific anti-CD33 x anti-CD3 scFv-scFv control molecule (SEQ ID NO: 176). It was surprisingly found that bivalent and full length IgG based Fynomer-antibody fusion proteins show potencies in a redirected kill assay that are in the same range as currently used scFv-scFv proteins, but which do not suffer from the drawbacks of suboptimal biophysical properties and short in vivo half-life.

Table 6 summarizes the $EC_{50}$ values obtained for the proteins tested:

Table 6:

| Fynomer | SEQ ID NO. | $EC_{50}$ (pM) |
| --- | --- | --- |
| COVA467 | 2, 175 | 2.1 |
| scFv-scFv control (COVA463) | 176 | 1.6 |

**Claims**

1. A bispecific binding molecule that specifically binds to (i) CD3 and (ii) a surface target antigen on cancer cells, wherein the binding molecule comprises

   (a) an antibody specifically binding to CD3, and
   (b) a Fyn-SH3-derived polypeptide binding to a surface target antigen on cancer cells,

   wherein said Fyn-SH3-derived polypeptide consists of the amino acid sequence of SEQ ID NO: 1, with the proviso that

   (i) at least one amino acid within amino acids positions 10 to 19 of SEQ ID NO: 1 is substituted, deleted or added, and
   (ii) at least one amino acid within amino acids positions 29 to 36 of SEQ ID NO: 1 is substituted, deleted or added,

   provided that said polypeptide has at least 85% sequence identity to the amino acid sequence of SEQ ID NO: 1, wherein the determination of identity excludes amino acid positions 12 to 17 and 31 to 34 of SEQ ID NO: 1.

2. The binding molecule according to claim 1, wherein the antibody specifically binding to CD3 comprises an Fc part which allows for extending the *in vivo* half-life of the bispecific binding molecule.

3. The binding molecule according to any one of claims 1 or 2, wherein the binding molecule comprises two Fyn-SH3-derived polypeptides specifically binding to EGFR, HER2, CD33, CD19, CEA, EPHA2, melanoma-associated chondroitin sulfate proteoglycan, IGFR1, fibroblast-activating protein alpha, prostate stem cell antigen (PSCA), c-MET, EpCAM, or PSMA.

4. The binding molecule according to any one of claims 1 to 3, wherein the Fyn-SH3-derived polypeptide specifically binds to HER2 and comprises any one of the amino acid sequences chosen from the group consisting of SEQ ID NOs: 4 and 9-159, preferably SEQ ID NO: 4.

5. The binding molecule according to any one of claims 1 to 3, wherein the Fyn-SH3-derived polypeptide specifically binds to EGFR and comprises any one of the amino acid sequences chosen from the group consisting of SEQ ID NOs: 169, 187-197 and 201-203, preferably SEQ ID NO: 169 or 187.

6. The binding molecule according to any one of claims 1 to 3, wherein the Fyn-SH3-derived polypeptide specifically binds to CD33 and comprises any one of the amino acid sequences chosen from the group consisting of SEQ ID NOs: 174, 177-186 and 207, preferably SEQ ID NO: 174.

7. The binding molecule according to any one of claims 1 to 4, wherein the binding molecule comprises two Fyn-SH3-derived polypeptides specifically binding to HER2, and wherein the first of said two polypeptides is linked to the first N-terminus of the two heavy chains of said antibody and the second of said two polypeptides is linked to the second N-terminus of the two heavy chains of said antibody.

8. The binding molecule according to any one of claims 1 to 3, or 5, wherein the binding molecule comprises two Fyn-SH3-derived polypeptides specifically binding to EGFR, and wherein (i) the first of said two polypeptides is linked to the first N-terminus of the two heavy chains of said antibody and the second of said two polypeptides is linked to the second N-terminus of the two heavy chains of said antibody, (ii) the first of said two polypeptides is linked to the first C-terminus of the two heavy chains of said antibody and the second of said two polypeptides is linked to the second C-terminus of the two heavy chains of said antibody, (iii) the first of said two polypeptides is linked to the first N-terminus of the two light chains of said antibody and the second of said two polypeptides is linked to the second N-terminus of the two light chains of said antibody, or (iv) the first of said two polypeptides is linked to the first C-terminus of the two light chains of said antibody and the second of said two polypeptides is linked to the second C-terminus of the two light chains of said antibody.

9. The binding molecule according to any one of claims 1 to 3, or 6, wherein the binding molecule comprises two Fyn-SH3-derived polypeptides specifically binding to CD33, and wherein the first of said two polypeptides is linked to the first C-terminus of the two light chains of said antibody and the second of said two polypeptides is linked to the second C-terminus of the two light chains of said antibody.

10. The binding molecule according to any one of claims 1 to 9, wherein the antibody specifically binding to CD3 and the Fyn-SH3-derived polypeptide are linked by a linker, preferably wherein the linker is a peptide-linker, more preferably wherein the peptide linker has a sequence selected from the group consisting of SEQ ID Nos: 162, 160 and 161.

11. A nucleic acid molecule encoding the binding molecule of any one of claims 1 to 10.

12. A vector comprising the nucleic acid molecule of claim 11.

13. A host cell or a non-human host transformed with the vector of claim 12.

14. A pharmaceutical composition comprising the binding molecule of any one of claims 1 to 10, the nucleic acid molecule of claim 11, the vector of claim 12, and/or the host cell or non-human host of claim 13.

15. The pharmaceutical composition of claim 14 for use in a method of treating or preventing cancer.

16. The pharmaceutical composition for use according to claim 15, wherein said cancer is selected from the group consisting of breast cancer, hematological malignancies such as NHL, B-ALL, AML, other cancers as prostate cancer, melanoma, lung cancer, gastric cancer, ovarian cancer, head-and-neck cancer, colorectal cancer, pancreatic cancer, thyroid cancer, glioma, bladder cancer, kidney cancer, liver cancer, or malignant ascites, preferably wherein said cancer expresses the surface target antigen being specifically bound by the binding molecule of any one of claims 1 to 10 in an amount being at least two times higher as the amount expressed on non-tumor cells.

**Patentansprüche**

1. Bispezifisches Bindungsmolekül, welches spezifisch an (i) CD3 und (ii) ein Oberflächenzielantigen auf Krebszellen bindet,
wobei das Bindungsmolekül Folgendes umfasst:

(a) einen spezifisch an CD3 bindenden Antikörper und
(b) ein an ein Oberflächenzielantigen auf Krebszellen bindendes, von Fyn-SH3 abgeleitetes Polypeptid,

wobei das von Fyn-SH3 abgeleitete Polypeptid aus der Aminosäuresequenz von SEQ ID NO: 1 besteht, mit der Maßgabe, dass

(i) mindestens eine Aminosäure in den Aminosäurepositionen 10 bis 19 von SEQ ID NO: 1 substituiert, deletiert oder addiert ist und
(ii) mindestens eine Aminosäure in den Aminosäurepositionen 29 bis 36 von SEQ ID NO: 1 substituiert, deletiert oder addiert ist,

mit der Maßgabe, dass das Polypeptid mindestens 85% Sequenzidentität zur Aminosäuresequenz von SEQ ID NO: 1 aufweist, wobei die Bestimmung der Identität die Aminosäurepositionen 12 bis 17 und 31 bis 34 von SEQ ID NO: 1 ausschließt.

2. Bindungsmolekül nach Anspruch 1, wobei der spezifisch an CD3 bindende Antikörper einen Fc-Teil umfasst, welcher eine Verlängerung der Invivo-Halbwertszeit des bispezifischen Bindungsmoleküls erlaubt.

3. Bindungsmolekül nach Anspruch 1 oder 2, wobei das Bindungsmolekül zwei von Fyn-SH3 abgeleitete Polypeptide umfasst, die spezifisch an EGFR, HER2, CD33, CD19, CEA, EPHA2, melanomassoziiertes Chondroitinsulfatproteoglykan, IGFR1, fibroblastenaktivierendes Protein alpha, Prostatastammzellantigen (Prostate Stern Cell Antigen, PSCA), c-MET, EpCAM oder PSMA binden.

4. Bindungsmolekül nach einem der Ansprüche 1 bis 3, wobei das von Fyn-SH3 abgeleitete Polypeptid spezifisch an HER2 bindet und eine der Aminosäuresequenzen ausgewählt aus der Gruppe bestehend aus SEQ ID NOs: 4 und 9-159, vorzugsweise SEQ ID NO: 4, umfasst.

5. Bindungsmolekül nach einem der Ansprüche 1 bis 3, wobei das von Fyn-SH3 abgeleitete Polypeptid spezifisch an EGFR bindet und eine der Aminosäuresequenzen ausgewählt aus der Gruppe bestehend aus SEQ ID NOs: 169, 187-197 und 201-203, vorzugsweise SEQ ID NO: 169 oder 187, umfasst.

6. Bindungsmolekül nach einem der Ansprüche 1 bis 3, wobei das von Fyn-SH3 abgeleitete Polypeptid spezifisch an CD33 bindet und eine der Aminosäuresequenzen ausgewählt aus der Gruppe bestehend aus SEQ ID NOs: 174, 177-186 und 207, vorzugsweise SEQ ID NO: 174, umfasst.

7. Bindungsmolekül nach einem der Ansprüche 1 bis 4, wobei das Bindungsmolekül zwei spezifisch an HER2 bindende, von Fyn-SH3 abgeleitete Polypeptide umfasst und wobei das erste der beiden Polypeptide an den ersten N-Terminus der beiden schweren Ketten des Antikörpers gebunden ist und das zweite der beiden Polypeptide an den zweiten N-Terminus der beiden schweren Ketten des Antikörpers gebunden ist.

8. Bindungsmolekül nach einem der Ansprüche 1 bis 3 oder 5, wobei das Bindungsmolekül zwei spezifisch an EGFR bindende, von Fyn-SH3 abgeleitete Polypeptide umfasst und wobei (i) das erste der beiden Polypeptide an den ersten N-Terminus der beiden schweren Ketten des Antikörpers gebunden ist und das zweite der beiden Polypeptide an den zweiten N-Terminus der beiden schweren Ketten des Antikörpers gebunden ist, (ii) das erste der beiden Polypeptide an den ersten C-Terminus der beiden schweren Ketten des Antikörpers gebunden ist und das zweite der beiden Polypeptide an den zweiten C-Terminus der beiden schweren Ketten des Antikörpers gebunden ist, (iii) das erste der beiden Polypeptide an den ersten N-Terminus der beiden leichten Ketten des Antikörpers gebunden ist und das zweite der beiden Polypeptide an den zweiten N-Terminus der beiden leichten Ketten des Antikörpers gebunden ist oder (iv) das erste der beiden Polypeptide an den ersten C-Terminus der beiden leichten Ketten des Antikörpers gebunden ist und das zweite der beiden Polypeptide an den zweiten C-Terminus der beiden leichten Ketten des Antikörpers gebunden ist.

9. Bindungsmolekül nach einem der Ansprüche 1 bis 3 oder 6, wobei das Bindungsmolekül zwei spezifisch an CD33 bindende, von Fyn-SH3 abgeleitete Polypeptide umfasst, und wobei das erste der beiden Polypeptide an den ersten C-Terminus der beiden leichten Ketten des Antikörpers gebunden ist und das zweite der beiden Polypeptide an den zweiten C-Terminus der beiden leichten Ketten des Antikörpers gebunden ist.

10. Bindungsmolekül nach einem der Ansprüche 1 bis 9, wobei der spezifisch an CD3 bindende Antikörper und das von Fyn-SH3 abgeleitete Polypeptid über einen Linker miteinander verbunden sind, wobei es sich bei dem Linker vorzugsweise um einen Peptid-Linker handelt und wobei der Peptid-Linker besonders bevorzugt eine aus der aus SEQ ID NOs: 162, 160 und 161 bestehenden Gruppe ausgewählte Sequenz aufweist.

11. Nukleinsäuremolekül, codierend für das Bindungsmolekül nach einem der Ansprüche 1 bis 10.

12. Vektor, umfassend das Nukleinsäuremolekül nach Anspruch 11.

13. Wirtszelle oder nichtmenschlicher Wirt, transformiert mit dem Vektor nach Anspruch 12.

14. Pharmazeutische Zusammensetzung, umfassend das Bindungsmolekül nach einem der Ansprüche 1 bis 10, das Nukleinsäuremolekül nach Anspruch 11, den Vektor nach Anspruch 12 und/oder die Wirtszelle oder den nichtmenschlichen Wirt nach Anspruch 13.

15. Pharmazeutische Zusammensetzung nach Anspruch 14 zur Verwendung bei der Behandlung oder Prävention von Krebs.

16. Pharmazeutische Zusammensetzung zur Verwendung nach Anspruch 15, wobei die Krebserkrankung aus der aus Brustkrebs, hämatologischen Malignitäten wie NHL, B-ALL, AML, anderen Krebserkrankungen wie Prostatakrebs, Melanom, Lungenkrebs, Magenkrebs, Eierstockkrebs, Kopf- und Halskrebs, Kolorektalkrebs, Bauchspeicheldrüsenkrebs, Schildrüsenkrebs, Gliom, Blasenkrebs, Nierenkrebs, Leberkrebs oder malignen Asziten bestehenden Gruppe ausgewählt ist und wobei die Krebserkrankung vorzugsweise ein Oberflächenzielantigen exprimiert, das spezifisch von dem Bindungsmolekül nach einem der Ansprüche 1 bis 10 gebunden wird, in einer Menge, die mindestens doppelt so hoch ist wie die auf Nichttumorzellen exprimierte Menge.

**Revendications**

1. Molécule de liaison bispécifique qui se lie spécifiquement à (i) un CD3 et (ii) un antigène cible de surface sur des cellules cancéreuses,
ladite molécule de liaison comprenant

   (a) un anticorps se liant spécifiquement au CD3, et
   (b) un polypeptide dérivé de Fyn-SH3, qui se lie à un antigène cible de surface sur des cellules cancéreuses, dans laquelle ledit polypeptide dérivé de Fyn-SH3 est constitué par la séquence d'acides aminés de SEQ ID n° : 1,

   à condition que

   (i) au moins un acide aminé au sein des positions d'acides aminés 10 à 19 de la SEQ ID n° : 1 soit substitué, supprimé ou ajouté, et
   (ii) au moins un acide aminé au sein des positions d'acides aminés 29 à 36 de la SEQ ID n° : 1 soit substitué, supprimé ou ajouté,

   à condition que ledit polypeptide ait une identité de séquence d'au moins 85% avec la séquence d'acides aminés de SEQ ID n° : 1, dans laquelle la détermination d'une identité exclut les positions d'acides aminés 12 à 17 et 31 à 34 de la SEQ ID n° : 1.

2. Molécule de liaison selon la revendication 1, dans laquelle l'anticorps se liant spécifiquement au CD3 comprend une partie Fc qui permet d'obtenir un allongement de la demi-vie *in vivo* de la molécule de liaison bispécifique.

3. Molécule de liaison selon l'une quelconque des revendications 1 ou 2, la molécule de liaison comprenant deux polypeptides dérivés de Fyn-SH3 qui se lient spécifiquement aux EGFR, HER2, CD33, CD19, CEA, EPHA2, protéoglycane à sulfate de chondroïtine associé à un mélanome, IGFR1, protéine alpha activant les fibroblastes, antigène de cellules souches prostatiques (PSCA), c-MET, EpCAM ou PSMA.

4. Molécule de liaison selon l'une quelconque des revendications 1 à 3, dans laquelle le polypeptide dérivé de Fyn-SH3 se lie spécifiquement à HER2 et comprend n'importe laquelle des séquences d'acides aminés choisies dans le groupe constitué par les SEQ ID n° : 4 et 9 à 159, de préférence la SEQ ID n° : 4.

5. Molécule de liaison selon l'une quelconque des revendications 1 à 3, dans laquelle le polypeptide dérivé de Fyn-SH3 se lie spécifiquement à l'EGFR et comprend n'importe laquelle des séquences d'acides aminés choisies dans le groupe constitué par les SEQ ID n° : 169, 187 à 197 et 201 à 203, de préférence la SEQ ID n° : 169 ou la 187.

6. Molécule de liaison selon l'une quelconque des revendications 1 à 3, dans laquelle le polypeptide dérivé de Fyn-SH3 se lie spécifiquement au CD33 et comprend n'importe laquelle des séquences d'acides aminés choisies dans le groupe constitué par les SEQ ID n° : 174, 177 à 186 et 207, de préférence la SEQ ID n° : 174.

7. Molécule de liaison selon l'une quelconque des revendications 1 à 4, la molécule de liaison comprenant deux polypeptides dérivés de Fyn-SH3 qui se lient spécifiquement à HER2, et dans laquelle le premier desdits deux polypeptides est lié à la première extrémité N-terminale des deux chaînes lourdes dudit anticorps et le deuxième desdits deux polypeptides est lié à la deuxième extrémité N-terminale des deux chaînes lourdes dudit anticorps.

8. Molécule de liaison selon l'une quelconque des revendications 1 à 3 ou 5, la molécule de liaison comprenant deux polypeptides dérivés de Fyn-SH3 qui se lient spécifiquement à l'EGFR, et dans laquelle (i) le premier desdits deux polypeptides est lié à la première extrémité N-terminale des deux chaînes lourdes dudit anticorps et le deuxième desdits deux polypeptides est lié à la deuxième extrémité N-terminale des deux chaînes lourdes dudit anticorps, (ii) le premier desdits deux polypeptides est lié à la première extrémité C-terminale des deux chaînes lourdes dudit anticorps et le deuxième desdits deux polypeptides est lié à la deuxième extrémité C-terminale des deux chaînes lourdes dudit anticorps, (iii) le premier desdits deux polypeptides est lié à la première extrémité N-terminale des deux chaînes légères dudit anticorps et le deuxième desdits deux polypeptides est lié à la deuxième extrémité N-terminale des deux chaînes légères dudit anticorps ou (iv) le premier desdits deux polypeptides est lié à la première extrémité C-terminale des deux chaînes légères dudit anticorps et le deuxième desdits deux polypeptides est lié à la deuxième extrémité C-terminale des deux chaînes légères dudit anticorps.

9. Molécule de liaison selon l'une quelconque des revendications 1 à 3 ou 6, la molécule de liaison comprenant deux polypeptides dérivés de Fyn-SH3 qui se lient spécifiquement au CD33, et dans laquelle le premier desdits deux polypeptides est lié à la première extrémité C-terminale des deux chaînes légères dudit anticorps et le deuxième desdits deux polypeptides est lié à la deuxième extrémité C-terminale des deux chaînes légères dudit anticorps.

10. Molécule de liaison selon l'une quelconque des revendications 1 à 9, dans laquelle l'anticorps se liant spécifiquement au CD33 et le polypeptide dérivé de Fyn-SH3 sont liés par un lieur, de préférence dans laquelle le lieur es un lieur peptidique, mieux préféré dans laquelle le lieur peptidique a une séquence sélectionnée dans le groupe constitué par la SEQ ID n° : 162, 160 et 161.

11. Molécule d'acide nucléique codant pour la molécule de liaison de l'une quelconque des revendications 1 à 10.

12. Vecteur comprenant la molécule d'acide nucléique de la revendication 11.

13. Cellule hôte ou hôte non humain transformé(e) avec le vecteur de la revendication 12.

14. Composition pharmaceutique comprenant la molécule de liaison de l'une quelconque des revendications 1 à 10, la molécule d'acide nucléique de la revendication 11, le vecteur de la revendication 12 et/ou la cellule hôte ou l'hôte non humain de la revendication 13.

15. Composition pharmaceutique de la revendication 14 destinée à être utilisée dans un procédé de traitement ou de prévention d'un cancer.

16. Composition pharmaceutique destinée à être utilisée selon la revendication 15, dans laquelle ledit cancer est sélectionné dans le groupe constitué par un cancer du sein, des malignités hématologiques telles que NHL, B-ALL, AML, d'autres cancers tels qu'un cancer de la prostate, un mélanome, un cancer du poumon, un cancer de l'estomac, un cancer de l'ovaire, un cancer de la tête et du cou, un cancer colorectal, un cancer du pancréas, un cancer de la thyroïde, un gliome, un cancer de la vessie, un cancer du rein, un cancer du foie ou des ascites malignes, de préférence dans laquelle ledit cancer exprime l'antigène cible de surface étant spécifiquement lié par la molécule de liaison de l'une quelconque des revendications 1 à 10, en une quantité qui est au moins deux fois supérieure à la quantité exprimée sur des cellules non tumorales.

Figure 1

Figure 2

**Figure 3**

**A**

**B**

Figure 4

Figure 5

**Figure 6**

**Figure 7**

**Figure 8**

**Figure 9**

**Figure 10**

**Figure 11**

A

**Figure 11- continued**

B

C

D

**Figure 11- continued**

**E**

**Figure 12**

## Figure 13

**Figure 14**

**Figure 15**

A

Figure 15 – continued

B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008022759 A
- US 6080560 A
- US 4946778 A
- WO 2010037835 A
- EP 13164555 A

### Non-patent literature cited in the description

- **MLECNIK B. et al.** *Cancer Metastasis Rev,* 2011, vol. 30, 5-12
- **BAUERLE PA et al.** *Cancer Res,* 2009, vol. 69 (12), 4941-4944
- **BARGOU et al.** *Science,* 2008, vol. 321 (5891), 974-977
- **NORMAN DJ.** *Ther Drug Monit,* 1995, vol. 17 (6), 615-620
- **ALBEROLA-ILA J. et al.** *J Immunol,* 1991, vol. 146 (4), 1085-1092
- **CONRAD et al.** *Cytometry,* 2007, vol. 71 (11), 925-933
- **MAY C. et al.** *Biochemical Pharmacology,* 2012, vol. 84, 1105-1112
- **HUI R et al.** *Asian Pacific Journal of Cancer Prevention,* 2012, vol. 13, 2795-2798
- **CHAN A. ; CARTER P.** *Nat Rev Immunol,* 2010, vol. 10 (5), 301-316
- **STAERZ et al.** *Proc Natl Acad Sci USA,* 1986, vol. 83, 1453-1457
- **KUFER P. et al.** *Trends Biotechnol,* 2004, vol. 22, 238-244
- **LINDHOFER et al.** *J Immunol,* 1995, vol. 155, 219-225
- **LINKE R. et al.** *Mabs,* 2010, vol. 2, 129-136
- **CHAMES P. et al.** *Mabs,* 2009, vol. 1, 539-547
- **JAGER M. et al.** *Cancer Res,* 2012, vol. 69, 4941-4944
- **BAEUERLE PA et al.** *Cancer Res,* 2009, vol. 69, 4941-4944
- **DREIER T. et al.** *J Immunol,* 2003, vol. 170, 4397-4402
- **BRISCHWEIN K. et al.** *Mol Immunol,* 2006, vol. 43, 1129-1143
- **BARGOU R. et al.** *Science,* 2008, vol. 321, 974-977
- **TOPP MS et al.** *J Clin Oncol,* 2011, vol. 29, 2493-2498
- **FRIEDRICH M.** *Mol Cancer Ther,* 2012, vol. 11 (12), 2664-2673
- **BAEUERLE P et al.** *Curr Opin Mol Ther,* 2009, vol. 11, 22-30
- **KONTERMANN R.** *Acta Pharmacologica Sinica,* 2005, vol. 26 (1), 1-9
- **MOORE et al.** *Blood,* 2011, vol. 117, 4542-4551
- **MØLHØJ M. et al.** *Mol Immunol,* 2007, vol. 44 (8), 1935-1943
- **KONTERMANN R.** *Mabs,* 2012, vol. 4 (2), 182-197
- **STROP et al.** *J Mol Biol,* 2012, vol. 420, 204-219
- **HARLOW ; LANE.** Antibodies: A Laboratory Manual. Cold Spring Harbor Laboratory Press, 1988
- **HARLOW ; LANE.** Using Antibodies: A Laboratory Manual. Cold Spring Harbor Laboratory Press, 1999
- **SELA.** *Science,* 1969, vol. 166, 1365
- **LAVER.** *Cell,* 1990, vol. 61, 553-6
- **GRABULOVSKI et al.** *JBC,* 2007, vol. 282, 3196-3204
- **BERTSCHINGER et al.** *Protein Eng Des Sel,* 2007, vol. 20 (2), 57-68
- **GEBAUER ; SKERRA.** *Curr Opinion in Chemical Biology,* 2009, vol. 13, 245-255
- **SCHLATTER et al.** *MAbs,* 2012, vol. 4 (4), 1-12
- **SEMBA et al.** *Proc. Natl. Acad. Sci. U S A,* 1986, vol. 83 (15), 5459-63
- **KAWAKAMI et al.** *Mol Cell Biol.,* 1986, vol. 6 (12), 4195-201
- **COOKE ; PERLMUTTER.** *New Biol.,* 1989, vol. 1 (1), 66-74
- **RESH.** *Int. J. Biochem. Cell Biol.,* 1998, vol. 30 (11), 1159-62
- **HOOGENBOOM et al.** Multi-subunit proteins on the surface of filamentous phage: methodologies for displaying antibody (Fab) heavy and light chains. *Nucleic Acids Res,* 1991, vol. 19 (15), 4133-7
- **XIAOQUIN HUANG ; WEBB MILLER.** *Advances in Applied Mathematics,* 1991, vol. 12, 337-357
- **THOMPSON et al.** *Nucleic Acids Res.,* 1994, vol. 22 (22), 4673-4680

- **STROHL W.** *Curr Opin Biotechnol,* 2009, vol. 20, 685-691
- **JOHNSON G. ; WU T.T.** *Nucleic Acids Res.,* 2000, vol. 28, 214-218
- **SHIELDS RL et al.** *J. Biol. Chem.,* 2001, vol. 276, 6591-6604
- **BAUERLE PA ; REINHARDT C.** *Cancer Res.,* 2009, vol. 69 (12), 4941-4944
- **RADER C.** *Blood,* 2011, vol. 117 (17), 4403-4404
- **WOLF et al.** *Drug Discov Today,* 2005, vol. 10 (18), 1237-1244
- **LUTTERBUESE R. et al.** *PNAS,* 2010, vol. 107 (28), 12605-12610
- **YINGJIE et al.** *Mol Cancer Ther,* 2005, vol. 4 (3), 435-442
- **LATTRICH et al.** *Oncology Reports,* 2008, vol. 19, 811-817
- *CHEMICAL ABSTRACTS,* 876387-05-2
- *CHEMICAL ABSTRACTS,* 881191-44-2
- **RIECHMANN et al.** *Nature,* 1988, vol. 332 (6162), 332-323
- **KASHMIRI et al.** *Methods,* vol. 36 (1), 25-34
- **SCHLECHTER et al.** *Nature,* 1984, vol. 312, 513-516
- **KINCH MS ; CARLES-KINCH K.** *Clin. Exp. Metastasis,* 2003, vol. 20 (1), 59-68
- **UDAYAKUMAR et al.** *Oncogene,* 2011, vol. 30, 4921-4929
- **RAPONI et al.** *Leuk. Lymphoma,* 2011, vol. 52 (6), 1098-1107
- **STRNAD et al.** *Cancer Res.,* 1989, vol. 49 (2), 314-317
- Remington: The Science and Practice of Pharmacy. Pharmaceutical Press, 1990
- **JAGER et al.** *Cancer Research,* 2009, vol. 69 (10), 4270-6
- **HAAS A.** *Immunobiology,* 2009, vol. 214 (6), 441-53
- **WU.** *Journal of Biopharmaceutical Statistics,* 2010, vol. 20 (5), 954-964

SEQUENCE LISTING

<110> Covagen AG

<120> Novel bispecific binding molecules with antitumoral activity

<130> W1579 PCT

<150> EP 13 16 4555.8
<151> 2013-04-19

<160> 209

<170> BiSSAP 1.2

<210> 1
<211> 63
<212> PRT
<213> Homo sapiens

<400> 1

```
        Gly Val Thr Leu Phe Val Ala Leu Tyr Asp Tyr Glu Ala Arg Thr Glu
        1               5                   10                  15
        Asp Asp Leu Ser Phe His Lys Gly Glu Lys Phe Gln Ile Leu Asn Ser
                    20                  25                  30
        Ser Glu Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
            50                  55                  60
```

<210> 2
<211> 449
<212> PRT
<213> Artificial Sequence

<220>
<223> anti-CD3 antibody

<> 2

EP 2 986 630 B1

```
Gln Val Gln Leu Val Gln Ser Gly Gly Gly Val Val Gln Pro Gly Arg
1                   5                   10                  15
Ser Leu Arg Leu Ser Cys Lys Ala Ser Gly Tyr Thr Phe Thr Arg Tyr
            20                  25                  30
Thr Met His Trp Val Arg Gln Ala Pro Gly Lys Gly Leu Glu Trp Ile
            35                  40                  45
Gly Tyr Ile Asn Pro Ser Arg Gly Tyr Thr Asn Tyr Asn Gln Lys Val
        50                  55                  60
Lys Asp Arg Phe Thr Ile Ser Arg Asp Asn Ser Lys Asn Thr Ala Phe
65                  70                  75                  80
Leu Gln Met Asp Ser Leu Arg Pro Glu Asp Thr Gly Val Tyr Phe Cys
                85                  90                  95
Ala Arg Tyr Tyr Asp Asp His Tyr Cys Leu Asp Tyr Trp Gly Gln Gly
            100                 105                 110
Thr Pro Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
                180                 185                 190
Ser Ser Leu Gly Thr Gln Thr Tyr Ile Cys Asn Val Asn His Lys Pro
        195                 200                 205
Ser Asn Thr Lys Val Asp Lys Lys Val Glu Pro Lys Ser Cys Asp Lys
    210                 215                 220
Thr His Thr Cys Pro Pro Cys Pro Ala Pro Glu Ala Ala Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
            275                 280                 285
Ala Lys Thr Lys Pro Arg Glu Glu Gln Tyr Asn Ser Thr Tyr Arg Val
        290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
            325                 330                 335
Thr Ile Ser Lys Ala Lys Gly Gln Pro Arg Glu Pro Gln Val Tyr Thr
            340                 345                 350
Leu Pro Pro Ser Arg Glu Glu Met Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
            435                 440                 445
Lys
```

<210> 3
<211> 213

<212> PRT
<213> Artificial Sequence

<220>
<223> anti-CD3 antibody

<400> 3

```
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Val Ser Tyr Met
            20                  25                  30
Asn Trp Tyr Gln Gln Thr Pro Gly Lys Ala Pro Lys Arg Trp Ile Tyr
            35                  40                  45
Asp Thr Ser Lys Leu Ala Ser Gly Val Pro Ser Arg Phe Ser Gly Ser
        50                  55                  60
Gly Ser Gly Thr Asp Tyr Thr Phe Thr Ile Ser Ser Leu Gln Pro Glu
65                  70                  75                  80
Asp Ile Ala Thr Tyr Tyr Cys Gln Gln Trp Ser Ser Asn Pro Phe Thr
                85                  90                  95
Phe Gly Gln Gly Thr Lys Leu Gln Ile Thr Arg Thr Val Ala Ala Pro
        100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
        130                 135                 140

Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
            165                 170                 175
Thr Leu Thr Leu Ser Lys Ala Asp Tyr Glu Lys His Lys Val Tyr Ala
        180                 185                 190
Cys Glu Val Thr His Gln Gly Leu Ser Ser Pro Val Thr Lys Ser Phe
        195                 200                 205
Asn Arg Gly Glu Cys
        210
```

<210> 4
<211> 63
<212> PRT
<213> Artificial Sequence

<220>
<223> anti-HER2 Fynomer C12

<400> 4

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Met
            20                  25                  30
Glu Asp Gly Val Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 5
<211> 68

<212> PRT
<213> Artificial Sequence

<220>
<223> anti-HER2 Fynomer

<220>
<221> VARIANT
<222> 12,13,14,15,16,17,18,34,35,36,41,44,53,55,59
<223>

```
        Gly, Val, Thr, Leu, Phe, Ala, Tyr, Asp, Ser, His, Lys, Glu, Gln,
        Ile, Trp, Arg, Met, Pro, Asn, Cys
```

<220>
<221> VARIANT
<222> 19,20,21,37,38,39
<223>

```
        Gly, Val, Thr, Leu, Phe, Ala, Tyr, Asp, Ser, His, Lys, Glu, Gln,
        Ile, Trp, Arg, Met, Pro, Asn, Cys
```

<400> 5

```
        Gly Val Thr Leu Phe Val Ala Leu Tyr Asp Tyr Xaa Xaa Xaa Xaa Xaa
        1               5               10              15
        Xaa Xaa Xaa Xaa Xaa Leu Ser Phe His Lys Gly Glu Lys Phe Gln Ile
                    20              25              30
        Leu Xaa Xaa Xaa Xaa Xaa Xaa Gly Xaa Trp Trp Xaa Ala Arg Ser Leu
                35              40              45
        Thr Thr Gly Glu Xaa Gly Xaa Ile Pro Ser Xaa Tyr Val Ala Pro Val
            50              55              60
        Asp Ser Ile Gln
        65
```

<210> 6
<211> 68
<212> PRT
<213> Artificial Sequence

<220>
<223> anti-HER2 Fynomer

<220>
<221> VARIANT
<222> 12
<223> Thr, Glu, Asp, Gln, Tyr, Val, Trp, Asn, Ser, Phe, Lys

<220>
<221> VARIANT
<222> 13
<223> Ser, Ala, Arg, Thr

<220>
<221> VARIANT
<222> 14

<223> Tyr, Arg, His, Thr, Asn, Val, Trp, Ser

<220>
<221> VARIANT
<222> 15
<223> Asn, Asp, Met, Tyr, Arg, Pro, Glu, Leu, His, Thr, Gly, Phe

<220>
<221> VARIANT
<222> 16
<223>

```
Thr, Ser, Pro, Gln, Arg, Lys, Gly, Ala, Asp, Met, Asn, Leu, Phe,
Tyr, Glu
```

<220>
<221> VARIANT
<222> 17
<223>

```
Arg, Met, Lys, Asp, Phe, Thr, Gly, His, Ser, Pro, Asn, Gln, Tyr,
Leu, Ala, Pro
```

<220>
<221> VARIANT
<222> 19
<223> Asp, Gly, Val, Leu, His, Asn, Arg, Phe, Ser, Ala

<220>
<221> VARIANT
<222> 19
<223> Gly, Ser, Glu, Asp, Pro, Tyr or is absent

<220>
<221> VARIANT
<222> 20
<223> Gln, Asp, Ser, His or is absent

<220>
<221> VARIANT
<222> 21
<223> Asp, Val or is absent

<220>
<221> VARIANT
<222> 34
<223>

```
Arg, Lys, Gln, Asn, Ser, Gly, Trp, Met, His, Leu, Phe, Glu, Thr,
Pro, Ala, Asp, Val
```

<220>
<221> VARIANT
<222> 35
<223>

```
        Met, Arg, Glu, Gly, Asn, Asp, Ser, Ala, Gln, Phe, Pro, Lys, Tyr,
        Thr, His, Val, Leu, Trp
```

<220>
<221> VARIANT
<222> 36
<223>

```
        Glu, Trp, Pro, Arg, Lys, Ser, Val, Asn, Asp, His, Gly, Thr, Gln,
        Ala, Tyr, Leu, Met
```

<220>
<221> VARIANT
<222> 37
<223> Asp, Arg, Gln, Ser, Ala, Asn, Pro, Ile, His, Thr, Tyr, Glu, Leu, Lys, Met, Val, Ile, Trp or is absent

<220>
<221> VARIANT
<222> 38
<223> Gly, Ser, Ile, Leu, Ala, Val, Thr, Glu, Asp, Gln, Arg, Pro, Lys, Met, His, Tyr or is absent

<220>
<221> VARIANT
<222> 39
<223> Lys, Gly, Arg, Ala, Thr, Val, Ser, Ile, Glu, Gln, Pro, Asp, Asn, His or is absent

<220>
<221> VARIANT
<222> 41
<223> Val, Asp, Thr, Ile, Tyr

<220>
<221> VARIANT
<222> 44
<223> Glu, Ala, Arg, Thr, Gln

<220>
<221> VARIANT
<222> 53
<223> Thr, Ile, Val

<220>
<221> VARIANT
<222> 55
<223> Tyr, Leu, Phe

<220>
<221> VARIANT
<222> 59
<223> Asn, Ser

<400> 6

```
Gly Val Thr Leu Phe Val Ala Leu Tyr Asp Tyr Xaa Xaa Xaa Xaa Xaa
1               5                   10              15
Xaa Xaa Xaa Xaa Xaa Leu Ser Phe His Lys Gly Glu Lys Phe Gln Ile
        20                  25                  30
Leu Xaa Xaa Xaa Xaa Xaa Xaa Gly Xaa Trp Trp Xaa Ala Arg Ser Leu
        35                  40                  45

Thr Thr Gly Glu Xaa Gly Xaa Ile Pro Ser Xaa Tyr Val Ala Pro Val
    50              55                  60
Asp Ser Ile Gln
65
```

<210> 7
<211> 7
<212> PRT
<213> Artificial Sequence

<220>
<223> mutated RT-loop

<400> 7

```
Thr Ser Tyr Asn Thr Arg Asp
1                   5
```

<210> 8
<211> 4
<212> PRT
<213> Artificial Sequence

<220>
<223> mutated SRC-loop

<400> 8
Arg Met Glu Asp 1

<210> 9
<211> 67
<212> PRT
<213> Artificial Sequence

<220>
<223> anti-HER2 Fynomer

<400> 9

```
Gly Val Thr Leu Phe Val Ala Leu Tyr Asp Tyr Glu Ala Arg Asp Ser
1               5                   10              15
Met Gly Gly Gln Asp Leu Ser Phe His Lys Gly Glu Lys Phe Gln Ile
        20                  25                  30
Leu Lys Arg Trp Arg Gly Gly Asp Trp Trp Glu Ala Arg Ser Leu Thr
        35                  40                  45
Thr Gly Glu Thr Gly Tyr Ile Pro Ser Asn Tyr Val Ala Pro Val Asp
    50              55                  60
Ser Ile Gln
65
```

<210> 10
<211> 67
<212> PRT
<213> Artificial Sequence

<220>
<223> anti-HER2 Fynomer

<400> 10

```
Gly Val Thr Leu Phe Val Ala Leu Tyr Asp Tyr Glu Ala Arg Asp Ser
1               5                   10                  15
Met Gly Gly Gln Asp Leu Ser Phe His Lys Gly Glu Lys Phe Gln Ile
            20                  25                  30
Leu Thr Arg Trp Ala Gln Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
65
```

<210> 11
<211> 64
<212> PRT
<213> Artificial Sequence

<220>
<223> anti-HER2 Fynomer

<400> 11

```
Gly Val Thr Leu Phe Val Ala Leu Tyr Asp Tyr Gln Ala Tyr Gly Met
1               5                   10                  15
Tyr Asp Leu Ser Phe His Lys Gly Glu Lys Phe Gln Ile Leu Ser Pro
            20                  25                  30
Lys Asp Thr Gly Tyr Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 12
<211> 66
<212> PRT
<213> Artificial Sequence

<220>
<223> anti-HER2 Fynomer

<400> 12

```
Gly Val Thr Leu Phe Val Ala Leu Tyr Asp Tyr Glu Ala Arg Glu Phe
1               5                   10              15
Met Gly Gly Gln Asp Leu Ser Phe His Lys Gly Glu Lys Phe Gln Ile
            20                  25              30
Leu Thr Met Trp Lys Gly Asp Trp Trp Glu Ala Arg Ser Leu Thr Thr
            35                  40              45
Gly Glu Thr Gly Tyr Ile Pro Ser Asn Tyr Val Ala Pro Val Asp Ser
        50                  55                  60
Ile Gln
    65
```

<210> 13
<211> 67
<212> PRT
<213> Artificial Sequence

<220>
<223> anti-HER2 Fynomer

<400> 13

```
Gly Val Thr Leu Phe Val Ala Leu Tyr Asp Tyr Glu Ala Arg Pro Tyr
1               5                   10              15
Leu Gly Gly Gln Asp Leu Ser Phe His Lys Gly Glu Lys Phe Gln Ile
            20                  25              30
Leu His Ala Ser Met Leu Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60

                    Ser Ile Gln
                        65
```

<210> 14
<211> 63
<212> PRT
<213> Artificial Sequence

<220>
<223> anti-HER2 Fynomer

<400> 14

```
Gly Val Thr Leu Phe Val Ala Leu Tyr Asp Tyr Thr Ser Tyr Asn Gln
1               5                   10              15
His Asp Leu Ser Phe His Lys Gly Glu Lys Phe Gln Ile Leu Gly Asp
            20                  25              30
Asn Ile Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 15
<211> 67
<212> PRT
<213> Artificial Sequence

<220>
<223> anti-HER2 Fynomer

<400> 15

```
        Gly Val Thr Leu Phe Val Ala Leu Tyr Asp Tyr Glu Ala Arg Leu Ser
        1               5               10              15
        Ser His Pro His Val Leu Ser Phe His Lys Gly Glu Lys Phe Gln Ile
                20              25              30
        Leu Asn Arg Val Ser Ile Gly Asp Trp Trp Glu Ala Arg Ser Leu Thr
                35              40              45
        Thr Gly Glu Thr Gly Tyr Ile Pro Ser Asn Tyr Val Ala Pro Val Asp
                50              55              60
        Ser Ile Gln
        65
```

<210> 16
<211> 67
<212> PRT
<213> Artificial Sequence

<220>
<223> anti-HER2 Fynomer

<400> 16

```
        Gly Val Thr Leu Phe Val Ala Leu Tyr Asp Tyr Glu Ala Arg Pro Tyr
        1               5               10              15
        Leu Gly Gly Gln Asp Leu Ser Phe His Lys Gly Glu Lys Phe Gln Ile
                20              25              30
        Leu Asn His Pro Pro Thr Gly Asp Trp Trp Glu Ala Arg Ser Leu Thr
                35              40              45
        Thr Gly Glu Thr Gly Tyr Ile Pro Ser Asn Tyr Val Ala Pro Val Asp
                50              55              60
        Ser Ile Gln
        65
```

<210> 17
<211> 65
<212> PRT
<213> Artificial Sequence

<220>
<223> anti-HER2 Fynomer

<400> 17

```
        Gly Val Thr Leu Phe Val Ala Leu Tyr Asp Tyr Glu Ala Tyr Asp Leu
        1               5               10              15
        Ser Arg Pro Asp Leu Ser Phe His Lys Gly Glu Lys Phe Gln Ile Leu
                20              25              30
        Asn Ser Ser Glu Gly Thr Trp Trp Glu Ala Arg Ser Leu Thr Thr Gly
                35              40              45
        Glu Thr Gly Phe Ile Pro Ser Asn Tyr Val Ala Pro Val Asp Ser Ile
                50              55              60
        Gln
        65
```

<210> 18
<211> 67
<212> PRT
<213> Artificial Sequence

<220>
<223> anti-HER2 Fynomer

<400> 18

```
Gly Val Thr Leu Phe Val Ala Leu Tyr Asp Tyr Glu Ala Arg Met Pro
1               5                   10                  15
Lys Val Ser Asp Leu Ser Phe His Lys Gly Glu Lys Phe Gln Ile Leu
            20                  25                  30
Gln Glu Pro Gln Ser Lys Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
65
```

<210> 19
<211> 67
<212> PRT
<213> Artificial Sequence

<220>
<223> anti-HER2 Fynomer

<400> 19

```
Gly Val Thr Leu Phe Val Ala Leu Tyr Asp Tyr Glu Ala Arg Pro Gly
1               5                   10                  15
Arg His Ser Ser Asp Leu Ser Phe His Lys Gly Glu Lys Phe Gln Ile
            20                  25                  30
Leu His Gln Ser Asn Val Gly Asp Trp Trp Glu Ala Arg Ser Leu Thr
        35                  40                  45
Thr Gly Glu Thr Gly Tyr Ile Pro Ser Asn Tyr Val Ala Pro Val Asp
        50                  55                  60
Ser Ile Gln
65
```

<210> 20
<211> 64
<212> PRT
<213> Artificial Sequence

<220>
<223> anti-HER2 Fynomer

<400> 20

```
Gly Val Thr Leu Phe Val Ala Leu Tyr Asp Tyr Gln Thr Thr Arg Pro
1               5                   10                  15
His Asp Leu Ser Phe His Lys Gly Glu Lys Phe Gln Ile Leu Gly Arg
            20                  25                  30
Thr Gln Ile Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
            50                  55                  60
```

<210> 21
<211> 63
<212> PRT
<213> Artificial Sequence

<220>
<223> anti-HER2 Fynomer

<400> 21

```
Gly Val Thr Leu Phe Val Ala Leu Tyr Asp Tyr Gln Thr Asn Asn Ser
1               5                   10                  15
Arg Asp Leu Ser Phe His Lys Gly Glu Lys Phe Gln Ile Leu Gly Asn
            20                  25                  30
Thr Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 22
<211> 63
<212> PRT
<213> Artificial Sequence

<220>
<223> anti-HER2 Fynomer

<400> 22

```
Gly Val Thr Leu Phe Val Ala Leu Tyr Asp Tyr Tyr Ser Tyr Asn Thr
1               5                   10                  15
His Asp Leu Ser Phe His Lys Gly Glu Lys Phe Gln Ile Leu Ser Arg
            20                  25                  30
Ala Asn Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 23
<211> 63
<212> PRT
<213> Artificial Sequence

<220>
<223> anti-HER2 Fynomer

<400> 23

```
Gly Val Thr Leu Phe Val Ala Leu Tyr Asp Tyr Tyr Ser Tyr Asn Asn
1               5                   10              15
His Asp Leu Ser Phe His Lys Gly Glu Lys Phe Gln Ile Leu Gln Glu
                20                  25                  30
Leu Gln Gly Asp Trp Trp Glu Ala Arg Ser Leu Thr Thr Gly Glu Thr
        35                  40                  45
Gly Tyr Ile Pro Ser Asn Tyr Val Ala Pro Val Asp Ser Ile Gln
        50                  55                  60
```

<210> 24
<211> 63
<212> PRT
<213> Artificial Sequence

<220>
<223> anti-HER2 Fynomer

<400> 24

```
Gly Val Thr Leu Phe Val Ala Leu Tyr Asp Tyr Arg Ser Tyr Asn Thr
1               5                   10              15
Arg Asp Leu Ser Phe His Lys Gly Glu Lys Phe Gln Ile Leu Thr Lys
                20                  25                  30
Ser Ser Gly Asp Trp Trp Glu Ala Arg Ser Leu Thr Thr Gly Glu Thr
        35                  40                  45
Gly Tyr Ile Pro Ser Asn Tyr Val Ala Pro Val Asp Ser Ile Gln
        50                  55                  60
```

<210> 25
<211> 65
<212> PRT
<213> Artificial Sequence

<220>
<223> anti-HER2 Fynomer

<400> 25

```
Gly Val Thr Leu Phe Val Ala Leu Tyr Asp Tyr Thr Ser Tyr Asn Thr
1               5                   10              15
Arg Asp Leu Ser Phe His Lys Gly Glu Lys Phe Gln Ile Leu Lys Ala
                20                  25                  30
His Ser Leu Ala Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
        50                  55                  60
Gln
65
```

<210> 26
<211> 65
<212> PRT
<213> Artificial Sequence

<220>
<223> anti-HER2 Fynomer

<400> 26

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Glu
            20                  25                  30
Gln Asp Leu Arg Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
    50                  55                  60
Gln
65
```

<210> 27
<211> 63
<212> PRT
<213> Artificial Sequence

<220>
<223> anti-HER2 Fynomer

<400> 27

```
Gly Val Thr Leu Phe Val Ala Leu Tyr Asp Tyr Thr Ser Tyr Asn Lys
1               5                   10                  15
Leu Asp Leu Ser Phe His Lys Gly Glu Lys Phe Gln Ile Leu Arg Thr
            20                  25                  30
Asp Ala Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 28
<211> 63
<212> PRT
<213> Artificial Sequence

<220>
<223> anti-HER2 Fynomer

<400> 28

```
Gly Val Thr Leu Phe Val Ala Leu Tyr Asp Tyr Thr Ser Tyr Asn Gln
1               5                   10                  15
Arg Asp Leu Ser Phe His Lys Gly Glu Lys Phe Gln Ile Leu Gly Pro
            20                  25                  30
Lys Leu Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 29
<211> 63
<212> PRT
<213> Artificial Sequence

<220>
<223> anti-HER2 Fynomer

<400> 29

```
Gly Val Thr Leu Phe Val Ala Leu Tyr Asp Tyr Thr Ser Tyr Asn Asn
1               5                   10                  15
His Asp Leu Ser Phe His Lys Gly Glu Lys Phe Gln Ile Leu Asn Gln
            20                  25                  30
His Asn Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 30
<211> 63
<212> PRT
<213> Artificial Sequence

<220>
<223> anti-HER2 Fynomer

<400> 30

```
Gly Val Thr Leu Phe Val Ala Leu Tyr Asp Tyr Thr Ser Tyr Asn Leu
1               5                   10                  15
Arg Asp Leu Ser Phe His Lys Gly Glu Lys Phe Gln Ile Leu Asn Ser
            20                  25                  30
Ser Glu Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 31
<211> 65
<212> PRT
<213> Artificial Sequence

<220>
<223> anti-HER2 Fynomer

<400> 31

```
Gly Val Thr Leu Phe Val Ala Leu Tyr Asp Tyr Thr Ser Tyr Asn Pro
1               5                   10                  15
Arg Asp Leu Ser Phe His Lys Gly Glu Lys Phe Gln Ile Leu Gly Ala
            20                  25                  30
Thr Asp Ala Thr Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 32
<211> 63
<212> PRT
<213> Artificial Sequence

<220>
<223> anti-HER2 Fynomer

<400> 32

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Asn Glu
            20                  25                  30
Leu Asn Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 33
<211> 63
<212> PRT
<213> Artificial Sequence

<220>
<223> anti-HER2 Fynomer

<400> 33

```
Gly Val Thr Leu Phe Val Ala Leu Tyr Asp Tyr Thr Ser Tyr Asn Ser
1               5                   10                  15
Ser Asp Leu Ser Phe His Lys Gly Glu Lys Phe Gln Ile Leu Arg Glu
            20                  25                  30
Ala Asp Gly Asp Trp Trp Glu Ala Arg Ser Leu Thr Thr Gly Glu Thr
            35                  40                  45

Gly Tyr Ile Pro Ser Asn Tyr Val Ala Pro Val Asp Ser Ile Gln
        50                  55                  60
```

<210> 34
<211> 67
<212> PRT
<213> Artificial Sequence

<220>
<223> anti-HER2 Fynomer

<400> 34

```
Gly Val Thr Leu Phe Val Ala Leu Tyr Asp Tyr Glu Ala Arg Arg Asp
1               5                   10                  15
His Ser Pro His Asp Leu Ser Phe His Lys Gly Glu Lys Phe Gln Ile
            20                  25                  30
Leu Asn Leu Tyr Gln Val Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
65
```

<210> 35
<211> 66
<212> PRT
<213> Artificial Sequence

<220>

<223> anti-HER2 Fynomer

<400> 35

```
        Gly Val Thr Leu Phe Val Ala Leu Tyr Asp Tyr Glu Ala Arg Glu Ala
        1               5                   10                  15
        Leu Gly Gly Gln Asp Leu Ser Phe His Lys Gly Glu Lys Phe Gln Ile
                    20                  25                  30
        Leu Ser Pro Gln Thr Gly Asp Trp Trp Glu Ala Arg Ser Leu Thr Thr
                    35                  40                  45
        Gly Glu Thr Gly Tyr Ile Pro Ser Asn Tyr Val Ala Pro Val Asp Ser
                    50                  55                  60
        Ile Gln
        65
```

<210> 36
<211> 64
<212> PRT
<213> Artificial Sequence

<220>
<223> anti-HER2 Fynomer

<400> 36

```
        Gly Val Thr Leu Phe Val Ala Leu Tyr Asp Tyr Tyr Ser Val His Pro
        1               5                   10                  15
        Gly Asp Leu Ser Phe His Lys Gly Glu Lys Phe Gln Ile Leu Ser Asn
                    20                  25                  30
        Tyr Gln Val Gly Asp Trp Trp Glu Ala Arg Ser Leu Thr Thr Gly Glu
                    35                  40                  45
        Thr Gly Tyr Ile Pro Ser Asn Tyr Val Ala Pro Val Asp Ser Ile Gln
                    50                  55                  60
```

<210> 37
<211> 63
<212> PRT
<213> Artificial Sequence

<220>
<223> anti-HER2 Fynomer

<400> 37

```
        Gly Val Thr Leu Phe Val Ala Leu Tyr Asp Tyr Tyr Ser Tyr Asn Ser
        1               5                   10                  15
        Arg Asp Leu Ser Phe His Lys Gly Glu Lys Phe Gln Ile Leu Ala Gln
                    20                  25                  30
        His Asp Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                    50                  55                  60
```

<210> 38
<211> 63
<212> PRT
<213> Artificial Sequence

<220>
<223> anti-HER2 Fynomer

<400> 38

```
        Gly Val Thr Leu Phe Val Ala Leu Tyr Asp Tyr Thr Ser Tyr Asn Met
        1               5                   10                  15
        Arg Asp Leu Ser Phe His Lys Gly Glu Lys Phe Gln Ile Leu Gly Arg
                    20                  25                  30
        Gly Pro Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                50                  55                  60
```

<210> 39
<211> 65
<212> PRT
<213> Artificial Sequence

<220>
<223> anti-HER2 Fynomer

<400> 39

```
        Gly Val Thr Leu Phe Val Ala Leu Tyr Asp Tyr Thr Ser Tyr Asn Thr
        1               5                   10                  15
        Arg Asp Leu Ser Phe His Lys Gly Glu Lys Phe Gln Ile Leu Gly Arg
                    20                  25                  30
        Pro Arg Asp Val Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                50                  55                  60
        Gln
        65
```

<210> 40
<211> 63
<212> PRT
<213> Artificial Sequence

<220>
<223> anti-HER2 Fynomer

<400> 40

```
        Gly Val Thr Leu Phe Val Ala Leu Tyr Asp Tyr Thr Ser Tyr Asn Thr
        1               5                   10                  15
        Arg Asp Leu Ser Phe His Lys Gly Glu Lys Phe Gln Ile Leu His Thr
                    20                  25                  30
        Thr Lys Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                50                  55                  60
```

<210> 41
<211> 65
<212> PRT
<213> Artificial Sequence

<220>
<223> anti-HER2 Fynomer

<400> 41

```
        Gly Val Thr Leu Phe Val Ala Leu Tyr Asp Tyr Thr Ser Tyr Asn Thr
        1               5                   10                  15
        Thr Asp Leu Ser Phe His Lys Gly Glu Lys Phe Gln Ile Leu His Trp
                    20                  25                  30
        Asn Gly Gly Ser Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                    50                  55                  60
        Gln
        65
```

<210> 42
<211> 63
<212> PRT
<213> Artificial Sequence

<220>
<223> anti-HER2 Fynomer

<400> 42

```
        Gly Val Thr Leu Phe Val Ala Leu Tyr Asp Tyr Thr Ser Tyr Asn Thr
        1               5                   10                  15
        Thr Asp Leu Ser Phe His Lys Gly Glu Lys Phe Gln Ile Leu Asn Asn
                    20                  25                  30
        Thr Thr Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                    50                  55                  60
```

<210> 43
<211> 65
<212> PRT
<213> Artificial Sequence

<220>
<223> anti-HER2 Fynomer

<400> 43

```
        Gly Val Thr Leu Phe Val Ala Leu Tyr Asp Tyr Trp Ser Tyr Asn Ser
        1               5                   10                  15
        Arg Asp Leu Ser Phe His Lys Gly Glu Lys Phe Gln Ile Leu Asn Pro
                    20                  25                  30
        Glu Glu Thr Ser Gly Asp Trp Trp Glu Ala Arg Ser Leu Thr Thr Gly

                    35                  40                  45
        Glu Thr Gly Tyr Ile Pro Ser Asn Tyr Val Ala Pro Val Asp Ser Ile
                    50                  55                  60
        Gln
        65
```

<210> 44

<211> 65
<212> PRT
<213> Artificial Sequence

<220>
<223> anti-HER2 Fynomer

<400> 44

```
Gly Val Thr Leu Phe Val Ala Leu Tyr Asp Tyr Thr Ser Tyr Asn Pro
1               5                   10                  15
Arg Asp Leu Ser Phe His Lys Gly Glu Lys Phe Gln Ile Leu Asn Pro
            20                  25                  30
Arg Gln Arg Ile Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
        50                  55                  60
Gln
65
```

<210> 45
<211> 63
<212> PRT
<213> Artificial Sequence

<220>
<223> anti-HER2 Fynomer

<400> 45

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Asn Arg
            20                  25                  30
Pro Met Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 46
<211> 65
<212> PRT
<213> Artificial Sequence

<220>
<223> anti-HER2 Fynomer

<400> 46

```
Gly Val Thr Leu Phe Val Ala Leu Tyr Asp Tyr Thr Ser Tyr Asn Gln
1               5                   10                  15
His Asp Leu Ser Phe His Lys Gly Glu Lys Phe Gln Ile Leu Pro Thr
                20                  25                  30
Thr Asp Thr Ala Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 47
<211> 63
<212> PRT
<213> Artificial Sequence

<220>
<223> anti-HER2 Fynomer

<400> 47

```
Gly Val Thr Leu Phe Val Ala Leu Tyr Asp Tyr Thr Ser Tyr Asn Thr
1               5                   10                  15
Met Asp Leu Ser Phe His Lys Gly Glu Lys Phe Gln Ile Leu Arg Glu
                20                  25                  30
Thr Thr Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 48
<211> 63
<212> PRT
<213> Artificial Sequence

<220>
<223> anti-HER2 Fynomer

<400> 48

```
Gly Val Thr Leu Phe Val Ala Leu Tyr Asp Tyr Thr Ser Tyr Asn Thr
1               5                   10                  15
His Asp Leu Ser Phe His Lys Gly Glu Lys Phe Gln Ile Leu Arg Gln
                20                  25                  30
Asn Asn Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 49
<211> 63
<212> PRT
<213> Artificial Sequence

<220>
<223> anti-HER2 Fynomer

<400> 49

```
Gly Val Thr Leu Phe Val Ala Leu Tyr Asp Tyr Thr Ser Tyr Asn Ser
1               5                   10                  15
Thr Asp Leu Ser Phe His Lys Gly Glu Lys Phe Gln Ile Leu Ser Ala
            20                  25                  30
Pro Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
            50                  55                  60
```

<210> 50
<211> 63
<212> PRT
<213> Artificial Sequence

<220>
<223> anti-HER2 Fynomer

<400> 50

```
Gly Val Thr Leu Phe Val Ala Leu Tyr Asp Tyr Thr Ser Tyr Asn Ala
1               5                   10                  15
Arg Asp Leu Ser Phe His Lys Gly Glu Lys Phe Gln Ile Leu Ser Ala
            20                  25                  30
Pro Thr Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
            50                  55                  60
```

<210> 51
<211> 65
<212> PRT
<213> Artificial Sequence

<220>
<223> anti-HER2 Fynomer

<400> 51

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Asp
            20                  25                  30
Ala Thr Leu Glu Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 52
<211> 65
<212> PRT
<213> Artificial Sequence

<220>
<223> anti-HER2 Fynomer

<400> 52

```
Gly Val Thr Leu Phe Val Ala Leu Tyr Asp Tyr Thr Ser Tyr Asn Lys
1               5                   10                  15
Arg Asp Leu Ser Phe His Lys Gly Glu Lys Phe Gln Ile Leu Ser Glu
            20                  25                  30
Thr Ser Pro Glu Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 53
<211> 65
<212> PRT
<213> Artificial Sequence

<220>
<223> anti-HER2 Fynomer

<400> 53

```
Gly Val Thr Leu Phe Val Ala Leu Tyr Asp Tyr Val Ser Tyr Asn Thr
1               5                   10                  15
Thr Asp Leu Ser Phe His Lys Gly Glu Lys Phe Gln Ile Leu Ser His
            20                  25                  30

Thr Thr Ser Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 54
<211> 63
<212> PRT
<213> Artificial Sequence

<220>
<223> anti-HER2 Fynomer

<400> 54

```
Gly Val Thr Leu Phe Val Ala Leu Tyr Asp Tyr Thr Ser Tyr Asn Thr
1               5                   10                  15
Thr Asp Leu Ser Phe His Lys Gly Glu Lys Phe Gln Ile Leu Ser Met
            20                  25                  30
Ala Val Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
            50                  55                  60
```

<210> 55
<211> 65
<212> PRT
<213> Artificial Sequence

<220>

<223> anti-HER2 Fynomer

<400> 55

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Asn
            20                  25                  30
Gly Pro Asp Pro Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
    50                  55                  60
Gln
65
```

<210> 56
<211> 63
<212> PRT
<213> Artificial Sequence

<220>
<223> anti-HER2 Fynomer

<400> 56

```
Gly Val Thr Leu Phe Val Ala Leu Tyr Asp Tyr Thr Ser Tyr Asn Thr
1               5                   10                  15
Thr Asp Leu Ser Phe His Lys Gly Glu Lys Phe Gln Ile Leu Ser Asn
            20                  25                  30
Pro Pro Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 57
<211> 65
<212> PRT
<213> Artificial Sequence

<220>
<223> anti-HER2 Fynomer

<400> 57

```
Gly Val Thr Leu Phe Val Ala Leu Tyr Asp Tyr Asn Ser Tyr Asn Lys
1               5                   10                  15
Arg Asp Leu Ser Phe His Lys Gly Glu Lys Phe Gln Ile Leu Ser Gln
            20                  25                  30
Ala Ala Glu Asp Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
    50                  55                  60
Gln
65
```

<210> 58
<211> 63
<212> PRT

<213> Artificial Sequence

<220>
<223> anti-HER2 Fynomer

<400> 58

```
Gly Val Thr Leu Phe Val Ala Leu Tyr Asp Tyr Thr Ser Tyr Asn Thr
1               5                   10                  15
Lys Asp Leu Ser Phe His Lys Gly Glu Lys Phe Gln Ile Leu Ser Gln
            20                  25                  30
Ser Gly Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 59
<211> 65
<212> PRT
<213> Artificial Sequence

<220>
<223> anti-HER2 Fynomer

<400> 59

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Tyr
            20                  25                  30
Pro Arg Thr Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 60
<211> 65
<212> PRT
<213> Artificial Sequence

<220>
<223> anti-HER2 Fynomer

<400> 60

```
Gly Val Thr Leu Phe Val Ala Leu Tyr Asp Tyr Thr Ser Tyr Asn Glu
1               5                   10                  15
Arg Asp Leu Ser Phe His Lys Gly Glu Lys Phe Gln Ile Leu Thr Lys
            20                  25                  30
Thr Pro Arg Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 61
<211> 65
<212> PRT
<213> Artificial Sequence

<220>
<223> anti-HER2 Fynomer

<400> 61

```
Gly Val Thr Leu Phe Val Ala Leu Tyr Asp Tyr Val Ser Tyr Asn Thr
1               5               10              15
Asn Asp Leu Ser Phe His Lys Gly Glu Lys Phe Gln Ile Leu Asp Ser
            20              25              30
Gln Glu Pro Asn Gly Asp Trp Trp Glu Ala Arg Ser Leu Thr Thr Gly
        35              40              45
Glu Thr Gly Tyr Ile Pro Ser Asn Tyr Val Ala Pro Val Asp Ser Ile
    50              55              60
Gln
65
```

<210> 62
<211> 65
<212> PRT
<213> Artificial Sequence

<220>
<223> anti-HER2 Fynomer

<400> 62

```
Gly Val Thr Leu Phe Val Ala Leu Tyr Asp Tyr Thr Ser Tyr Asn Gln
1               5               10              15
His Asp Leu Ser Phe His Lys Gly Glu Lys Phe Gln Ile Leu Gly Gln
            20              25              30
Tyr Pro Lys Thr Gly Asp Trp Trp Glu Ala Arg Ser Leu Thr Thr Gly
        35              40              45
Glu Thr Gly Tyr Ile Pro Ser Asn Tyr Val Ala Pro Val Asp Ser Ile
    50              55              60
Gln
65
```

<210> 63
<211> 65
<212> PRT
<213> Artificial Sequence

<220>
<223> anti-HER2 Fynomer

<400> 63

```
Gly Val Thr Leu Phe Val Ala Leu Tyr Asp Tyr Thr Ser Tyr Asn Asn
1               5                   10                  15
Thr Asp Leu Ser Phe His Lys Gly Glu Lys Phe Gln Ile Leu Lys Gln
            20                  25                  30
Gln Ala Gly Asp Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
        50                  55                  60
Gln
65
```

<210> 64
<211> 65
<212> PRT
<213> Artificial Sequence

<220>
<223> anti-HER2 Fynomer

<400> 64

```
Gly Val Thr Leu Phe Val Ala Leu Tyr Asp Tyr Thr Ser Tyr Asn Asn
1               5                   10                  15
Thr Asp Leu Ser Phe His Lys Gly Glu Lys Phe Gln Ile Leu Asn Ala
            20                  25                  30
His Gln Ser Ala Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
        50                  55                  60
Gln
65
```

<210> 65
<211> 65
<212> PRT
<213> Artificial Sequence

<220>
<223> anti-HER2 Fynomer

<400> 65

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Asn Pro
            20                  25                  30
Gln Ser Arg Thr Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
        50                  55                  60
Gln
65
```

<210> 66
<211> 65
<212> PRT
<213> Artificial Sequence

<220>
<223> anti-HER2 Fynomer

<400> 66

```
        Gly Val Thr Leu Phe Val Ala Leu Tyr Asp Tyr Thr Ser Tyr Asn Ser
        1               5                   10                  15

        His Asp Leu Ser Phe His Lys Gly Glu Lys Phe Gln Ile Leu Pro Gly
                    20                  25                  30
        Gln Ser Met Thr Gly Asp Trp Trp Glu Ala Arg Ser Leu Thr Thr Gly
                35                  40                  45
        Glu Thr Gly Tyr Ile Pro Ser Asn Tyr Val Ala Pro Val Asp Ser Ile
                50                  55                  60
        Gln
        65
```

<210> 67
<211> 65
<212> PRT
<213> Artificial Sequence

<220>
<223> anti-HER2 Fynomer

<400> 67

```
        Gly Val Thr Leu Phe Val Ala Leu Tyr Asp Tyr Thr Ser Tyr Asn Ala
        1               5                   10                  15
        His Asp Leu Ser Phe His Lys Gly Glu Lys Phe Gln Ile Leu Pro Arg
                    20                  25                  30
        Gln Asp Thr Ala Gly Asp Trp Trp Glu Ala Arg Ser Leu Thr Thr Gly
                35                  40                  45
        Glu Thr Gly Tyr Ile Pro Ser Asn Tyr Val Ala Pro Val Asp Ser Ile
                50                  55                  60
        Gln
        65
```

<210> 68
<211> 63
<212> PRT
<213> Artificial Sequence

<220>
<223> anti-HER2 Fynomer

<400> 68

```
        Gly Val Thr Leu Phe Val Ala Leu Tyr Asp Tyr Thr Ser Tyr Asn Thr
        1               5                   10                  15
        Arg Asp Leu Ser Phe His Lys Gly Glu Lys Phe Gln Ile Leu Gln Ala
                    20                  25                  30
        Leu Gly Gly Asp Trp Trp Glu Ala Arg Ser Leu Thr Thr Gly Glu Thr
                35                  40                  45
        Gly Tyr Ile Pro Ser Asn Tyr Val Ala Pro Val Asp Ser Ile Gln
            50                  55                  60
```

<210> 69

<211> 65
<212> PRT
<213> Artificial Sequence

<220>
<223> anti-HER2 Fynomer

<400> 69

```
Gly Val Thr Leu Phe Val Ala Leu Tyr Asp Tyr Thr Ser Tyr Asn Glu
1               5                   10                  15
Phe Asp Leu Ser Phe His Lys Gly Glu Lys Phe Gln Ile Leu Gln Gly
            20                  25                  30
Thr Gln Leu Ala Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
                50                  55                  60
Gln
65
```

<210> 70
<211> 63
<212> PRT
<213> Artificial Sequence

<220>
<223> anti-HER2 Fynomer

<400> 70

```
Gly Val Thr Leu Phe Val Ala Leu Tyr Asp Tyr Thr Ser Tyr Asn Asp
1               5                   10                  15
Arg Asp Leu Ser Phe His Lys Gly Glu Lys Phe Gln Ile Leu Gln His
            20                  25                  30
Lys Glu Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 71
<211> 63
<212> PRT
<213> Artificial Sequence

<220>
<223> anti-HER2 Fynomer

<400> 71

```
Gly Val Thr Leu Phe Val Ala Leu Tyr Asp Tyr Tyr Ser Tyr Asn Asn
1               5                   10                  15
Thr Asp Leu Ser Phe His Lys Gly Glu Lys Phe Gln Ile Leu Gln Lys
            20                  25                  30
Arg Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 72
<211> 63
<212> PRT
<213> Artificial Sequence

<220>
<223> anti-HER2 Fynomer

<400> 72

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Gln Lys
            20                  25                  30
Ser Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 73
<211> 65
<212> PRT
<213> Artificial Sequence

<220>
<223> anti-HER2 Fynomer

<400> 73

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Gln Pro
            20                  25                  30
Asn Ser Ala Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 74
<211> 63
<212> PRT
<213> Artificial Sequence

<220>
<223> anti-HER2 Fynomer

<400> 74

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Gln Pro
            20                  25                  30
Gln Val Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 75
<211> 63
<212> PRT
<213> Artificial Sequence

<220>
<223> anti-HER2 Fynomer

<400> 75

```
Gly Val Thr Leu Phe Val Ala Leu Tyr Asp Tyr Tyr Ser Tyr Asn Lys
1               5                   10                  15
Thr Asp Leu Ser Phe His Lys Gly Glu Lys Phe Gln Ile Leu Gln Gln
            20                  25                  30
His Glu Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 76
<211> 64
<212> PRT
<213> Artificial Sequence

<220>
<223> anti-HER2 Fynomer

<400> 76

```
Gly Val Thr Leu Phe Val Ala Leu Tyr Asp Tyr Thr Ser Tyr Asn Leu
1               5                   10                  15
Arg Asp Leu Ser Phe His Lys Gly Glu Lys Phe Gln Ile Leu Gln Gln
            20                  25                  30
Asn Leu Asp Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
    50                  55                  60
```

<210> 77
<211> 63
<212> PRT
<213> Artificial Sequence

<220>
<223> anti-HER2 Fynomer

<400> 77

```
Gly Val Thr Leu Phe Val Ala Leu Tyr Asp Tyr Thr Ser Tyr Asn Leu
1               5                   10                  15
Arg Asp Leu Ser Phe His Lys Gly Glu Lys Phe Gln Ile Leu Gln Ser
            20                  25                  30
His Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 78
<211> 64
<212> PRT
<213> Artificial Sequence

<220>
<223> anti-HER2 Fynomer

<400> 78

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Gln Ser
            20                  25                  30
Arg Ala Ser Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 79
<211> 64
<212> PRT
<213> Artificial Sequence

<220>
<223> anti-HER2 Fynomer

<400> 79

```
Gly Val Thr Leu Phe Val Ala Leu Tyr Asp Tyr Thr Ser Tyr Asn Ala
1               5                   10                  15
Arg Asp Leu Ser Phe His Lys Gly Glu Lys Phe Gln Ile Leu Gln Thr
            20                  25                  30
Ser Leu Thr Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 80
<211> 64
<212> PRT
<213> Artificial Sequence

<220>
<223> anti-HER2 Fynomer

<400> 80

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Gln Thr
            20                  25                  30
Thr Ala Met Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 81

<211> 65
<212> PRT
<213> Artificial Sequence

<220>
<223> anti-HER2 Fynomer

<400> 81

```
Gly Val Thr Leu Phe Val Ala Leu Tyr Asp Tyr Thr Ser Tyr Asn Arg
1               5                   10                  15
Arg Asp Leu Ser Phe His Lys Gly Glu Lys Phe Gln Ile Leu Gln Val
            20                  25                  30
Asn Pro Met Asp Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 82
<211> 64
<212> PRT
<213> Artificial Sequence

<220>
<223> anti-HER2 Fynomer

<400> 82

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Gln
            20                  25                  30
Gln Arg Gly Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 83
<211> 65
<212> PRT
<213> Artificial Sequence

<220>
<223> anti-HER2 Fynomer

<400> 83

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Val
            20                  25                  30
Pro Gln Asp Asp Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 84
<211> 64
<212> PRT
<213> Artificial Sequence

<220>
<223> anti-HER2 Fynomer

<400> 84

```
Gly Val Thr Leu Phe Val Ala Leu Tyr Asp Tyr Thr Ser Tyr Asn Pro
1               5                   10                  15
Asn Asp Leu Ser Phe His Lys Gly Glu Lys Phe Gln Ile Leu Ser Gln
            20                  25                  30
Gln Asp Gly Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 85
<211> 65
<212> PRT
<213> Artificial Sequence

<220>
<223> anti-HER2 Fynomer

<400> 85

```
Gly Val Thr Leu Phe Val Ala Leu Tyr Asp Tyr Thr Ser Tyr Asn Arg
1               5                   10                  15
Arg Asp Leu Ser Phe His Lys Gly Glu Lys Phe Gln Ile Leu Ser Ser
            20                  25                  30
Asn Arg Ala Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 86
<211> 65
<212> PRT
<213> Artificial Sequence

<220>
<223> anti-HER2 Fynomer

<400> 86

```
Gly Val Thr Leu Phe Val Ala Leu Tyr Asp Tyr Thr Ser Tyr Asn Asn
1               5                   10                  15
Arg Asp Leu Ser Phe His Lys Gly Glu Lys Phe Gln Ile Leu Thr Pro
            20                  25                  30
Asp Ser Arg Gln Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
    50                  55                  60
Gln
65
```

<210> 87
<211> 64
<212> PRT
<213> Artificial Sequence

<220>
<223> anti-HER2 Fynomer

<400> 87

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Thr Pro
            20                  25                  30
Pro Gln His Gly Asp Trp Trp Glu Ala Arg Ser Leu Thr Thr Gly Glu
        35                  40                  45
Thr Gly Tyr Ile Pro Ser Asn Tyr Val Ala Pro Val Asp Ser Ile Gln
    50                  55                  60
```

<210> 88
<211> 65
<212> PRT
<213> Artificial Sequence

<220>
<223> anti-HER2 Fynomer

<400> 88

```
Gly Val Thr Leu Phe Val Ala Leu Tyr Asp Tyr Thr Ser Tyr Asn Asn
1               5                   10                  15
Thr Asp Leu Ser Phe His Lys Gly Glu Lys Phe Gln Ile Leu Thr Gln
            20                  25                  30
Asp Pro Leu His Gly Asp Trp Trp Glu Ala Arg Ser Leu Thr Thr Gly
        35                  40                  45
Glu Thr Gly Tyr Ile Pro Ser Asn Tyr Val Ala Pro Val Asp Ser Ile
    50                  55                  60
Gln
65
```

<210> 89
<211> 63
<212> PRT
<213> Artificial Sequence

<220>
<223> anti-HER2 Fynomer

<400> 89

```
        Gly Val Thr Leu Phe Val Ala Leu Tyr Asp Tyr Thr Ser Tyr Asn Asp
        1               5                   10                  15
        Arg Asp Leu Ser Phe His Lys Gly Glu Lys Phe Gln Ile Leu Thr Gln
                    20                  25                  30
        Lys Gln Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                50                  55                  60
```

<210> 90
<211> 65
<212> PRT
<213> Artificial Sequence

<220>
<223> anti-HER2 Fynomer

<400> 90

```
        Gly Val Thr Leu Phe Val Ala Leu Tyr Asp Tyr Ser Ser Tyr Asn Thr
        1               5                   10                  15
        Thr Asp Leu Ser Phe His Lys Gly Glu Lys Phe Gln Ile Leu Thr Gln
                    20                  25                  30
        Pro Pro Leu Ser Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                50                  55                  60
        Gln
        65
```

<210> 91
<211> 65
<212> PRT
<213> Artificial Sequence

<220>
<223> anti-HER2 Fynomer

<400> 91

```
        Gly Val Thr Leu Phe Val Ala Leu Tyr Asp Tyr Thr Ser Tyr Asn Thr
        1               5                   10                  15
        Gln Asp Leu Ser Phe His Lys Gly Glu Lys Phe Gln Ile Leu Asp Ser
                    20                  25                  30
        Glu Thr Gly Lys Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                50                  55                  60
        Gln
        65
```

<210> 92
<211> 65

<212> PRT
<213> Artificial Sequence

<220>
<223> anti-HER2 Fynomer

<400> 92

```
Gly Val Thr Leu Phe Val Ala Leu Tyr Asp Tyr Thr Ser Tyr Asn Gln
1               5                   10                  15

Arg Asp Leu Ser Phe His Lys Gly Glu Lys Phe Gln Ile Leu Arg Lys
            20                  25                  30
Pro Lys Tyr Asn Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 93
<211> 63
<212> PRT
<213> Artificial Sequence

<220>
<223> anti-HER2 Fynomer

<400> 93

```
Gly Val Thr Leu Phe Val Ala Leu Tyr Asp Tyr Thr Ser Tyr Asn Gln
1               5                   10                  15
Arg Asp Leu Ser Phe His Lys Gly Glu Lys Phe Gln Ile Leu Ser Glu
            20                  25                  30
Pro Leu Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 94
<211> 64
<212> PRT
<213> Artificial Sequence

<220>
<223> anti-HER2 Fynomer

<400> 94

```
Gly Val Thr Leu Phe Val Ala Leu Tyr Asp Tyr Thr Ser Tyr Asn Gln
1               5                   10                  15
Arg Asp Leu Ser Phe His Lys Gly Glu Lys Phe Gln Ile Leu Val His
            20                  25                  30
Asp Pro Ala Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 95
<211> 62
<212> PRT
<213> Artificial Sequence

<220>
<223> anti-HER2 Fynomer

<400> 95

```
Gly Val Thr Leu Phe Val Ala Leu Tyr Asp Tyr Val Ser Trp Asn Thr
1               5                   10                  15
Leu Asp Leu Ser Phe His Lys Gly Glu Lys Phe Gln Ile Leu Arg Met
            20                  25                  30
Pro Gly Asp Trp Trp Glu Ala Arg Ser Leu Thr Thr Gly Glu Thr Gly
            35                  40                  45
Tyr Ile Pro Ser Asn Tyr Val Ala Pro Val Asp Ser Ile Gln
            50                  55                  60
```

<210> 96
<211> 64
<212> PRT
<213> Artificial Sequence

<220>
<223> anti-HER2 Fynomer

<400> 96

```
Gly Val Thr Leu Phe Val Ala Leu Tyr Asp Tyr Glu Ala Arg Leu Tyr
1               5                   10                  15
Ser Leu Asp Leu Ser Phe His Lys Gly Glu Lys Phe Gln Ile Leu Asn
            20                  25                  30
Arg Arg Trp Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
            50                  55                  60
```

<210> 97
<211> 64
<212> PRT
<213> Artificial Sequence

<220>
<223> anti-HER2 Fynomer

<400> 97

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Ala Pro
            20                  25                  30
Pro Asn Gly Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
            50                  55                  60
```

<210> 98
<211> 64
<212> PRT
<213> Artificial Sequence

<220>
<223> anti-HER2 Fynomer

<400> 98

```
Gly Val Thr Leu Phe Val Ala Leu Tyr Asp Tyr Thr Ser Tyr Asn Ser
1                   5                   10                  15
Arg Asp Leu Ser Phe His Lys Gly Glu Lys Phe Gln Ile Leu Ala Arg
            20                  25                  30
Met Pro Pro Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
    50                  55                  60
```

<210> 99
<211> 66
<212> PRT
<213> Artificial Sequence

<220>
<223> anti-HER2 Fynomer

<400> 99

```
Gly Val Thr Leu Phe Val Ala Leu Tyr Asp Tyr Glu Ala Arg Phe Arg
1                   5                   10                  15
Arg Asn Tyr Ser Asp Leu Ser Phe His Lys Gly Glu Lys Phe Gln Ile
            20                  25                  30
Leu Ser Ala Gln Gln Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
    50                  55                  60
Ile Gln
65
```

<210> 100
<211> 65
<212> PRT
<213> Artificial Sequence

<220>
<223> anti-HER2 Fynomer

<400> 100

```
Gly Val Thr Leu Phe Val Ala Leu Tyr Asp Tyr Asp Arg Arg Tyr Gly
1               5                   10                  15
Ala Asp Leu Ser Phe His Lys Gly Glu Lys Phe Gln Ile Leu Arg Asp
            20                  25                  30
Glu Ala Val Ser Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 101
<211> 65
<212> PRT
<213> Artificial Sequence

<220>
<223> anti-HER2 Fynomer

<400> 101

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu His Asp
            20                  25                  30
Pro Pro Ser Asn Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 102
<211> 64
<212> PRT
<213> Artificial Sequence

<220>
<223> anti-HER2 Fynomer

<400> 102

```
Gly Val Thr Leu Phe Val Ala Leu Tyr Asp Tyr Glu Ala Arg Tyr Ala
1               5                   10                  15
Pro Ala Asp Leu Ser Phe His Lys Gly Glu Lys Phe Gln Ile Leu Asn
            20                  25                  30
His Asp Arg Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
            50                  55                  60
```

<210> 103
<211> 65
<212> PRT
<213> Artificial Sequence

<220>
<223> anti-HER2 Fynomer

<400> 103

```
Gly Val Thr Leu Phe Val Ala Leu Tyr Asp Tyr Thr Ser Ser Tyr Pro
1                   5                   10                  15
Gly Asp Leu Ser Phe His Lys Gly Glu Lys Phe Gln Ile Leu Ser Asn
            20                  25                  30
Asp Pro Val His Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 104
<211> 65
<212> PRT
<213> Artificial Sequence

<220>
<223> anti-HER2 Fynomer

<400> 104

```
Gly Val Thr Leu Phe Val Ala Leu Tyr Asp Tyr Gln Thr Trp Thr Pro
1                   5                   10                  15
Gly Asp Leu Ser Phe His Lys Gly Glu Lys Phe Gln Ile Leu Thr Gln
            20                  25                  30
Asp Glu Gln Gln Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 105
<211> 65
<212> PRT
<213> Artificial Sequence

<220>
<223> anti-HER2 Fynomer

<400> 105

```
Gly Val Thr Leu Phe Val Ala Leu Tyr Asp Tyr Phe Thr Asn Thr Pro
1                   5                   10                  15
Gly Asp Leu Ser Phe His Lys Gly Glu Lys Phe Gln Ile Leu Asn Thr
            20                  25                  30
Ser Tyr Leu Thr Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 106
<211> 63
<212> PRT

<213> Artificial Sequence

<220>
<223> anti-HER2 Fynomer

<400> 106

```
Gly Val Thr Leu Phe Val Ala Leu Tyr Asp Tyr Lys Thr His Asn Pro
1               5                   10                  15
Gly Asp Leu Ser Phe His Lys Gly Glu Lys Phe Gln Ile Leu Gln Gly
            20                  25                  30
Arg Val Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 107
<211> 65
<212> PRT
<213> Artificial Sequence

<220>
<223> anti-HER2 Fynomer

<400> 107

```
Gly Val Thr Leu Phe Val Ala Leu Tyr Asp Tyr Gln Thr Tyr Thr Pro
1               5                   10                  15
Gly Asp Leu Ser Phe His Lys Gly Glu Lys Phe Gln Ile Leu Ser Lys
            20                  25                  30
Pro Pro Gln Thr Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 108
<211> 65
<212> PRT
<213> Artificial Sequence

<220>
<223> anti-HER2 Fynomer

<400> 108

```
Gly Val Thr Leu Phe Val Ala Leu Tyr Asp Tyr Glu Ala Arg Tyr Gln
1               5                   10                  15
Asp Leu Glu Asp Leu Ser Phe His Lys Gly Glu Lys Phe Gln Ile Leu
```

```
                              20                      25                      30
            Asn Gly Arg Arg Gly Asp Trp Trp Glu Ala Arg Ser Leu Thr Thr Gly
                    35                      40                      45
            Glu Thr Gly Tyr Ile Pro Ser Asn Tyr Val Ala Pro Val Asp Ser Ile
                    50                      55                      60
            Gln
            65
```

<210> 109
<211> 63
<212> PRT
<213> Artificial Sequence

<220>
<223> anti-HER2 Fynomer

<400> 109

```
            Gly Val Thr Leu Phe Val Ala Leu Tyr Asp Tyr Asp Arg His Tyr Thr
            1                   5                   10                      15
            Phe Asp Leu Ser Phe His Lys Gly Glu Lys Phe Gln Ile Leu Gln Asn
                    20                      25                      30
            Lys Gln Gly Asp Trp Trp Glu Ala Arg Ser Leu Thr Thr Gly Glu Thr
                    35                      40                      45
            Gly Tyr Ile Pro Ser Asn Tyr Val Ala Pro Val Asp Ser Ile Gln
                    50                      55                      60
```

<210> 110
<211> 63
<212> PRT
<213> Artificial Sequence

<220>
<223> anti-HER2 Fynomer

<400> 110

```
            Gly Val Thr Leu Phe Val Ala Leu Tyr Asp Tyr Thr Ser Tyr Asn Thr
            1                   5                   10                      15
            Arg Asp Leu Ser Phe His Lys Gly Glu Lys Phe Gln Ile Leu Ser Asp
                    20                      25                      30
            Ser Asp Gly Val Trp Trp Glu Ala Arg Ser Leu Thr Thr Gly Glu Thr
                    35                      40                      45
            Gly Tyr Ile Pro Ser Asn Tyr Val Ala Pro Val Asp Ser Ile Gln
                    50                      55                      60
```

<210> 111
<211> 65
<212> PRT
<213> Artificial Sequence

<220>
<223> anti-HER2 Fynomer

<400> 111

78

```
Gly Val Thr Leu Phe Val Ala Leu Tyr Asp Tyr Glu Ala Arg Asp Arg
1               5               10              15
Pro Thr Asp Leu Ser Phe His Lys Gly Glu Lys Phe Gln Ile Leu Gly
            20              25              30
Asp Glu Gln Ile Gly Asp Trp Trp Glu Ala Arg Ser Leu Thr Thr Gly
            35              40              45
Glu Thr Gly Tyr Ile Pro Ser Asn Tyr Val Ala Pro Val Asp Ser Ile
        50              55              60
Gln
65
```

<210> 112
<211> 64
<212> PRT
<213> Artificial Sequence

<220>
<223> anti-HER2 Fynomer

<400> 112

```
Gly Val Thr Leu Phe Val Ala Leu Tyr Asp Tyr Thr Thr Tyr Arg Lys
1               5               10              15
Gly Asp Leu Ser Phe His Lys Gly Glu Lys Phe Gln Ile Leu Asn Arg
            20              25              30
Val Ser Ile Gly Asp Trp Trp Glu Ala Arg Ser Leu Thr Thr Gly Glu
            35              40              45
Thr Gly Tyr Ile Pro Ser Asn Tyr Val Ala Pro Val Asp Ser Ile Gln
        50              55              60
```

<210> 113
<211> 64
<212> PRT
<213> Artificial Sequence

<220>
<223> anti-HER2 Fynomer

<400> 113

```
Gly Val Thr Leu Phe Val Ala Leu Tyr Asp Tyr Thr Ser Tyr Asn Thr
1               5               10              15
Arg Asp Leu Ser Phe His Lys Gly Glu Lys Phe Gln Ile Leu Asn Arg
            20              25              30
Val Ser Ile Gly Asp Trp Trp Glu Ala Arg Ser Leu Thr Thr Gly Glu
            35              40              45
Thr Gly Tyr Ile Pro Ser Asn Tyr Val Ala Pro Val Asp Ser Ile Gln
        50              55              60
```

<210> 114
<211> 63
<212> PRT
<213> Artificial Sequence

<220>
<223> anti-HER2 Fynomer

<400> 114

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Gly Asp
            20                  25                  30
Asn Ile Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 115
<211> 63
<212> PRT
<213> Artificial Sequence

<220>
<223> anti-HER2 Fynomer

<400> 115

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Asn Ser
            20                  25                  30
Ser Asp Gly Thr Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 116
<211> 64
<212> PRT
<213> Artificial Sequence

<220>
<223> anti-HER2 Fynomer

<400> 116

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Trp Ser
            20                  25                  30
Asp Ala Leu Gly Asp Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
        50                  55                  60
```

<210> 117
<211> 63
<212> PRT
<213> Artificial Sequence

<220>
<223> anti-HER2 Fynomer

<400> 117

```
Gly Val Thr Leu Phe Val Ala Leu Tyr Asp Tyr Thr Ser Tyr Asn Thr
1               5                   10              15
Arg Asp Leu Ser Phe His Lys Gly Glu Lys Phe Gln Ile Leu Met Ala
            20                  25              30
Trp Ala Gly Asp Trp Trp Glu Ala Arg Ser Leu Thr Thr Gly Glu Thr
            35                  40              45
Gly Tyr Ile Pro Ser Asn Tyr Val Ala Pro Val Asp Ser Ile Gln
        50              55              60
```

<210> 118
<211> 65
<212> PRT
<213> Artificial Sequence

<220>
<223> anti-HER2 Fynomer

<400> 118

```
Gly Val Thr Leu Phe Val Ala Leu Tyr Asp Tyr Glu Ala Arg Glu Gly
1               5                   10              15

Gly Asn Asp Leu Ser Phe His Lys Gly Glu Lys Phe Gln Ile Leu Asn
            20                  25              30
Arg Val Ser Ile Gly Asp Trp Trp Glu Ala Arg Ser Leu Thr Thr Gly
            35                  40              45
Glu Thr Gly Tyr Ile Pro Ser Asn Tyr Val Ala Pro Val Asp Ser Ile
        50              55              60
Gln
65
```

<210> 119
<211> 66
<212> PRT
<213> Artificial Sequence

<220>
<223> anti-HER2 Fynomer

<400> 119

```
Gly Val Thr Leu Phe Val Ala Leu Tyr Asp Tyr Glu Ala His Asp Gln
1               5                   10              15
His Arg Pro Asp Leu Ser Phe His Lys Gly Glu Lys Phe Gln Ile Leu
            20                  25              30
Asn Arg Val Ser Ile Gly Asp Trp Trp Glu Ala Arg Ser Leu Thr Thr
            35                  40              45
Gly Glu Thr Gly Tyr Ile Pro Ser Asn Tyr Val Ala Pro Val Asp Ser
        50              55              60
Ile Gln
65
```

<210> 120
<211> 66
<212> PRT
<213> Artificial Sequence

<220>

<223> anti-HER2 Fynomer

<400> 120

```
        Gly Val Thr Leu Phe Val Ala Leu Tyr Asp Tyr Glu Ala Arg Leu Ser
        1               5                   10                  15
        Ser His Pro His Val Leu Ser Phe His Lys Gly Glu Lys Phe Gln Ile
                    20                  25                  30
        Leu Asn Ser Ser Ala Gly Asp Trp Trp Glu Ala Arg Ser Leu Thr Thr
                    35                  40                  45
        Gly Glu Thr Gly Tyr Ile Pro Ser Asn Tyr Val Ala Pro Val Asp Ser
            50                  55                  60
        Ile Gln
        65
```

<210> 121
<211> 64
<212> PRT
<213> Artificial Sequence

<220>
<223> anti-HER2 Fynomer

<400> 121

```
        Gly Val Thr Leu Phe Val Ala Leu Tyr Asp Tyr Thr Ser Tyr Asn Ser
        1               5                   10                  15
        Arg Asp Leu Ser Phe His Lys Gly Glu Lys Phe Gln Ile Leu Leu Met
                    20                  25                  30
        His Pro Gly Gly Asp Trp Trp Glu Ala Arg Ser Leu Thr Thr Gly Glu


                    35                  40                  45
        Thr Gly Tyr Ile Pro Ser Asn Tyr Val Ala Pro Val Asp Ser Ile Gln
            50                  55                  60
```

<210> 122
<211> 64
<212> PRT
<213> Artificial Sequence

<220>
<223> anti-HER2 Fynomer

<400> 122

```
        Gly Val Thr Leu Phe Val Ala Leu Tyr Asp Tyr Glu Ala Arg His Ala
        1               5                   10                  15
        Pro Val Asp Leu Ser Phe His Lys Gly Glu Lys Phe Gln Ile Leu Gly
                    20                  25                  30
        Asp Asn Ile Gly Asp Trp Trp Glu Ala Arg Ser Leu Thr Thr Gly Glu
                    35                  40                  45
        Thr Gly Tyr Ile Pro Ser Asn Tyr Val Ala Pro Val Asp Ser Ile Gln
            50                  55                  60
```

<210> 123
<211> 64
<212> PRT
<213> Artificial Sequence

<220>
<223> anti-HER2 Fynomer

<400> 123

```
        Gly Val Thr Leu Phe Val Ala Leu Tyr Asp Tyr Glu Ala Arg Thr Lys
        1               5                   10                  15
        His Phe Asp Leu Ser Phe His Lys Gly Glu Lys Phe Gln Ile Leu Ser
                    20                  25                  30
        Gln Pro His Gly Asp Trp Trp Glu Ala Arg Ser Leu Thr Thr Gly Glu
                    35                  40                  45
        Thr Gly Tyr Ile Pro Ser Asn Tyr Val Ala Pro Val Asp Ser Ile Gln
                    50                  55                  60
```

<210> 124
<211> 64
<212> PRT
<213> Artificial Sequence

<220>
<223> anti-HER2 Fynomer

<400> 124

```
        Gly Val Thr Leu Phe Val Ala Leu Tyr Asp Tyr Glu Ala Arg His Glu
        1               5                   10                  15
        Asn Phe Asp Leu Ser Phe His Lys Gly Glu Lys Phe Gln Ile Leu Asn
                    20                  25                  30
        Arg Gly Ala Gly Asp Trp Trp Glu Ala Arg Ser Leu Thr Thr Gly Glu
                    35                  40                  45
        Thr Gly Tyr Ile Pro Ser Asn Tyr Val Ala Pro Val Asp Ser Ile Gln
                    50                  55                  60
```

<210> 125
<211> 65
<212> PRT
<213> Artificial Sequence

<220>
<223> anti-HER2 Fynomer

<400> 125

```
        Gly Val Thr Leu Phe Val Ala Leu Tyr Asp Tyr Glu Ala Arg Pro Asp
        1               5                   10                  15
        Ser His Asp Leu Ser Phe His Lys Gly Glu Lys Phe Gln Ile Leu Asn
                    20                  25                  30
        Arg Val Ser Ile Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                    50                  55                  60
        Gln
        65
```

<210> 126
<211> 63
<212> PRT
<213> Artificial Sequence

<220>
<223> anti-HER2 Fynomer

<400> 126

```
Gly Val Thr Leu Phe Val Ala Leu Tyr Asp Tyr Glu Ala Arg Arg Thr
1               5                   10                  15
His Asp Leu Ser Phe His Lys Gly Glu Lys Phe Gln Ile Leu Ser Gln
            20                  25                  30
Pro His Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 127
<211> 65
<212> PRT
<213> Artificial Sequence

<220>
<223> anti-HER2 Fynomer

<400> 127

```
Gly Val Thr Leu Phe Val Ala Leu Tyr Asp Tyr Glu Ala Arg Pro Met
1               5                   10                  15
Ser Ser Asp Leu Ser Phe His Lys Gly Glu Lys Phe Gln Ile Leu Asn
            20                  25                  30
Arg Val Ser Ile Gly Asp Trp Trp Glu Ala Arg Ser Leu Thr Thr Gly
            35                  40                  45
Glu Thr Gly Tyr Ile Pro Ser Asn Tyr Val Ala Pro Val Asp Ser Ile
        50                  55                  60
Gln
65
```

<210> 128
<211> 63
<212> PRT
<213> Artificial Sequence

<220>
<223> anti-HER2 Fynomer

<400> 128

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Phe
            20                  25                  30
Asn Pro Gly Asp Trp Trp Glu Ala Arg Ser Leu Thr Thr Gly Glu Thr
            35                  40                  45
Gly Tyr Ile Pro Ser Ser Tyr Val Ala Pro Val Asp Ser Ile Gln
        50                  55                  60
```

<210> 129
<211> 63
<212> PRT
<213> Artificial Sequence

<220>
<223> anti-HER2 Fynomer

<400> 129

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Phe Pro
            20                  25                  30
Asp Asp Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 130
<211> 63
<212> PRT
<213> Artificial Sequence

<220>
<223> anti-HER2 Fynomer

<400> 130

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Gln
            20                  25                  30
Pro His Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 131
<211> 63
<212> PRT
<213> Artificial Sequence

<220>
<223> anti-HER2 Fynomer

<400> 131

```
Gly Val Thr Leu Phe Val Ala Leu Tyr Asp Tyr Thr Ser Tyr Asn Thr
1               5                   10                  15

Arg Asp Leu Ser Phe His Lys Gly Glu Lys Phe Gln Ile Leu Gly Lys
            20                  25                  30
Gly Ser Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 132
<211> 63
<212> PRT
<213> Artificial Sequence

<220>
<223> anti-HER2 Fynomer

<400> 132

```
Gly Val Thr Leu Phe Val Ala Leu Tyr Asp Tyr Thr Ser Tyr Asn Ser
1               5                   10                  15
Arg Asp Leu Ser Phe His Lys Gly Glu Lys Phe Gln Ile Leu Ser Asp
            20                  25                  30
Gln His Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 133
<211> 63
<212> PRT
<213> Artificial Sequence

<220>
<223> anti-HER2 Fynomer

<400> 133

```
Gly Val Thr Leu Phe Val Ala Leu Tyr Asp Tyr Thr Ser Tyr Asn Asp
1               5                   10                  15
Arg Asp Leu Ser Phe His Lys Gly Glu Lys Phe Gln Ile Leu Ser Ala
            20                  25                  30
Pro Gln Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 134
<211> 63
<212> PRT
<213> Artificial Sequence

<220>
<223> anti-HER2 Fynomer

<400> 134

```
Gly Val Thr Leu Phe Val Ala Leu Tyr Asp Tyr Thr Ser Tyr Asn Asp
1               5                   10                  15
Arg Asp Leu Ser Phe His Lys Gly Glu Lys Phe Gln Ile Leu Glu Tyr
            20                  25                  30
Thr Thr Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 135
<211> 65
<212> PRT
<213> Artificial Sequence

<220>

<223> anti-HER2 Fynomer

<400> 135

```
        Gly Val Thr Leu Phe Val Ala Leu Tyr Asp Tyr Thr Ser Tyr Asn Thr
        1                   5                   10                  15
        Arg Asp Leu Ser Phe His Lys Gly Glu Lys Phe Gln Ile Leu Thr Thr
                        20                  25                  30
        Glu Ala Thr Asp Gly Asp Trp Trp Glu Ala Arg Ser Leu Thr Thr Gly
                        35                  40                  45
        Glu Thr Gly Tyr Ile Pro Ser Asn Tyr Val Ala Pro Val Asp Ser Ile
                50                  55                  60
        Gln
        65
```

<210> 136
<211> 63
<212> PRT
<213> Artificial Sequence

<220>
<223> anti-HER2 Fynomer

<400> 136

```
        Gly Val Thr Leu Phe Val Ala Leu Tyr Asp Tyr Thr Ser Tyr Asn Ser
        1                   5                   10                  15
        Arg Asp Leu Ser Phe His Lys Gly Glu Lys Phe Gln Ile Leu Gln Asn
                        20                  25                  30
        Ser Gln Gly Asp Trp Trp Glu Ala Arg Ser Leu Thr Thr Gly Glu Thr
                        35                  40                  45
        Gly Tyr Ile Pro Ser Asn Tyr Val Ala Pro Val Asp Ser Ile Gln
                50                  55                  60
```

<210> 137
<211> 63
<212> PRT
<213> Artificial Sequence

<220>
<223> anti-HER2 Fynomer

<400> 137

```
        Gly Val Thr Leu Phe Val Ala Leu Tyr Asp Tyr Thr Ser Tyr Asn Ser
        1                   5                   10                  15
        Arg Asp Leu Ser Phe His Lys Gly Glu Lys Phe Gln Ile Leu Gly Asn
                        20                  25                  30
        Thr Gln Gly Asp Trp Trp Glu Ala Arg Ser Leu Thr Thr Gly Glu Thr
                        35                  40                  45
        Gly Tyr Ile Pro Ser Asn Tyr Val Ala Pro Val Asp Ser Ile Gln
                50                  55                  60
```

<210> 138
<211> 63
<212> PRT
<213> Artificial Sequence

<220>
<223> anti-HER2 Fynomer

<400> 138

```
Gly Val Thr Leu Phe Val Ala Leu Tyr Asp Tyr Thr Ser Tyr Asn Ala
1               5                   10                  15
Gln Asp Leu Ser Phe His Lys Gly Glu Lys Phe Gln Ile Leu Gly Ala
            20                  25                  30
Arg Tyr Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 139
<211> 63
<212> PRT
<213> Artificial Sequence

<220>
<223> anti-HER2 Fynomer

<400> 139

```
Gly Val Thr Leu Phe Val Ala Leu Tyr Asp Tyr Thr Ser Tyr Asn Ala
1               5                   10                  15
Arg Asp Leu Ser Phe His Lys Gly Glu Lys Phe Gln Ile Leu Gly His
            20                  25                  30
His Ser Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 140
<211> 63
<212> PRT
<213> Artificial Sequence

<220>
<223> anti-HER2 Fynomer

<400> 140

```
Gly Val Thr Leu Phe Val Ala Leu Tyr Asp Tyr Thr Ser Tyr Asn Met
1               5                   10                  15
Arg Asp Leu Ser Phe His Lys Gly Glu Lys Phe Gln Ile Leu Asn Asn
            20                  25                  30
Ser Asp Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 141
<211> 65
<212> PRT
<213> Artificial Sequence

<220>

<223> anti-HER2 Fynomer

<400> 141

```
        Gly Val Thr Leu Phe Val Ala Leu Tyr Asp Tyr Thr Ser Tyr Asn Asp
        1               5                   10                  15
        Arg Asp Leu Ser Phe His Lys Gly Glu Lys Phe Gln Ile Leu Ser Lys
                    20                  25                  30
        Asp Ser Ala Leu Gly Asp Trp Trp Glu Ala Arg Ser Leu Thr Thr Gly
                    35                  40                  45
        Glu Thr Gly Tyr Ile Pro Ser Asn Tyr Val Ala Pro Val Asp Ser Ile
                    50                  55                  60
        Gln
        65
```

<210> 142
<211> 63
<212> PRT
<213> Artificial Sequence

<220>
<223> anti-HER2 Fynomer

<400> 142

```
        Gly Val Thr Leu Phe Val Ala Leu Tyr Asp Tyr Thr Ser Tyr Asn Ser
        1               5                   10                  15
        Arg Asp Leu Ser Phe His Lys Gly Glu Lys Phe Gln Ile Leu Asn Arg
                    20                  25                  30
        Gly Ala Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                    50                  55                  60
```

<210> 143
<211> 63
<212> PRT
<213> Artificial Sequence

<220>
<223> anti-HER2 Fynomer

<400> 143

```
        Gly Val Thr Leu Phe Val Ala Leu Tyr Asp Tyr Thr Ser Tyr Asn Thr
        1               5                   10                  15
        His Asp Leu Ser Phe His Lys Gly Glu Lys Phe Gln Ile Leu Lys Met
                    20                  25                  30
        Gln Ser Gly Asp Trp Trp Glu Ala Arg Ser Leu Thr Thr Gly Glu Thr
                    35                  40                  45
        Gly Tyr Ile Pro Ser Asn Tyr Val Ala Pro Val Asp Ser Ile Gln
                    50                  55                  60
```

<210> 144
<211> 63
<212> PRT
<213> Artificial Sequence

<220>
<223> anti-HER2 Fynomer

<400> 144

```
Gly Val Thr Leu Phe Val Ala Leu Tyr Asp Tyr Thr Ser Tyr Asn Arg
1               5                   10                  15
Arg Asp Leu Ser Phe His Lys Gly Glu Lys Phe Gln Ile Leu Glu Thr
            20                  25                  30
Gln Asn Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 145
<211> 63
<212> PRT
<213> Artificial Sequence

<220>
<223> anti-HER2 Fynomer

<400> 145

```
Gly Val Thr Leu Phe Val Ala Leu Tyr Asp Tyr Thr Ser Tyr Asn Ala
1               5                   10                  15
Arg Asp Leu Ser Phe His Lys Gly Glu Lys Phe Gln Ile Leu Gly Ser
            20                  25                  30
Thr Ala Gly Asp Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
        50                  55                  60
```

<210> 146
<211> 67
<212> PRT
<213> Artificial Sequence

<220>
<223> anti-HER2 Fynomer

<400> 146

```
Gly Val Thr Leu Phe Val Ala Leu Tyr Asp Tyr Glu Ala Arg Asp Ser
1               5                   10                  15
Met Gly Gly Gln Asp Leu Ser Phe His Lys Gly Glu Lys Phe Gln Ile
            20                  25                  30
Leu Arg Ser Trp Pro Glu Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
65
```

<210> 147
<211> 67
<212> PRT
<213> Artificial Sequence

<220>
<223> ant-HER2 Fynomer

<400> 147

```
        Gly Val Thr Leu Phe Val Ala Leu Tyr Asp Tyr Glu Ala Arg Asp Ser
        1               5                   10                  15
        Met Gly Gly Gln Asp Leu Ser Phe His Lys Gly Glu Lys Phe Gln Ile
                20                  25                  30
        Leu Lys Thr Trp Glu Gly Gly Asp Trp Trp Glu Ala Arg Ser Leu Thr
                35                  40                  45
        Thr Gly Glu Thr Gly Tyr Ile Pro Ser Asn Tyr Val Ala Pro Val Asp
                50                  55                  60
        Ser Ile Gln
        65
```

<210> 148
<211> 67
<212> PRT
<213> Artificial Sequence

<220>
<223> anti-HER2 Fynomer

<400> 148

```
        Gly Val Thr Leu Phe Val Ala Leu Tyr Asp Tyr Glu Ala Arg Asp Ser
        1               5                   10                  15
        Met Gly Gly Gln Asp Leu Ser Phe His Lys Gly Glu Lys Phe Gln Ile

                20                  25                  30
        Leu Gln Ala Trp Gln Glu Gly Asp Trp Trp Glu Ala Arg Ser Leu Thr
                35                  40                  45
        Thr Gly Glu Thr Gly Tyr Ile Pro Ser Asn Tyr Val Ala Pro Val Asp
                50                  55                  60
        Ser Ile Gln
        65
```

<210> 149
<211> 63
<212> PRT
<213> Artificial Sequence

<220>
<223> anti-HER2 Fynomer

<400> 149

```
        Gly Val Thr Leu Phe Val Ala Leu Tyr Asp Tyr Thr Ser Tyr Asn Thr
        1               5                   10                  15
        Arg Asp Leu Ser Phe His Lys Gly Glu Lys Phe Gln Ile Leu Arg Met
                20                  25                  30
        Glu Asp Gly Val Trp Trp Ala Ala Arg Ser Leu Thr Thr Gly Glu Ile
                35                  40                  45
        Gly Tyr Ile Pro Ser Asn Tyr Val Ala Pro Val Asp Ser Ile Gln
                50                  55                  60
```

<210> 150

<211> 63
<212> PRT
<213> Artificial Sequence

<220>
<223> anti-HER2 Fynomer

<400> 150

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Met
            20                  25                  30
Glu Asp Gly Val Trp Trp Arg Ala Arg Ser Leu Thr Thr Gly Glu Ile
            35                  40                  45
Gly Leu Ile Pro Ser Asn Tyr Val Ala Pro Val Asp Ser Ile Gln
        50                  55                  60
```

<210> 151
<211> 63
<212> PRT
<213> Artificial Sequence

<220>
<223> anti-HER2 Fynomer

<400> 151

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Met
            20                  25                  30
Glu Asp Gly Val Trp Trp Thr Ala Arg Ser Leu Thr Thr Gly Glu Val
            35                  40                  45
Gly Phe Ile Pro Ser Asn Tyr Val Ala Pro Val Asp Ser Ile Gln
        50                  55                  60
```

<210> 152
<211> 63
<212> PRT
<213> Artificial Sequence

<220>
<223> anti-HER2 Fynomer

<400> 152

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Met
            20                  25                  30
Glu Asp Gly Ile Trp Trp Gln Ala Arg Ser Leu Thr Thr Gly Glu Thr
            35                  40                  45
Gly Phe Ile Pro Ser Asn Tyr Val Ala Pro Val Asp Ser Ile Gln
        50                  55                  60
```

<210> 153
<211> 65

<212> PRT
<213> Artificial Sequence

<220>
<223> anti-HER2 Fynomer

<400> 153

```
Gly Val Thr Leu Phe Val Ala Leu Tyr Asp Tyr Gln Ala Tyr Gly Met
1               5                   10                  15
Tyr Asp Leu Ser Phe His Lys Gly Glu Lys Phe Gln Ile Leu Pro Pro
            20                  25                  30
Tyr Pro Thr Gly Gly Tyr Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 154
<211> 64
<212> PRT
<213> Artificial Sequence

<220>
<223> anti-HER2 Fynomer

<400> 154

```
Gly Val Thr Leu Phe Val Ala Leu Tyr Asp Tyr Gln Ala Tyr Gly Met
1               5                   10                  15
Tyr Asp Leu Ser Phe His Lys Gly Glu Lys Phe Gln Ile Leu Gln Val
            20                  25                  30
Leu Asp Asn Gly Tyr Trp Trp Glu Ala Arg Ser Leu Thr Thr Gly Glu
            35                  40                  45
Thr Gly Tyr Ile Pro Ser Asn Tyr Val Ala Pro Val Asp Ser Ile Gln
            50                  55                  60
```

<210> 155
<211> 65
<212> PRT
<213> Artificial Sequence

<220>
<223> anti-HER2 Fynomer

<400> 155

```
Gly Val Thr Leu Phe Val Ala Leu Tyr Asp Tyr Gln Ala Tyr Gly Met
1               5                   10                  15
Tyr Asp Leu Ser Phe His Lys Gly Glu Lys Phe Gln Ile Leu Thr Ala
            20                  25                  30
Leu Pro Asp Arg Gly Tyr Trp Trp Glu Ala Arg Ser Leu Thr Thr Gly
            35                  40                  45
Glu Thr Gly Tyr Ile Pro Ser Asn Tyr Val Ala Pro Val Asp Ser Ile
            50                  55                  60
Gln
65
```

<210> 156
<211> 63
<212> PRT
<213> Artificial Sequence

<220>
<223> anti-HER2 Fynomer

<400> 156

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Arg Asp
            20                  25                  30
Asp Asp Gly Val Trp Trp Glu Ala Arg Ser Leu Thr Thr Gly Glu Thr
            35                  40                  45
Gly Tyr Ile Pro Ser Asn Tyr Val Ala Pro Val Asp Ser Ile Gln
    50                  55                  60
```

<210> 157
<211> 65
<212> PRT
<213> Artificial Sequence

<220>
<223> anti-HER2 Fynomer

<400> 157

```
Gly Val Thr Leu Phe Val Ala Leu Tyr Asp Tyr Thr Ser Tyr Asn Thr
1               5                   10                  15
Arg Asp Leu Ser Phe His Lys Gly Glu Lys Phe Gln Ile Leu Ser Phe
            20                  25                  30
Gln Ser Ala Gly Gly Val Trp Trp Glu Ala Arg Ser Leu Thr Thr Gly
            35                  40                  45
Glu Thr Gly Tyr Ile Pro Ser Asn Tyr Val Ala Pro Val Asp Ser Ile
    50                  55                  60
Gln
65
```

<210> 158
<211> 63
<212> PRT
<213> Artificial Sequence

<220>
<223> anti-HER2 Fynomer

<400> 158

```
Gly Val Thr Leu Phe Val Ala Leu Tyr Asp Tyr Thr Ser Tyr Asn Thr
1               5                   10                  15
```

```
       Arg Asp Leu Ser Phe His Lys Gly Glu Lys Phe Gln Ile Leu Ala Arg
               20                  25                  30
       Asp Asn Gly Val Trp Trp Glu Ala Arg Ser Leu Thr Thr Gly Glu Thr
               35                  40                  45
       Gly Tyr Ile Pro Ser Asn Tyr Val Ala Pro Val Asp Ser Ile Gln
               50                  55                  60
```

<210> 159
<211> 66
<212> PRT
<213> Artificial Sequence

<220>
<223> anti-HER2 Fynomer

<400> 159

```
       Gly Val Thr Leu Phe Val Ala Leu Tyr Asp Tyr Glu Ala Arg Asp Ser
       1               5                   10                  15
       Met Gly Gly Gln Asp Leu Ser Phe His Lys Gly Glu Lys Phe Gln Ile
               20                  25                  30
       Leu Trp Asn Thr Gly Gly Asp Trp Trp Glu Ala Arg Ser Leu Thr Thr
               35                  40                  45
       Gly Glu Thr Gly Tyr Ile Pro Ser Asn Tyr Val Ala Pro Val Asp Ser
               50                  55                  60
       Ile Gln
       65
```

<210> 160
<211> 5
<212> PRT
<213> Artificial Sequence

<220>
<223> GS-linker

<400> 160

```
                        Gly Gly Gly Gly Ser
                        1               5
```

<210> 161
<211> 10
<212> PRT
<213> Artificial Sequence

<220>
<223> GS-linker

<400> 161

```
              Gly Gly Gly Gly Ser Gly Gly Gly Gly Ser
              1               5                   10
```

<210> 162
<211> 15
<212> PRT
<213> Artificial Sequence

<220>
<223> GS-linker

<400> 162

```
Gly Gly Gly Gly Ser Gly Gly Gly Gly Ser Gly Gly Gly Gly Ser
1               5                   10                  15
```

<210> 163
<211> 527
<212> PRT
<213> Artificial Sequence

<220>
<223> COVA420 Heavy Chain

<400> 163

```
Gly Val Thr Leu Phe Val Ala Leu Tyr Asp Tyr Thr Ser Tyr Asn Thr
1               5               10              15
Arg Asp Leu Ser Phe His Lys Gly Glu Lys Phe Gln Ile Leu Arg Met
        20              25              30
Glu Asp Gly Val Trp Trp Glu Ala Arg Ser Leu Thr Thr Gly Glu Thr
        35              40              45
Gly Tyr Ile Pro Ser Asn Tyr Val Ala Pro Val Asp Ser Ile Gln Gly
    50              55              60
Gly Gly Gly Ser Gly Gly Gly Gly Ser Gly Gly Gly Gly Ser Gln Val
65              70              75              80
Gln Leu Val Gln Ser Gly Gly Gly Val Val Gln Pro Gly Arg Ser Leu
            85              90              95
Arg Leu Ser Cys Lys Ala Ser Gly Tyr Thr Phe Thr Arg Tyr Thr Met
            100             105             110
His Trp Val Arg Gln Ala Pro Gly Lys Gly Leu Glu Trp Ile Gly Tyr
        115             120             125
Ile Asn Pro Ser Arg Gly Tyr Thr Asn Tyr Asn Gln Lys Val Lys Asp
    130             135             140
Arg Phe Thr Ile Ser Arg Asp Asn Ser Lys Asn Thr Ala Phe Leu Gln
145             150             155             160
Met Asp Ser Leu Arg Pro Glu Asp Thr Gly Val Tyr Phe Cys Ala Arg
            165             170             175
Tyr Tyr Asp Asp His Tyr Cys Leu Asp Tyr Trp Gly Gln Gly Thr Pro
        180             185             190
Val Thr Val Ser Ser Ala Ser Thr Lys Gly Pro Ser Val Phe Pro Leu
        195             200             205
Ala Pro Ser Ser Lys Ser Thr Ser Gly Gly Thr Ala Ala Leu Gly Cys
    210             215             220
Leu Val Lys Asp Tyr Phe Pro Glu Pro Val Thr Val Ser Trp Asn Ser
225             230             235             240
Gly Ala Leu Thr Ser Gly Val His Thr Phe Pro Ala Val Leu Gln Ser
            245             250             255
Ser Gly Leu Tyr Ser Leu Ser Ser Val Val Thr Val Pro Ser Ser Ser
        260             265             270
Leu Gly Thr Gln Thr Tyr Ile Cys Asn Val Asn His Lys Pro Ser Asn
    275             280             285
Thr Lys Val Asp Lys Lys Val Glu Pro Lys Ser Cys Asp Lys Thr His
    290             295             300
Thr Cys Pro Pro Cys Pro Ala Pro Glu Ala Ala Gly Gly Pro Ser Val
305             310             315             320
Phe Leu Phe Pro Pro Lys Pro Lys Asp Thr Leu Met Ile Ser Arg Thr
            325             330             335
Pro Glu Val Thr Cys Val Val Val Asp Val Ser His Glu Asp Pro Glu
        340             345             350
Val Lys Phe Asn Trp Tyr Val Asp Gly Val Glu Val His Asn Ala Lys
        355             360             365
Thr Lys Pro Arg Glu Glu Gln Tyr Asn Ser Thr Tyr Arg Val Val Ser
    370             375             380
Val Leu Thr Val Leu His Gln Asp Trp Leu Asn Gly Lys Glu Tyr Lys
385             390             395             400
Cys Lys Val Ser Asn Lys Ala Leu Pro Ala Pro Ile Glu Lys Thr Ile
            405             410             415
Ser Lys Ala Lys Gly Gln Pro Arg Glu Pro Gln Val Tyr Thr Leu Pro
        420             425             430
```

97

```
Pro Ser Arg Glu Glu Met Thr Lys Asn Gln Val Ser Leu Thr Cys Leu
        435                 440                 445
Val Lys Gly Phe Tyr Pro Ser Asp Ile Ala Val Glu Trp Glu Ser Asn
        450                 455                 460
Gly Gln Pro Glu Asn Asn Tyr Lys Thr Thr Pro Pro Val Leu Asp Ser
465                 470                 475                 480
Asp Gly Ser Phe Phe Leu Tyr Ser Lys Leu Thr Val Asp Lys Ser Arg
                485                 490                 495
Trp Gln Gln Gly Asn Val Phe Ser Cys Ser Val Met His Glu Ala Leu
                500                 505                 510
His Asn His Tyr Thr Gln Lys Ser Leu Ser Leu Ser Pro Gly Lys
        515                 520                 525
```

<210> 164
<211> 213
<212> PRT
<213> Artificial Sequence

<220>
<223> COVA420 Light Chain

<400> 164

```
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Ser Val Ser Tyr Met
        20                  25                  30
Asn Trp Tyr Gln Gln Thr Pro Gly Lys Ala Pro Lys Arg Trp Ile Tyr
        35                  40                  45
Asp Thr Ser Lys Leu Ala Ser Gly Val Pro Ser Arg Phe Ser Gly Ser
        50                  55                  60
Gly Ser Gly Thr Asp Tyr Thr Phe Thr Ile Ser Ser Leu Gln Pro Glu
65                  70                  75                  80
Asp Ile Ala Thr Tyr Tyr Cys Gln Gln Trp Ser Ser Asn Pro Phe Thr
                85                  90                  95
Phe Gly Gln Gly Thr Lys Leu Gln Ile Thr Arg Thr Val Ala Ala Pro
                100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
        130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
                180                 185                 190
Cys Glu Val Thr His Gln Gly Leu Ser Ser Pro Val Thr Lys Ser Phe
        195                 200                 205
Asn Arg Gly Glu Cys
        210
```

<210> 165
<211> 527
<212> PRT
<213> Artificial Sequence

<220>
<223> COVA422 Heavy Chain

<400> 165

```
Gln Val Gln Leu Val Gln Ser Gly Gly Gly Val Val Gln Pro Gly Arg
1               5                   10                  15
Ser Leu Arg Leu Ser Cys Lys Ala Ser Gly Tyr Thr Phe Thr Arg Tyr
```

```
                    20                      25                      30
Thr Met His Trp Val Arg Gln Ala Pro Gly Lys Gly Leu Glu Trp Ile
        35                      40                      45
Gly Tyr Ile Asn Pro Ser Arg Gly Tyr Thr Asn Tyr Asn Gln Lys Val
        50                      55                      60
Lys Asp Arg Phe Thr Ile Ser Arg Asp Asn Ser Lys Asn Thr Ala Phe
65                      70                      75                      80
Leu Gln Met Asp Ser Leu Arg Pro Glu Asp Thr Gly Val Tyr Phe Cys
                85                      90                      95
Ala Arg Tyr Tyr Asp Asp His Tyr Cys Leu Asp Tyr Trp Gly Gln Gly
            100                     105                     110
Thr Pro Val Thr Val Ser Ser Ala Ser Thr Lys Gly Pro Ser Val Phe
        115                     120                     125
Pro Leu Ala Pro Ser Ser Lys Ser Thr Ser Gly Gly Thr Ala Ala Leu
        130                     135                     140
Gly Cys Leu Val Lys Asp Tyr Phe Pro Glu Pro Val Thr Val Ser Trp
145                     150                     155                     160
Asn Ser Gly Ala Leu Thr Ser Gly Val His Thr Phe Pro Ala Val Leu
            165                     170                     175
Gln Ser Ser Gly Leu Tyr Ser Leu Ser Ser Val Val Thr Val Pro Ser
        180                     185                     190
Ser Ser Leu Gly Thr Gln Thr Tyr Ile Cys Asn Val Asn His Lys Pro
        195                     200                     205
Ser Asn Thr Lys Val Asp Lys Lys Val Glu Pro Lys Ser Cys Asp Lys
210                     215                     220
Thr His Thr Cys Pro Pro Cys Pro Ala Pro Glu Ala Ala Gly Gly Pro
225                     230                     235                     240
Ser Val Phe Leu Phe Pro Pro Lys Pro Lys Asp Thr Leu Met Ile Ser
            245                     250                     255
Arg Thr Pro Glu Val Thr Cys Val Val Asp Val Ser His Glu Asp
        260                     265                     270
Pro Glu Val Lys Phe Asn Trp Tyr Val Asp Gly Val Glu Val His Asn
        275                     280                     285
Ala Lys Thr Lys Pro Arg Glu Glu Gln Tyr Asn Ser Thr Tyr Arg Val
        290                     295                     300
Val Ser Val Leu Thr Val Leu His Gln Asp Trp Leu Asn Gly Lys Glu
305                     310                     315                     320
Tyr Lys Cys Lys Val Ser Asn Lys Ala Leu Pro Ala Pro Ile Glu Lys
            325                     330                     335
Thr Ile Ser Lys Ala Lys Gly Gln Pro Arg Glu Pro Gln Val Tyr Thr
        340                     345                     350
Leu Pro Pro Ser Arg Glu Glu Met Thr Lys Asn Gln Val Ser Leu Thr
        355                     360                     365
Cys Leu Val Lys Gly Phe Tyr Pro Ser Asp Ile Ala Val Glu Trp Glu
    370                     375                     380
Ser Asn Gly Gln Pro Glu Asn Asn Tyr Lys Thr Thr Pro Pro Val Leu
385                     390                     395                     400
Asp Ser Asp Gly Ser Phe Phe Leu Tyr Ser Lys Leu Thr Val Asp Lys
            405                     410                     415
Ser Arg Trp Gln Gln Gly Asn Val Phe Ser Cys Ser Val Met His Glu
            420                     425                     430
Ala Leu His Asn His Tyr Thr Gln Lys Ser Leu Ser Leu Ser Pro Gly
        435                     440                     445
Lys Gly Gly Gly Gly Ser Gly Gly Gly Ser Gly Gly Gly Gly Ser
        450                     455                     460
Gly Val Thr Leu Phe Val Ala Leu Tyr Asp Tyr Thr Ser Tyr Asn Thr
465                     470                     475                     480
Arg Asp Leu Ser Phe His Lys Gly Glu Lys Phe Gln Ile Leu Arg Met
            485                     490                     495
Glu Asp Gly Val Trp Trp Glu Ala Arg Ser Leu Thr Thr Gly Glu Thr
            500                     505                     510
Gly Tyr Ile Pro Ser Asn Tyr Val Ala Pro Val Asp Ser Ile Gln
        515                     520                     525
```

<210> 166
<211> 213
<212> PRT
<213> Artificial Sequence

<220>
<223> COVA422 Light Chain

<400> 166

```
Asp Ile Gln Met Thr Gln Ser Pro Ser Ser Leu Ser Ala Ser Val Gly
1               5                   10                  15
Asp Arg Val Thr Ile Thr Cys Ser Ala Ser Ser Ser Val Ser Tyr Met
            20                  25                  30
Asn Trp Tyr Gln Gln Thr Pro Gly Lys Ala Pro Lys Arg Trp Ile Tyr
        35                  40                  45
Asp Thr Ser Lys Leu Ala Ser Gly Val Pro Ser Arg Phe Ser Gly Ser
    50                  55                  60
Gly Ser Gly Thr Asp Tyr Thr Phe Thr Ile Ser Ser Leu Gln Pro Glu
65                  70                  75                  80
Asp Ile Ala Thr Tyr Tyr Cys Gln Gln Trp Ser Ser Asn Pro Phe Thr
                85                  90                  95
Phe Gly Gln Gly Thr Lys Leu Gln Ile Thr Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
            195                 200                 205
Asn Arg Gly Glu Cys
            210
```

<210> 167
<211> 495
<212> PRT
<213> Artificial Sequence

<220>
<223> COVA446: anti-HER2 x anti CD3 single chain Fv

<400> 167

```
Glu Val Gln Leu Val Glu Ser Gly Gly Gly Leu Val Gln Pro Gly Gly
1               5                   10              15
Ser Leu Arg Leu Ser Cys Ala Ala Ser Gly Phe Asn Ile Lys Asp Thr
            20                  25              30
Tyr Ile His Trp Val Arg Gln Ala Pro Gly Lys Gly Leu Glu Trp Val
            35                  40              45
Ala Arg Ile Tyr Pro Thr Asn Gly Tyr Thr Arg Tyr Ala Asp Ser Val
        50                  55              60
Lys Gly Arg Phe Thr Ile Ser Ala Asp Thr Ser Lys Asn Thr Ala Tyr
65                  70              75              80
Leu Gln Met Asn Ser Leu Arg Ala Glu Asp Thr Ala Val Tyr Tyr Cys
            85                  90              95
Ser Arg Trp Gly Gly Asp Gly Phe Tyr Ala Met Asp Tyr Trp Gly Gln
            100                 105             110
Gly Thr Leu Val Thr Val Ser Ser Gly Gly Gly Gly Ser Gly Gly Gly
```

```
                    115                        120                          125
       Gly Ser Gly Gly Gly Gly Ser Asp Ile Gln Met Thr Gln Ser Pro Ser
           130                        135                    140
       Ser Leu Ser Ala Ser Val Gly Asp Arg Val Thr Ile Thr Cys Arg Ala
           145                    150                    155                    160
       Ser Gln Asp Val Asn Thr Ala Val Ala Trp Tyr Gln Gln Lys Pro Gly
                       165                    170                        175
       Lys Ala Pro Lys Leu Leu Ile Tyr Ser Ala Ser Phe Leu Tyr Ser Gly
                   180                    185                    190
       Val Pro Ser Arg Phe Ser Gly Ser Arg Ser Gly Thr Asp Phe Thr Leu
                   195                        200                    205
       Thr Ile Ser Ser Leu Gln Pro Glu Asp Phe Ala Thr Tyr Tyr Cys Gln
           210                        215                    220
       Gln His Tyr Thr Thr Pro Pro Thr Phe Gly Gln Gly Thr Lys Val Glu
       225                        230                    235                    240
       Ile Lys Gly Gly Gly Gly Ser Glu Val Gln Leu Val Glu Ser Gly Gly
                       245                    250                    255
       Gly Leu Val Gln Pro Gly Gly Ser Leu Lys Leu Ser Cys Ala Ala Ser
                   260                    265                    270
       Gly Phe Thr Phe Asn Lys Tyr Ala Met Trp Val Arg Gln Ala Pro Gly
                   275                    280                    285
       Lys Gly Leu Glu Trp Val Ala Arg Ile Arg Ser Lys Tyr Asn Asn Tyr
           290                    295                    300
       Ala Thr Tyr Tyr Ala Asp Ser Val Lys Asp Arg Phe Thr Ile Ser Arg
       305                        310                    315                    320
       Asp Asp Ser Lys Asn Thr Ala Tyr Leu Gln Met Asn Asn Leu Lys Thr
                       325                    330                    335
       Glu Asp Thr Ala Val Tyr Tyr Cys Val Arg His Gly Asn Phe Gly Asn
                   340                    345                    350
       Ser Tyr Ile Ser Tyr Trp Ala Tyr Trp Gly Gln Gly Thr Leu Val Thr
                   355                    360                    365
       Val Ser Ser Gly Gly Gly Gly Ser Gly Gly Gly Ser Gly Gly Gly
           370                    375                    380
       Gly Ser Gln Thr Val Val Thr Gln Glu Pro Ser Leu Thr Val Ser Pro
       385                        390                    395                    400
       Gly Gly Thr Val Thr Leu Thr Cys Gly Ser Ser Thr Gly Ala Val Thr
                       405                    410                    415
       Ser Gly Asn Tyr Pro Asn Trp Val Gln Gln Lys Pro Gly Gln Ala Pro
                   420                    425                    430
       Arg Gly Leu Ile Gly Gly Thr Lys Phe Leu Ala Pro Gly Thr Pro Ala
                   435                    440                    445
       Arg Phe Ser Gly Ser Leu Leu Gly Gly Lys Ala Ala Leu Thr Leu Ser
           450                    455                    460
       Gly Val Gln Pro Glu Asp Glu Ala Glu Tyr Tyr Cys Val Leu Trp Tyr
       465                        470                    475                    480
       Ser Asn Arg Trp Val Phe Gly Gly Gly Thr Lys Leu Thr Val Leu
                   485                    490                    495
```

<210> 168
<211> 68
<212> PRT
<213> Artificial Sequence

<220>
<223> anti-HER2 Fynomer

<220>
<221> VARIANT
<222> 12,13,14,15,16,17,18,34,35,36,41,44,53,55,59
<223> Gly, Val, Thr, Leu, Phe, Ala, Tyr, Asp, Ser, His, Lys, Glu, Gln, Ile, Trp, Arg, Met, Pro, Asn

<220>
<221> VARIANT
<222> 19,20,21,37,38,39
<223> Gly, Val, Thr, Leu, Phe, Ala, Tyr, Asp, Ser, His, Lys, Glu, Gln, Ile, Trp, Arg, Met, Pro, Asn

<400> 168

```
Gly Val Thr Leu Phe Val Ala Leu Tyr Asp Tyr Xaa Xaa Xaa Xaa Xaa
1               5                   10                  15
Xaa Xaa Xaa Xaa Xaa Leu Ser Phe His Lys Gly Glu Lys Phe Gln Ile
            20                  25                  30
Leu Xaa Xaa Xaa Xaa Xaa Xaa Gly Xaa Trp Trp Xaa Ala Arg Ser Leu
            35                  40                  45
Thr Thr Gly Glu Xaa Gly Xaa Ile Pro Ser Xaa Tyr Val Ala Pro Val
        50                  55                  60
Asp Ser Ile Gln
65
```

<210> 169
<211> 66
<212> PRT
<213> Artificial Sequence

<220>
<223> anti-EGFR Fynomer ER7L2D6

<400> 169

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Ser Phe Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 170
<211> 532
<212> PRT
<213> Artificial Sequence

<220>
<223> COVA493 HC

<400> 170

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Ser Phe Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln Gly Gly Gly Gly Ser Gly Gly Gly Gly Ser Gly Gly Gly Gly
65                  70                  75                  80
Ser Gln Val Gln Leu Gln Gln Ser Gly Ala Glu Leu Ala Arg Pro Gly
            85                  90                  95
Ala Ser Val Lys Met Ser Cys Lys Ala Ser Gly Tyr Thr Phe Thr Arg
            100                 105                 110
Ser Thr Met His Trp Val Lys Gln Arg Pro Gly Gln Gly Leu Glu Trp
            115                 120                 125
Ile Gly Tyr Ile Asn Pro Ser Ser Ala Tyr Thr Asn Tyr Asn Gln Lys
```

**105**

```
                130                      135                          140
      Phe Lys Asp Lys Ala Thr Leu Thr Ala Asp Lys Ser Ser Ser Thr Ala
      145                     150                     155                     160
      Tyr Met Gln Leu Ser Ser Leu Thr Ser Glu Asp Ser Ala Val Tyr Tyr
                           165                     170                     175
      Cys Ala Ser Pro Gln Val His Tyr Asp Tyr Asn Gly Phe Pro Tyr Trp
                       180                     185                     190
      Gly Gln Gly Thr Leu Val Thr Val Ser Ala Ala Ser Thr Lys Gly Pro
                       195                     200                     205
      Ser Val Phe Pro Leu Ala Pro Ser Ser Lys Ser Thr Ser Gly Gly Thr
                   210                     215                     220
      Ala Ala Leu Gly Cys Leu Val Lys Asp Tyr Phe Pro Glu Pro Val Thr
      225                     230                     235                     240
      Val Ser Trp Asn Ser Gly Ala Leu Thr Ser Gly Val His Thr Phe Pro
                           245                     250                     255
      Ala Val Leu Gln Ser Ser Gly Leu Tyr Ser Leu Ser Ser Val Val Thr
                       260                     265                     270
      Val Pro Ser Ser Ser Leu Gly Thr Gln Thr Tyr Ile Cys Asn Val Asn
                   275                     280                     285
      His Lys Pro Ser Asn Thr Lys Val Asp Lys Lys Val Glu Pro Lys Ser
               290                     295                     300
      Cys Asp Lys Thr His Thr Cys Pro Pro Cys Pro Ala Pro Glu Leu Leu
      305                     310                     315                     320
      Gly Gly Pro Ser Val Phe Leu Phe Pro Pro Lys Pro Lys Asp Thr Leu
                           325                     330                     335
      Met Ile Ser Arg Thr Pro Glu Val Thr Cys Val Val Val Ala Val Ser
                       340                     345                     350
      His Glu Asp Pro Glu Val Lys Phe Asn Trp Tyr Val Asp Gly Val Glu
                   355                     360                     365
      Val His Asn Ala Lys Thr Lys Pro Arg Glu Glu Gln Tyr Asn Ser Thr
               370                     375                     380
      Tyr Arg Val Val Ser Val Leu Thr Val Leu His Gln Asp Trp Leu Asn
      385                     390                     395                     400
      Gly Lys Glu Tyr Lys Cys Lys Val Ser Asn Lys Ala Leu Ala Ala Pro
                           405                     410                     415
      Ile Glu Lys Thr Ile Ser Lys Ala Lys Gly Gln Pro Arg Glu Pro Gln
                   420                     425                     430
      Val Tyr Thr Leu Pro Pro Ser Arg Glu Glu Met Thr Lys Asn Gln Val
               435                     440                     445
      Ser Leu Thr Cys Leu Val Lys Gly Phe Tyr Pro Ser Asp Ile Ala Val
      450                     455                     460
      Glu Trp Glu Ser Asn Gly Gln Pro Glu Asn Asn Tyr Lys Thr Thr Pro
      465                     470                     475                     480
      Pro Val Leu Asp Ser Asp Gly Ser Phe Phe Leu Tyr Ser Lys Leu Thr
                       485                     490                     495
      Val Asp Lys Ser Arg Trp Gln Gln Gly Asn Val Phe Ser Cys Ser Val
                   500                     505                     510
      Met His Glu Ala Leu His Asn His Tyr Thr Gln Lys Ser Leu Ser Leu
               515                     520                     525
      Ser Pro Gly Lys
               530
```

<210> 171
<211> 451
<212> PRT
<213> Artificial Sequence

<220>
<223> COVA489 HC, anti-CD3 antibody

<400> 171

```
Gln Val Gln Leu Gln Gln Ser Gly Ala Glu Leu Ala Arg Pro Gly Ala
1               5               10                      15

Ser Val Lys Met Ser Cys Lys Ala Ser Gly Tyr Thr Phe Thr Arg Ser
            20              25                      30
Thr Met His Trp Val Lys Gln Arg Pro Gly Gln Gly Leu Glu Trp Ile
        35              40                      45
Gly Tyr Ile Asn Pro Ser Ser Ala Tyr Thr Asn Tyr Asn Gln Lys Phe
    50              55                      60
Lys Asp Lys Ala Thr Leu Thr Ala Asp Lys Ser Ser Ser Thr Ala Tyr
65              70              75                      80
Met Gln Leu Ser Ser Leu Thr Ser Glu Asp Ser Ala Val Tyr Tyr Cys
            85              90                      95
Ala Ser Pro Gln Val His Tyr Asp Tyr Asn Gly Phe Pro Tyr Trp Gly
            100             105                     110
Gln Gly Thr Leu Val Thr Val Ser Ala Ala Ser Thr Lys Gly Pro Ser
        115             120                     125
Val Phe Pro Leu Ala Pro Ser Ser Lys Ser Thr Ser Gly Gly Thr Ala
    130             135                     140
Ala Leu Gly Cys Leu Val Lys Asp Tyr Phe Pro Glu Pro Val Thr Val
145             150             155                     160
Ser Trp Asn Ser Gly Ala Leu Thr Ser Gly Val His Thr Phe Pro Ala
            165             170                     175
Val Leu Gln Ser Ser Gly Leu Tyr Ser Leu Ser Ser Val Val Thr Val
        180             185                     190
Pro Ser Ser Ser Leu Gly Thr Gln Thr Tyr Ile Cys Asn Val Asn His
    195             200             205
Lys Pro Ser Asn Thr Lys Val Asp Lys Lys Val Glu Pro Lys Ser Cys
210             215             220
Asp Lys Thr His Thr Cys Pro Pro Cys Pro Ala Pro Glu Leu Leu Gly
225             230             235                     240
Gly Pro Ser Val Phe Leu Phe Pro Pro Lys Pro Lys Asp Thr Leu Met
            245             250                     255
Ile Ser Arg Thr Pro Glu Val Thr Cys Val Val Val Ala Val Ser His
        260             265                     270
Glu Asp Pro Glu Val Lys Phe Asn Trp Tyr Val Asp Gly Val Glu Val
    275             280             285
His Asn Ala Lys Thr Lys Pro Arg Glu Glu Gln Tyr Asn Ser Thr Tyr
290             295             300
Arg Val Val Ser Val Leu Thr Val Leu His Gln Asp Trp Leu Asn Gly
305             310             315                     320
Lys Glu Tyr Lys Cys Lys Val Ser Asn Lys Ala Leu Ala Ala Pro Ile
            325             330                     335
Glu Lys Thr Ile Ser Lys Ala Lys Gly Gln Pro Arg Glu Pro Gln Val
        340             345                     350
Tyr Thr Leu Pro Pro Ser Arg Glu Glu Met Thr Lys Asn Gln Val Ser
    355             360                     365
Leu Thr Cys Leu Val Lys Gly Phe Tyr Pro Ser Asp Ile Ala Val Glu
    370             375                     380
Trp Glu Ser Asn Gly Gln Pro Glu Asn Asn Tyr Lys Thr Thr Pro Pro
385             390                     395             400
Val Leu Asp Ser Asp Gly Ser Phe Phe Leu Tyr Ser Lys Leu Thr Val
            405             410                     415
Asp Lys Ser Arg Trp Gln Gln Gly Asn Val Phe Ser Cys Ser Val Met
        420             425                     430
His Glu Ala Leu His Asn His Tyr Thr Gln Lys Ser Leu Ser Leu Ser
    435             440                     445
Pro Gly Lys
450
```

<210> 172

<211> 213
<212> PRT
<213> Artificial Sequence

<220>
<223> COVA489 LC, anti-CD3 antibody

<400> 172

```
Gln Val Val Leu Thr Gln Ser Pro Ala Ile Met Ser Ala Phe Pro Gly
1               5               10              15
Glu Lys Val Thr Met Thr Cys Ser Ala Ser Ser Ser Val Ser Tyr Met
            20              25              30
Asn Trp Tyr Gln Gln Lys Ser Gly Thr Ser Pro Lys Arg Trp Ile Tyr
        35              40              45
Asp Ser Ser Lys Leu Ala Ser Gly Val Pro Ala Arg Phe Ser Gly Ser
    50              55              60
Gly Ser Gly Thr Ser Tyr Ser Leu Thr Ile Ser Ser Met Glu Thr Glu
65              70              75              80
Asp Ala Ala Thr Tyr Tyr Cys Gln Gln Trp Ser Arg Asn Pro Pro Thr
            85              90              95
Phe Gly Gly Gly Thr Lys Leu Gln Ile Thr Arg Thr Val Ala Ala Pro
        100             105             110
Ser Val Phe Ile Phe Pro Pro Ser Asp Glu Gln Leu Lys Ser Gly Thr
        115             120             125
Ala Ser Val Val Cys Leu Leu Asn Asn Phe Tyr Pro Arg Glu Ala Lys
    130             135             140
Val Gln Trp Lys Val Asp Asn Ala Leu Gln Ser Gly Asn Ser Gln Glu
145             150             155             160
Ser Val Thr Glu Gln Asp Ser Lys Asp Ser Thr Tyr Ser Leu Ser Ser
            165             170             175
Thr Leu Thr Leu Ser Lys Ala Asp Tyr Glu Lys His Lys Val Tyr Ala
            180             185             190
Cys Glu Val Thr His Gln Gly Leu Ser Ser Pro Val Thr Lys Ser Phe
            195             200             205
Asn Arg Gly Glu Cys
            210
```

<210> 173
<211> 501
<212> PRT
<213> Artificial Sequence

<220>
<223> COVA445, anti-EGFR/CD3 scFv control

<400> 173

```
Asp Ile Leu Leu Thr Gln Ser Pro Val Ile Leu Ser Val Ser Pro Gly
1               5               10              15
Glu Arg Val Ser Phe Ser Cys Arg Ala Ser Gln Ser Ile Gly Thr Asn
        20              25              30
Ile His Trp Tyr Gln Gln Arg Thr Asn Gly Ser Pro Arg Leu Leu Ile
        35              40              45
Lys Tyr Ala Ser Glu Ser Ile Ser Gly Ile Pro Ser Arg Phe Ser Gly
        50              55              60
Ser Gly Ser Gly Thr Asp Phe Thr Leu Ser Ile Asn Ser Val Glu Ser
65              70              75              80
Glu Asp Ile Ala Asp Tyr Tyr Cys Gln Gln Asn Asn Asn Trp Pro Thr
            85              90              95
Thr Phe Gly Ala Gly Thr Lys Leu Glu Leu Lys Gly Gly Gly Gly Ser
        100             105             110
Gly Gly Gly Gly Ser Gly Gly Gly Gly Ser Gln Val Gln Leu Lys Gln
        115             120             125
Ser Gly Pro Gly Leu Val Gln Pro Ser Gln Ser Leu Ser Ile Thr Cys
    130             135             140
Thr Val Ser Gly Phe Ser Leu Thr Asn Tyr Gly Val His Trp Val Arg
145             150             155             160
Gln Ser Pro Gly Lys Gly Leu Glu Trp Leu Gly Val Ile Trp Ser Gly
            165             170             175
```

```
Gly Asn Thr Asp Tyr Asn Thr Pro Phe Thr Ser Arg Leu Ser Ile Asn
            180                 185                 190
Lys Asp Asn Ser Lys Ser Gln Val Phe Phe Lys Met Asn Ser Leu Gln
            195                 200                 205
Ser Asn Asp Thr Ala Ile Tyr Tyr Cys Ala Arg Ala Leu Thr Tyr Tyr
            210                 215                 220
Asp Tyr Glu Phe Ala Tyr Trp Gly Gln Gly Thr Leu Val Thr Val Ser
225                 230                 235                 240
Ser Gly Gly Gly Gly Ser Glu Val Gln Leu Val Glu Ser Gly Gly Gly
            245                 250                 255
Leu Val Gln Pro Gly Gly Ser Leu Lys Leu Ser Cys Ala Ala Ser Gly
            260                 265                 270
Phe Thr Phe Asn Lys Tyr Ala Met Asn Trp Val Arg Gln Ala Pro Gly
            275                 280                 285
Lys Gly Leu Glu Trp Val Ala Arg Ile Arg Ser Lys Tyr Asn Asn Tyr
            290                 295                 300
Ala Thr Tyr Tyr Ala Asp Ser Val Lys Asp Arg Phe Thr Ile Ser Arg
305                 310                 315                 320
Asp Asp Ser Lys Asn Thr Ala Tyr Leu Gln Met Asn Asn Leu Lys Thr
            325                 330                 335
Glu Asp Thr Ala Val Tyr Tyr Cys Val Arg His Gly Asn Phe Gly Asn
            340                 345                 350
Ser Tyr Ile Ser Tyr Trp Ala Tyr Trp Gly Gln Gly Thr Leu Val Thr
            355                 360                 365
Val Ser Ser Gly Gly Gly Gly Ser Gly Gly Gly Ser Gly Gly Gly
            370                 375                 380
Gly Ser Gln Thr Val Val Thr Gln Glu Pro Ser Leu Thr Val Ser Pro
385                 390                 395                 400
Gly Gly Thr Val Thr Leu Thr Cys Gly Ser Ser Thr Gly Ala Val Thr
            405                 410                 415
Ser Gly Asn Tyr Pro Asn Trp Val Gln Gln Lys Pro Gly Gln Ala Pro
            420                 425                 430
Arg Gly Leu Ile Gly Gly Thr Lys Phe Leu Ala Pro Gly Thr Pro Ala
            435                 440                 445
Arg Phe Ser Gly Ser Leu Leu Gly Gly Lys Ala Ala Leu Thr Leu Ser
            450                 455                 460
Gly Val Gln Pro Glu Asp Glu Ala Glu Tyr Tyr Cys Val Leu Trp Tyr
465                 470                 475                 480
Ser Asn Arg Trp Val Phe Gly Gly Gly Thr Lys Leu Thr Val Leu His
            485                 490                 495
His His His His His
            500
```

<210> 174
<211> 63
<212> PRT
<213> Artificial Sequence

<220>
<223> anti-CD33 Fynomer EE1L1B3

<400> 174

Gly Val Thr Leu Phe Val Ala Leu Tyr Asp Tyr Glu Ala Leu Gly Ala
1               5                   10              15
His Glu Leu Ser Phe His Lys Gly Glu Lys Phe Gln Ile Leu Asn Ser
            20                  25                  30
Ser Glu Gly Pro Phe Trp Glu Ala His Ser Leu Thr Thr Gly Glu Thr
            35                  40                  45
Gly Trp Ile Pro Ser Asn Tyr Val Ala Pro Val Asp Ser Ile Gln
            50                  55                  60

<210> 175
<211> 291
<212> PRT
<213> Artificial Sequence

<220>
<223> COVA467 LC

<400> 175

Asp Ile Gln Met Thr Gln Ser Pro Ser Ser Leu Ser Ala Ser Val Gly
1               5                   10              15
Asp Arg Val Thr Ile Thr Cys Ser Ala Ser Ser Ser Val Ser Tyr Met
            20                  25                  30
Asn Trp Tyr Gln Gln Thr Pro Gly Lys Ala Pro Lys Arg Trp Ile Tyr
            35                  40                  45
Asp Thr Ser Lys Leu Ala Ser Gly Val Pro Ser Arg Phe Ser Gly Ser
            50                  55                  60
Gly Ser Gly Thr Asp Tyr Thr Phe Thr Ile Ser Ser Leu Gln Pro Glu
65                  70                  75                  80
Asp Ile Ala Thr Tyr Tyr Cys Gln Gln Trp Ser Ser Asn Pro Phe Thr
                85                  90                  95
Phe Gly Gln Gly Thr Lys Leu Gln Ile Thr Arg Thr Val Ala Ala Pro
            100                 105                 110
Ser Val Phe Ile Phe Pro Pro Ser Asp Glu Gln Leu Lys Ser Gly Thr
            115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
            195                 200                 205
Asn Arg Gly Glu Cys Gly Gly Gly Ser Gly Gly Gly Gly Ser Gly
    210                 215                 220
Gly Gly Gly Ser Gly Val Thr Leu Phe Val Ala Leu Tyr Asp Tyr Glu
225                 230                 235                 240
Ala Leu Gly Ala His Glu Leu Ser Phe His Lys Gly Glu Lys Phe Gln
                245                 250                 255
Ile Leu Asn Ser Ser Glu Gly Pro Phe Trp Glu Ala His Ser Leu Thr
            260                 265                 270
Thr Gly Glu Thr Gly Trp Ile Pro Ser Asn Tyr Val Ala Pro Val Asp
            275                 280                 285
Ser Ile Gln
    290

<210> 176
<211> 511

<212> PRT
<213> Artificial Sequence

<220>
<223> COVA463, anti-CD3/CD33 scFv control

<400> 176

```
Gln Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Gly Met Asn Trp Val Lys Gln Ala Pro Gly Gln Gly Leu Lys Trp Met
            35                  40                  45
Gly Trp Ile Asn Thr Tyr Thr Gly Glu Pro Thr Tyr Ala Asp Asp Phe
            50                  55                  60
```

```
Lys Gly Arg Val Thr Met Thr Ser Asp Thr Ser Thr Ser Thr Ala Tyr
65              70              75                          80
Leu Glu Leu His Asn Leu Arg Ser Asp Asp Thr Ala Val Tyr Tyr Cys
            85              90                          95
Ala Arg Trp Ser Trp Ser Asp Gly Tyr Tyr Val Tyr Phe Asp Tyr Trp
            100             105             110
Gly Gln Gly Thr Thr Val Thr Val Ser Ser Gly Gly Gly Gly Ser Gly
        115             120             125
Gly Gly Gly Ser Gly Gly Gly Gly Ser Asp Ile Val Met Thr Gln Ser
        130             135             140
Pro Asp Ser Leu Thr Val Ser Leu Gly Glu Arg Thr Thr Ile Asn Cys
145             150             155                         160
Lys Ser Ser Gln Ser Val Leu Asp Ser Ser Lys Asn Lys Asn Ser Leu
            165             170                         175
Ala Trp Tyr Gln Gln Lys Pro Gly Gln Pro Pro Lys Leu Leu Leu Ser
            180             185             190
Trp Ala Ser Thr Arg Glu Ser Gly Ile Pro Asp Arg Phe Ser Gly Ser
            195             200             205
Gly Ser Gly Thr Asp Phe Thr Leu Thr Ile Asp Ser Leu Gln Pro Glu
        210             215             220
Asp Ser Ala Thr Tyr Tyr Cys Gln Gln Ser Ala His Phe Pro Ile Thr
225             230             235                         240
Phe Gly Gln Gly Thr Arg Leu Glu Ile Lys Ser Gly Gly Gly Gly Ser
            245             250             255
Glu Val Gln Leu Val Glu Ser Gly Gly Gly Leu Val Gln Pro Gly Gly
        260             265             270
Ser Leu Lys Leu Ser Cys Ala Ala Ser Gly Phe Thr Phe Asn Lys Tyr
        275             280             285
Ala Met Asn Trp Val Arg Gln Ala Pro Gly Lys Gly Leu Glu Trp Val
        290             295             300
Ala Arg Ile Arg Ser Lys Tyr Asn Asn Tyr Ala Thr Tyr Tyr Ala Asp
305             310             315                         320
Ser Val Lys Asp Arg Phe Thr Ile Ser Arg Asp Asp Ser Lys Asn Thr
            325             330                         335
Ala Tyr Leu Gln Met Asn Asn Leu Lys Thr Glu Asp Thr Ala Val Tyr
        340             345             350
Tyr Cys Val Arg His Gly Asn Phe Gly Asn Ser Tyr Ile Ser Tyr Trp
        355             360             365
Ala Tyr Trp Gly Gln Gly Thr Leu Val Thr Val Ser Ser Gly Gly Gly
        370             375             380
Gly Ser Gly Gly Gly Gly Ser Gly Gly Gly Gly Ser Gln Thr Val Val
385             390             395                         400
Thr Gln Glu Pro Ser Leu Thr Val Ser Pro Gly Gly Thr Val Thr Leu
            405             410                         415
Thr Cys Gly Ser Ser Thr Gly Ala Val Thr Ser Gly Asn Tyr Pro Asn
            420             425             430
Trp Val Gln Gln Lys Pro Gly Gln Ala Pro Arg Gly Leu Ile Gly Gly
            435             440             445
Thr Lys Phe Leu Ala Pro Gly Thr Pro Ala Arg Phe Ser Gly Ser Leu
            450             455             460
Leu Gly Gly Lys Ala Ala Leu Thr Leu Ser Gly Val Gln Pro Glu Asp
465             470             475                         480
Glu Ala Glu Tyr Tyr Cys Val Leu Trp Tyr Ser Asn Arg Trp Val Phe
            485             490                         495
Gly Gly Gly Thr Lys Leu Thr Val Leu His His His His His His
            500             505             510
```

<210> 177
<211> 63
<212> PRT
<213> Artificial Sequence

<220>
<223> anti-CD33 Fynomer EE3L11B4

<400> 177

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Arg Gly
            20                  25                  30
Ser Lys Gly Pro Phe Trp Glu Ala His Ser Leu Thr Thr Gly Glu Thr
            35                  40                  45
Gly Trp Ile Pro Ser Asn Tyr Val Ala Pro Val Asp Ser Ile Gln
            50                  55                  60
```

<210> 178
<211> 64
<212> PRT
<213> Artificial Sequence

<220>
<223> anti-CD33 Fynomer EE4L12D5

<400> 178

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Ser Ser
            20                  25                  30
Leu Ala Val Gly Pro Phe Trp Glu Ala His Ser Leu Thr Thr Gly Glu
            35                  40                  45
Thr Gly Trp Ile Pro Ser Asn Tyr Val Ala Pro Val Asp Ser Ile Gln
            50                  55                  60
```

<210> 179
<211> 64
<212> PRT
<213> Artificial Sequence

<220>
<223> anti-CD33 Fynomer EE4L12D3

<400> 179

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Met Met
            20                  25                  30
Ser Asp Glu Gly Pro Phe Trp Glu Ala His Ser Leu Thr Thr Gly Glu
            35                  40                  45
Thr Gly Trp Ile Pro Ser Asn Tyr Val Ala Pro Val Asp Ser Ile Gln
            50                  55                  60
```

<210> 180
<211> 64
<212> PRT
<213> Artificial Sequence

<220>

<223> anti-CD33 Fynomer EE4L12G1

<400> 180

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Ser Pro
            20                  25                  30
Arg Ser Glu Gly Pro Phe Trp Glu Ala His Ser Leu Thr Thr Gly Glu
            35                  40                  45
Thr Gly Trp Ile Pro Ser Asn Tyr Val Ala Pro Val Asp Ser Ile Gln
            50                  55                  60
```

<210> 181
<211> 64
<212> PRT
<213> Artificial Sequence

<220>
<223> anti-CD33 Fynomer EE6L12C6

<400> 181

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Ser Ser
            20                  25                  30
Leu Leu Gln Gly Pro Phe Trp Glu Ala His Ser Leu Thr Thr Gly Glu
            35                  40                  45
Thr Gly Trp Ile Pro Ser Asn Tyr Val Ala Pro Val Asp Ser Ile Gln
            50                  55                  60
```

<210> 182
<211> 65
<212> PRT
<213> Artificial Sequence

<220>
<223> anti-CD33 Fynomer EE23L59C1

<400> 182

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Asp Val
            20                  25                  30
Thr Leu Ala Gly Gly Pro Phe Trp Glu Ala His Ser Leu Thr Thr Gly
            35                  40                  45
Glu Thr Gly Trp Ile Pro Ser Asn Tyr Val Ala Pro Val Asp Ser Ile
            50                  55                  60
Gln
65
```

<210> 183
<211> 65
<212> PRT
<213> Artificial Sequence

**115**

<220>
<223> anti-CD33 Fynomer EE23L59A6A

<400> 183

```
        Gly Val Thr Leu Phe Val Ala Leu Tyr Asp Tyr Glu Ala Leu Gly Ala
        1               5                   10                  15
        His Glu Leu Ser Phe His Lys Gly Glu Lys Phe Gln Ile Leu Tyr Val
                    20                  25                  30
        Ala Leu Glu Gly Gly Pro Phe Trp Glu Ala His Ser Leu Thr Thr Gly


                    35                  40                  45
        Glu Thr Gly Trp Ile Pro Ser Asn Tyr Val Ala Pro Val Asp Ser Ile
                    50                  55                  60
        Gln
        65
```

<210> 184
<211> 64
<212> PRT
<213> Artificial Sequence


<220>
<223> anti-CD33 Fynomer EE23L62E1

<400> 184


```
        Gly Val Thr Leu Phe Val Ala Leu Tyr Asp Tyr Glu Ala Leu Gly Ala
        1               5                   10                  15
        His Glu Leu Ser Phe His Lys Gly Glu Lys Phe Gln Ile Leu Leu Arg
                    20                  25                  30
        Val Gln Leu Gly Pro Phe Trp Glu Ala His Ser Leu Thr Thr Gly Glu
                    35                  40                  45
        Thr Gly Trp Ile Pro Ser Asn Tyr Val Ala Pro Val Asp Ser Ile Gln
                    50                  55                  60
```

<210> 185
<211> 65
<212> PRT
<213> Artificial Sequence


<220>
<223> anti-CD33 Fynomer EE23L62D6

<400> 185


```
        Gly Val Thr Leu Phe Val Ala Leu Tyr Asp Tyr Glu Ala Leu Gly Ala
        1               5                   10                  15
        His Glu Leu Ser Phe His Lys Gly Glu Lys Phe Gln Ile Leu Asp Val
                    20                  25                  30
        Gly Leu Arg Gly Gly Pro Phe Trp Glu Ala His Ser Leu Thr Thr Gly
                    35                  40                  45
        Glu Thr Gly Trp Ile Pro Ser Asn Tyr Val Ala Pro Val Asp Ser Ile
                    50                  55                  60
        Gln
        65
```

<210> 186

<211> 65
<212> PRT
<213> Artificial Sequence

<220>
<223> anti-CD33 Fynomer EE23L62H7

<400> 186

```
Gly Val Thr Leu Phe Val Ala Leu Tyr Asp Tyr Glu Ala Leu Gly Ala
1                   5                   10                  15
His Glu Leu Ser Phe His Lys Gly Glu Lys Phe Gln Ile Leu Leu Arg
                20                  25                  30
Leu Val Leu Leu Gly Pro Phe Trp Glu Ala His Ser Leu Thr Thr Gly
            35                  40                  45
Glu Thr Gly Trp Ile Pro Ser Asn Tyr Val Ala Pro Val Asp Ser Ile
        50                  55                  60
```

Gln

65

<210> 187
<211> 66
<212> PRT
<213> Artificial Sequence

<220>
<223> anti-EGFR Fynomer ER9L3D7

<400> 187

```
Gly Val Thr Leu Phe Val Ala Leu Tyr Asp Tyr Glu Ala Arg Gly Leu
1                   5                   10                  15
Asn Arg Met Phe Asp Leu Ser Phe His Lys Gly Glu Lys Phe Gln Ile
            20                  25                  30
Leu Ser Thr Glu Asn Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 188
<211> 66
<212> PRT
<213> Artificial Sequence

<220>
<223> anti-EGFR Fynomer ER5L1E2

<400> 188

```
Gly Val Thr Leu Phe Val Ala Val Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Asn Arg Glu Ser Glu Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 189
<211> 66
<212> PRT
<213> Artificial Sequence

<220>
<223> anti-EGFR Fynomer ER5L1E5

<400> 189

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Gln Phe Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 190
<211> 66
<212> PRT
<213> Artificial Sequence

<220>
<223> anti-EGFR Fynomer ER5L1H3

<400> 190

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Ala Phe Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 191
<211> 66
<212> PRT
<213> Artificial Sequence

<220>
<223> anti-EGFR Fynomer ER6L1A9

<400> 191

```
        Gly Val Thr Leu Phe Val Ala Leu Tyr Asp Tyr Glu Ala Arg Gly Leu
        1               5                   10                  15
        Asn Arg Leu Phe Asp Leu Ser Phe His Lys Gly Glu Lys Phe Gln Ile
                    20                  25                  30
        Leu Asp Arg Thr Asn Gly Asp Trp Trp Glu Ala Arg Ser Leu Thr Thr
                    35                  40                  45
        Gly Glu Thr Gly Tyr Ile Pro Ser Asn Tyr Val Ala Pro Val Asp Ser
            50                  55                  60
        Ile Gln
        65
```

<210> 192
<211> 66
<212> PRT
<213> Artificial Sequence

<220>
<223> anti-EGFR Fynomer ER6L1C10

<400> 192

```
        Gly Val Thr Leu Phe Val Ala Leu Tyr Asp Tyr Glu Ala Arg Gly Leu
        1               5                   10                  15
        His Arg Met Phe Asp Leu Ser Phe His Lys Gly Glu Lys Phe Gln Ile
                    20                  25                  30
        Leu Asn Arg Glu Thr Gly Asp Trp Trp Glu Ala Arg Ser Leu Thr Thr
                    35                  40                  45
        Gly Glu Thr Gly Tyr Ile Pro Ser Asn Tyr Val Ala Pro Val Asp Ser
            50                  55                  60
        Ile Gln
        65
```

<210> 193
<211> 66
<212> PRT
<213> Artificial Sequence

<220>
<223> anti-EGFR Fynomer ER6L1F9

<400> 193

```
        Gly Val Thr Leu Phe Val Ala Leu Tyr Asp Tyr Glu Ala Arg Gly Leu
        1               5                   10                  15
        Asn Arg Leu Phe Asp Leu Ser Phe His Lys Gly Glu Lys Phe Gln Ile
                    20                  25                  30
        Leu Asp Arg Thr Asn Gly Asp Trp Trp Glu Ala Arg Ser Leu Thr Thr
                    35                  40                  45
        Gly Glu Thr Gly Tyr Ile Pro Ser Asn Tyr Val Ala Pro Val Asp Ser
            50                  55                  60
        Ile Gln
        65
```

<210> 194
<211> 66
<212> PRT
<213> Artificial Sequence

<220>
<223> anti-EGFR Fynomer ER7L2E8

<400> 194

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Thr Arg Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
            50                  55                  60
Ile Gln
65
```

<210> 195
<211> 67
<212> **PRT**
<213> Artificial Sequence

<220>
<223> anti-EGFR Fynomer ER8L3G1

<400> 195

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Asp Thr Ser Glu Ser Gly Asp Trp Trp Glu Ala Arg Ser Leu Thr
            35                  40                  45
Thr Gly Glu Thr Gly Tyr Ile Pro Ser Asn Tyr Val Ala Pro Val Asp
            50                  55                  60
Ser Ile Gln
65
```

<210> 196
<211> 66
<212> PRT
<213> Artificial Sequence

<220>
<223> anti-EGFR Fynomer 3D5

<400> 196

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Leu Pro Gln Glu Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 197
<211> 66
<212> PRT
<213> Artificial Sequence

<220>
<223> anti-EGFR Fynomer 1C6

<400> 197

```
Gly Val Thr Leu Phe Val Ala Leu Tyr Asp Tyr Glu Ala Arg Gly Leu
1               5                   10                  15
His Arg Met Phe Asp Leu Ser Phe His Lys Gly Glu Lys Phe Gln Ile
            20                  25                  30
Leu Asp Arg Thr Asn Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln
65
```

<210> 198
<211> 294
<212> PRT
<213> Artificial Sequence

<220>
<223> COVA494 LC

<400> 198

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Ser Phe Glu Thr Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln Gly Gly Gly Gly Ser Gly Gly Gly Gly Ser Gly Gly Gly Gly
65                  70                  75                  80
Ser Gln Val Val Leu Thr Gln Ser Pro Ala Ile Met Ser Ala Phe Pro
                85                  90                  95
Gly Glu Lys Val Thr Met Thr Cys Ser Ala Ser Ser Ser Val Ser Tyr
            100                 105                 110
Met Asn Trp Tyr Gln Gln Lys Ser Gly Thr Ser Pro Lys Arg Trp Ile
            115                 120                 125
Tyr Asp Ser Ser Lys Leu Ala Ser Gly Val Pro Ala Arg Phe Ser Gly
    130                 135                 140
Ser Gly Ser Gly Thr Ser Tyr Ser Leu Thr Ile Ser Ser Met Glu Thr
145                 150                 155                 160

Glu Asp Ala Ala Thr Tyr Tyr Cys Gln Gln Trp Ser Arg Asn Pro Pro
                165                 170                 175
Thr Phe Gly Gly Gly Thr Lys Leu Gln Ile Thr Arg Thr Val Ala Ala
            180                 185                 190
Pro Ser Val Phe Ile Phe Pro Pro Ser Asp Glu Gln Leu Lys Ser Gly
            195                 200                 205
Thr Ala Ser Val Val Cys Leu Leu Asn Asn Phe Tyr Pro Arg Glu Ala
    210                 215                 220
Lys Val Gln Trp Lys Val Asp Asn Ala Leu Gln Ser Gly Asn Ser Gln
225                 230                 235                 240
Glu Ser Val Thr Glu Gln Asp Ser Lys Asp Ser Thr Tyr Ser Leu Ser
            245                 250                 255
Ser Thr Leu Thr Leu Ser Lys Ala Asp Tyr Glu Lys His Lys Val Tyr
            260                 265                 270
Ala Cys Glu Val Thr His Gln Gly Leu Ser Ser Pro Val Thr Lys Ser
    275                 280                 285
Phe Asn Arg Gly Glu Cys
    290
```

<210> 199
<211> 532
<212> PRT
<213> Artificial Sequence

<220>
<223> COVA497 HC

<400> 199

```
Gly Val Thr Leu Phe Val Ala Leu Tyr Asp Tyr Glu Ala Arg Gly Leu
1               5                   10                  15
Asn Arg Met Phe Asp Leu Ser Phe His Lys Gly Glu Lys Phe Gln Ile
            20                  25                  30
Leu Ser Thr Glu Asn Gly Asp Trp Trp Glu Ala Arg Ser Leu Thr Thr
            35                  40                  45
Gly Glu Thr Gly Tyr Ile Pro Ser Asn Tyr Val Ala Pro Val Asp Ser
        50                  55                  60
Ile Gln Gly Gly Gly Gly Ser Gly Gly Gly Gly Ser Gly Gly Gly Gly
65                  70                  75                  80
Ser Gln Val Gln Leu Gln Gln Ser Gly Ala Glu Leu Ala Arg Pro Gly
            85                  90                  95
Ala Ser Val Lys Met Ser Cys Lys Ala Ser Gly Tyr Thr Phe Thr Arg
            100                 105                 110
Ser Thr Met His Trp Val Lys Gln Arg Pro Gly Gln Gly Leu Glu Trp
            115                 120                 125
Ile Gly Tyr Ile Asn Pro Ser Ser Ala Tyr Thr Asn Tyr Asn Gln Lys
        130                 135                 140
Phe Lys Asp Lys Ala Thr Leu Thr Ala Asp Lys Ser Ser Ser Thr Ala
145                 150                 155                 160
Tyr Met Gln Leu Ser Ser Leu Thr Ser Glu Asp Ser Ala Val Tyr Tyr
            165                 170                 175
Cys Ala Ser Pro Gln Val His Tyr Asp Tyr Asn Gly Phe Pro Tyr Trp
            180                 185                 190
Gly Gln Gly Thr Leu Val Thr Val Ser Ala Ala Ser Thr Lys Gly Pro
            195                 200                 205
Ser Val Phe Pro Leu Ala Pro Ser Ser Lys Ser Thr Ser Gly Gly Thr
            210                 215                 220
Ala Ala Leu Gly Cys Leu Val Lys Asp Tyr Phe Pro Glu Pro Val Thr
225                 230                 235                 240
Val Ser Trp Asn Ser Gly Ala Leu Thr Ser Gly Val His Thr Phe Pro
            245                 250                 255
Ala Val Leu Gln Ser Ser Gly Leu Tyr Ser Leu Ser Ser Val Val Thr
            260                 265                 270
Val Pro Ser Ser Ser Leu Gly Thr Gln Thr Tyr Ile Cys Asn Val Asn
```

```
                275                      280                      285
        His Lys Pro Ser Asn Thr Lys Val Asp Lys Lys Val Glu Pro Lys Ser
            290                      295                      300
        Cys Asp Lys Thr His Thr Cys Pro Pro Cys Pro Ala Pro Glu Leu Leu
        305                      310                      315          320
        Gly Gly Pro Ser Val Phe Leu Phe Pro Pro Lys Pro Lys Asp Thr Leu
                    325                      330                      335
        Met Ile Ser Arg Thr Pro Glu Val Thr Cys Val Val Val Ala Val Ser
                    340                      345                      350
        His Glu Asp Pro Glu Val Lys Phe Asn Trp Tyr Val Asp Gly Val Glu
                    355                      360                      365
        Val His Asn Ala Lys Thr Lys Pro Arg Glu Glu Gln Tyr Asn Ser Thr
            370                      375                      380
        Tyr Arg Val Val Ser Val Leu Thr Val Leu His Gln Asp Trp Leu Asn
        385                      390                      395          400
        Gly Lys Glu Tyr Lys Cys Lys Val Ser Asn Lys Ala Leu Ala Ala Pro
                    405                      410                      415
        Ile Glu Lys Thr Ile Ser Lys Ala Lys Gly Gln Pro Arg Glu Pro Gln
                    420                      425                      430
        Val Tyr Thr Leu Pro Pro Ser Arg Glu Glu Met Thr Lys Asn Gln Val
                    435                      440                      445
        Ser Leu Thr Cys Leu Val Lys Gly Phe Tyr Pro Ser Asp Ile Ala Val
            450                      455                      460
        Glu Trp Glu Ser Asn Gly Gln Pro Glu Asn Asn Tyr Lys Thr Thr Pro
        465                      470                      475          480
        Pro Val Leu Asp Ser Asp Gly Ser Phe Phe Leu Tyr Ser Lys Leu Thr
                    485                      490                      495
        Val Asp Lys Ser Arg Trp Gln Gln Gly Asn Val Phe Ser Cys Ser Val
                    500                      505                      510
        Met His Glu Ala Leu His Asn His Tyr Thr Gln Lys Ser Leu Ser Leu
                    515                      520                      525
        Ser Pro Gly Lys
            530
```

<210> 200
<211> 294
<212> PRT
<213> Artificial Sequence

<220>
<223> COVA499 LC

<400> 200

```
Gln Val Val Leu Thr Gln Ser Pro Ala Ile Met Ser Ala Phe Pro Gly
1               5               10              15
Glu Lys Val Thr Met Thr Cys Ser Ala Ser Ser Ser Val Ser Tyr Met
        20              25              30
Asn Trp Tyr Gln Gln Lys Ser Gly Thr Ser Pro Lys Arg Trp Ile Tyr
        35              40              45
Asp Ser Ser Lys Leu Ala Ser Gly Val Pro Ala Arg Phe Ser Gly Ser
    50              55              60
Gly Ser Gly Thr Ser Tyr Ser Leu Thr Ile Ser Ser Met Glu Thr Glu
65              70              75              80
Asp Ala Ala Thr Tyr Tyr Cys Gln Gln Trp Ser Arg Asn Pro Pro Thr
                85              90              95
Phe Gly Gly Gly Thr Lys Leu Gln Ile Thr Arg Thr Val Ala Ala Pro
            100             105             110
Ser Val Phe Ile Phe Pro Pro Ser Asp Glu Gln Leu Lys Ser Gly Thr
        115             120             125
Ala Ser Val Val Cys Leu Leu Asn Asn Phe Tyr Pro Arg Glu Ala Lys
    130             135             140
Val Gln Trp Lys Val Asp Asn Ala Leu Gln Ser Gly Asn Ser Gln Glu
145             150             155             160

Ser Val Thr Glu Gln Asp Ser Lys Asp Ser Thr Tyr Ser Leu Ser Ser
                165             170             175
Thr Leu Thr Leu Ser Lys Ala Asp Tyr Glu Lys His Lys Val Tyr Ala
            180             185             190
Cys Glu Val Thr His Gln Gly Leu Ser Ser Pro Val Thr Lys Ser Phe
            195             200             205
Asn Arg Gly Glu Cys Gly Gly Gly Gly Ser Gly Gly Gly Gly Ser Gly
    210             215             220
Gly Gly Gly Ser Gly Val Thr Leu Phe Val Ala Leu Tyr Asp Tyr Glu
225             230             235             240
Ala Arg Gly Leu Asn Arg Met Phe Asp Leu Ser Phe His Lys Gly Glu
            245             250             255
Lys Phe Gln Ile Leu Ser Thr Glu Asn Gly Asp Trp Trp Glu Ala Arg
            260             265             270
Ser Leu Thr Thr Gly Glu Thr Gly Tyr Ile Pro Ser Asn Tyr Val Ala
            275             280             285
Pro Val Asp Ser Ile Gln
            290
```

<210> 201
<211> 67
<212> PRT
<213> Artificial Sequence

<220>
<223> anti-EGFR Fynomer

<220>
<221> VARIANT
<222> 8,17,19,34,35,36,37
<223> Gly, Val, Thr, Leu, Phe, Ala, Tyr, Asp, Ser, His, Lys, Glu, Gln, Ile, Trp, Arg, Met, Pro, Asn, Cys

<220>
<221> VARIANT
<222> 38
<223> Gly, Val, Thr, Leu, Phe, Ala, Tyr, Asp, Ser, His, Lys, Glu, Gln, Ile, Trp, Arg, Met, Pro, Asn, Cys or is absent

<400> 201

```
Gly Val Thr Leu Phe Val Ala Xaa Tyr Asp Tyr Glu Ala Arg Gly Leu
1               5                   10                  15
Xaa Arg Xaa Phe Asp Leu Ser Phe His Lys Gly Glu Lys Phe Gln Ile
            20                  25                  30
Leu Xaa Xaa Xaa Xaa Xaa Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
        65
```

<210> 202
<211> 67
<212> PRT
<213> Artificial Sequence

<220>
<223> anti-EGFR Fynomer

<220>
<221> VARIANT
<222> 8,17,19,34,35,36,37
<223> Gly, Val, Thr, Leu, Phe, Ala, Tyr, Asp, Ser, His, Lys, Glu, Gln, Ile, Trp, Arg, Met, Pro, Asn

<220>
<221> VARIANT
<222> 38
<223> Gly, Val, Thr, Leu, Phe, Ala, Tyr, Asp, Ser, His, Lys, Glu, Gln, Ile, Trp, Arg, Met, Pro, Asn or is absent

<400> 202

```
Gly Val Thr Leu Phe Val Ala Xaa Tyr Asp Tyr Glu Ala Arg Gly Leu
1               5                   10                  15
Xaa Arg Xaa Phe Asp Leu Ser Phe His Lys Gly Glu Lys Phe Gln Ile
            20                  25                  30
Leu Xaa Xaa Xaa Xaa Xaa Gly Asp Trp Trp Glu Ala Arg Ser Leu Thr
            35                  40                  45
Thr Gly Glu Thr Gly Tyr Ile Pro Ser Asn Tyr Val Ala Pro Val Asp
        50                  55                  60
Ser Ile Gln
65
```

<210> 203
<211> 67
<212> PRT
<213> Artificial Sequence

<220>
<223> anti-EGFR Fynomer

<220>
<221> VARIANT

<222> 8
<223> Leu, Val

<220>
<221> VARIANT
<222> 17
<223> Asn, His

<220>
<221> VARIANT
<222> 19
<223> Met, Leu

<220>
<221> VARIANT
<222> 34
<223> Ser, Asn, Gln, Ala, Asp, Thr, Leu

<220>
<221> VARIANT
<222> 35
<223> Phe, Thr, Arg, Pro

<220>
<221> VARIANT
<222> 36
<223> Ser, Glu, Thr, Gln

<220>
<221> VARIANT
<222> 37
<223> Glu, Thr, Ser, Asn

<220>
<221> VARIANT
<222> 38
<223> Ser or is absent

<400> 203

```
Gly Val Thr Leu Phe Val Ala Xaa Tyr Asp Tyr Glu Ala Arg Gly Leu
1               5                   10                  15
Xaa Arg Xaa Phe Asp Leu Ser Phe His Lys Gly Glu Lys Phe Gln Ile
        20                  25                  30
Leu Xaa Xaa Xaa Xaa Xaa Gly Asp Trp Trp Glu Ala Arg Ser Leu Thr
        35                  40                  45
Thr Gly Glu Thr Gly Tyr Ile Pro Ser Asn Tyr Val Ala Pro Val Asp
        50                  55                  60
Ser Ile Gln
        65
```

<210> 204
<211> 9
<212> PRT
<213> Artificial Sequence

<220>
<223> anti-EGFR Fynomer RT-loop

<220>
<221> VARIANT
<222> 6
<223> Asn, His

<220>
<221> VARIANT
<222> 8
<223> Met, Leu

<400> 204

```
                    Glu Ala Arg Gly Leu Xaa Arg Xaa Phe
                    1               5
```

<210> 205
<211> 65
<212> PRT
<213> Artificial Sequence

<220>
<223> anti-CD33 Fynomer

<220>
<221> VARIANT
<222> 31,32,33,34
<223>

```
        Gly, Val, Thr, Leu, Phe, Ala, Tyr, Asp, Ser, His, Lys, Glu, Gln,
        Ile, Trp, Arg, Met, Pro, Asn, Cys
```

<220>
<221> VARIANT
<222> 35,36
<223> Gly, Val, Thr, Leu, Phe, Ala, Tyr, Asp, Ser, His, Lys, Glu, Gln, Ile, Trp, Arg, Met, Pro, Asn, Cys or is absent

<400> 205

```
        Gly Val Thr Leu Phe Val Ala Leu Tyr Asp Tyr Glu Ala Leu Gly Ala
        1               5                   10                  15
        His Glu Leu Ser Phe His Lys Gly Glu Lys Phe Gln Ile Leu Xaa Xaa
                            20                  25                  30
        Xaa Xaa Xaa Xaa Gly Pro Phe Trp Glu Ala His Ser Leu Thr Thr Gly
                    35                  40                  45
        Glu Thr Gly Trp Ile Pro Ser Asn Tyr Val Ala Pro Val Asp Ser Ile
                    50                  55                  60
        Gln
        65
```

<210> 206
<211> 65
<212> PRT
<213> Artificial Sequence

<220>

<223> anti-CD33 Fynomer

<220>
<221> VARIANT
<222> 31,32,33,34
<223>

```
Gly, Val, Thr, Leu, Phe, Ala, Tyr, Asp, Ser, His, Lys, Glu, Gln,
Ile, Trp, Arg, Met, Pro, Asn
```

<220>
<221> VARIANT
<222> 35,36
<223> Gly, Val, Thr, Leu, Phe, Ala, Tyr, Asp, Ser, His, Lys, Glu, Gln, Ile, Trp, Arg, Met, Pro, Asn or is absent

<400> 206

```
Gly Val Thr Leu Phe Val Ala Leu Tyr Asp Tyr Glu Ala Leu Gly Ala
1               5               10              15
His Glu Leu Ser Phe His Lys Gly Glu Lys Phe Gln Ile Leu Xaa Xaa
            20              25              30
Xaa Xaa Xaa Xaa Gly Pro Phe Trp Glu Ala His Ser Leu Thr Thr Gly
        35              40              45
Glu Thr Gly Trp Ile Pro Ser Asn Tyr Val Ala Pro Val Asp Ser Ile
    50              55              60
Gln
65
```

<210> 207
<211> 65
<212> PRT
<213> Artificial Sequence

<220>
<223> anti-CD33 Fynomer

<220>
<221> VARIANT
<222> 31
<223> Asn, Arg, Ser, Leu, Met, Asp, Tyr

<220>
<221> VARIANT
<222> 32
<223> Ser, Gly, Pro, Arg, Met, Val

<220>
<221> VARIANT
<222> 33
<223> Leu, Thr, Gly, Ser, Val, Arg, Ala

<220>
<221> VARIANT
<222> 34
<223> Ser, Ala, Leu, Gln, Asp, Val, Lys, Glu

<220>

<221> VARIANT
<222> 35
<223> Glu, Val, Gln, Leu, Ala, Arg or is absent

<220>
<221> VARIANT
<222> 36
<223> Leu, Gly or is absent

<400> 207

```
Gly Val Thr Leu Phe Val Ala Leu Tyr Asp Tyr Glu Ala Leu Gly Ala
1               5                   10                  15
His Glu Leu Ser Phe His Lys Gly Glu Lys Phe Gln Ile Leu Xaa Xaa
            20                  25                  30
Xaa Xaa Xaa Xaa Gly Pro Phe Trp Glu Ala His Ser Leu Thr Thr Gly
        35                  40                  45
Glu Thr Gly Trp Ile Pro Ser Asn Tyr Val Ala Pro Val Asp Ser Ile
    50                  55                  60
Gln
65
```

<210> 208
<211> 7
<212> PRT
<213> Artificial Sequence

<220>
<223> anti-CD33 Fynomer RT-loop

<400> 208

```
                        Glu Ala Leu Gly Ala His Glu
                        1               5
```

<210> 209
<211> 9
<212> PRT
<213> Artificial Sequence

<220>
<223> anti-EGFR Fynomer RT-loop

<220>
<221> VARIANT
<222> 6
<223>

```
Ala, Cys, Asp, Glu, Phe, Gly, His, Ile, Lys, Leu, Met, Asn, Pro,
Gln, Arg, Ser, Thr, Val, Trp, Tyr
```

<220>
<221> VARIANT
<222> 8
<223>

```
Ala, Cys, Asp, Glu, Phe, Gly, His, Ile, Lys, Leu, Met, Asn, Pro,
Gln, Arg, Ser, Thr, Val, Trp, Tyr
```

<400> 209

```
Glu Ala Arg Gly Leu Xaa Arg Xaa Phe
1                   5
```